# EUROPEAN PATENT APPLICATION

(11) **EP 4 198 036 A1**
(43) Date of publication of application: **21.06.2023**
(21) Application number: 23150608.0
(22) Date of filing: 02.09.2017
(51) Int. Cl.: C07D 471/04, A61K 31/437, A61P 29/00

(54) **8-(AZETIDIN-1-YL)-[1,2,4]TRIAZOLO[1,5-A] PYRIDINYL COMPOUNDS, COMPOSITIONS AND METHODS OF USE THEREOF**

(30) Priority: 06.09.2016 WO PCT/CN2016/098215
(62) Divisional of application: 17761254.6
(71) Applicant: F. Hoffmann-La Roche AG, 4070 Basel (CH)
(72) Inventor: GOODACRE, Simon Charles, Harlow, Essex, CM19 5TR (GB); ZAK, Mark, South San Francisco, 94080 (US); ROMERO, F. Anthony, South San Francisco, 94080 (US); CHENG, Yun-Xing, Beijing, 100176 (CN); CHENG, Limin, Beijing, 100176 (CN); HUA, Rongbao, Beijing, 100176 (CN)
(74) Representative: Schirlin, Julien

(57) **Abstract**

Compounds of Formula (I), or a stereoisomer, tautomer, solvate, prodrug or salt thereof, and methods of use as Janus kinase inhibitors are described herein.

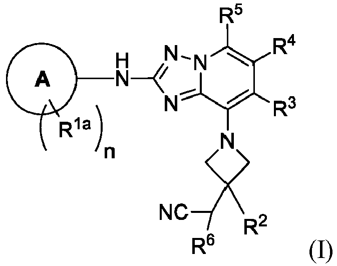

## Description

### CROSS-REFERENCE TO RELATED APPLICATION

This application claims the benefit of International Application No. PCT/CN2016/098215, filed September 6, 2016, which is incorporated herein by reference in its entirety.

### FIELD OF THE INVENTION

The field of the invention pertains to compounds of Formula (I) or (II), or a stereoisomer, tautomer, solvate, prodrug or salt thereof, and subformulas thereof, which are inhibitors of a Janus kinase, such as JAK1, as well as compositions containing these compounds, and methods of use including, but not limited to, diagnosis or treatment of patients suffering from a condition responsive to the inhibition of a JAK kinase.

### BACKGROUND OF INVENTION

Cytokine pathways mediate a broad range of biological functions, including many aspects of inflammation and immunity. Janus kinases (JAK), including JAK1, JAK2, JAK3 and TYK2, are cytoplasmic protein kinases that associate with type I and type II cytokine receptors and regulate cytokine signal transduction. Cytokine engagement with cognate receptors triggers activation of receptor associated JAKs and this leads to JAK-mediated tyrosine phosphorylation of signal transducer and activator of transcription (STAT) proteins and ultimately transcriptional activation of specific gene sets (Schindler et al., 2007, J. Biol. Chem. 282: 20059-63). JAK1, JAK2 and TYK2 exhibit broad patterns of gene expression, while JAK3 expression is limited to leukocytes. Cytokine receptors are typically functional as heterodimers, and as a result, more than one type of JAK kinase is usually associated with cytokine receptor complexes. The specific JAKs associated with different cytokine receptor complexes have been determined in many cases through genetic studies and corroborated by other experimental evidence. Exemplary therapeutic benefits of the inhibition of JAK enzymes are discussed, for example, in International Application No. WO 2013/014567.

JAK1 was initially identified in a screen for novel kinases (Wilks A.F., 1989, Proc. Natl. Acad. Sci. U.S.A. 86:1603-1607). Genetic and biochemical studies have shown that JAK1 is functionally and physically associated with the type I interferon (e.g., IFNalpha), type II interferon (e.g., IFNgamma), and IL-2 and IL-6 cytokine receptor complexes (Kisseleva et al., 2002, Gene 285:1-24; Levy et al., 2005, Nat. Rev. Mol. Cell Biol. 3:651-662; O'Shea et al., 2002, Cell, 109 (suppl.): S121-S131). JAK1 knockout mice die perinatally due to defects in LIF receptor signaling (Kisseleva et al., 2002, Gene 285:1-24; O'Shea et al., 2002, Cell, 109 (suppl.): S121-S131). Characterization of tissues derived from JAK1 knockout mice demonstrated critical roles for this kinase in the IFN, IL-10, IL-2/IL-4 and IL-6 pathways. A humanized monoclonal antibody targeting the IL-6 pathway (Tocilizumab) was approved by the European Commission for the treatment of moderate-to-severe rheumatoid arthritis (Scheinecker et al., 2009, Nat. Rev. Drug Discov. 8:273-274).

CD4 T cells play an important role in asthma pathogenesis through the production of TH2 cytokines within the lung, including IL-4, IL-9 and IL-13 (Cohn et al., 2004, Annu. Rev. Immunol. 22:789-815). IL-4 and IL-13 induce increased mucus production, recruitment of eosinophils to the lung, and increased production of IgE (Kasaian et al., 2008, Biochem. Pharmacol. 76(2): 147-155). IL-9 leads to mast cell activation, which exacerbates the asthma symptoms (Kearley et al., 2011, Am. J. Resp. Crit. Care Med., 183(7): 865-875). The IL-4Rα chain activates JAK1 and binds to either IL-4 or IL-13 when combined with the common gamma chain or the IL-13Rα1 chain respectively (Pernis et al., 2002, J. Clin. Invest. 109(10):1279-1283). The common gamma chain can also combine with IL-9Rα to bind to IL-9, and IL-9Rα activates JAK1 as well (Demoulin et al., 1996, Mol. Cell Biol. 16(9):4710-4716). While the common gamma chain activates JAK3, it has been shown that JAK1 is dominant over JAK3, and inhibition of JAK1 is sufficient to inactivate signaling through the common gamma chain despite JAK3 activity (Haan et al., 2011, Chem. Biol. 18(3):314-323). Inhibition of IL-4, IL-13 and IL-9 signaling by blocking the JAK/STAT signaling pathway can alleviate asthmatic symptoms in pre-clinical lung inflammation models (Mathew et al., 2001, J. Exp. Med. 193(9): 1087-1096; Kudlacz et. al., 2008, Eur. J. Pharmacol. 582(1-3): 154-161).

Biochemical and genetic studies have shown an association between JAK2 and single-chain (e.g., EPO), IL-3 and interferon gamma cytokine receptor families (Kisseleva et al., 2002, Gene 285:1-24; Levy et al., 2005, Nat. Rev. Mol. Cell Biol. 3:651-662; O'Shea et al., 2002, Cell, 109 (suppl.): S121-S131). Consistent with this, JAK2 knockout mice die of anemia (O'Shea et al., 2002, Cell, 109 (suppl.): S121-S131). Kinase activating mutations in JAK2 (e.g., JAK2 V617F) are associated with myeloproliferative disorders in humans.

JAK3 associates exclusively with the gamma common cytokine receptor chain, which is present in the IL-2, IL-4, IL-7, IL-9, IL-15 and IL-21 cytokine receptor complexes. JAK3 is critical for lymphoid cell development and proliferation and mutations in JAK3 result in severe combined immunodeficiency (SCID) (O'Shea et al., 2002, Cell, 109 (suppl.): S121-S131). Based on its role in regulating lymphocytes, JAK3 and JAK3-mediated pathways have been targeted for immunosuppressive indications (e.g., transplantation rejection and rheumatoid arthritis) (Baslund et al., 2005, Arthritis & Rheumatism 52:2686-2692; Changelian et al., 2003, Science 302: 875-878).

TYK2 associates with the type I interferon (e.g., IFNalpha), IL-6, IL-10, IL-12 and IL-23 cytokine receptor complexes (Kisseleva et al., 2002, Gene 285:1-24; Watford, W.T. & O'Shea, J.J., 2006, Immunity 25:695-697). Consistent with this, primary cells derived from a TYK2 deficient human are defective in type I interferon, IL-6, IL-10, IL-12 and IL-23 signaling. A fully human monoclonal antibody targeting the shared p40 subunit of the IL-12 and IL-23 cytokines (Ustekinumab) was recently approved by the European Commission for the treatment of moderate-to-severe plaque psoriasis (Krueger et al., 2007, N. Engl. J. Med. 356:580-92; Reich et al., 2009, Nat. Rev. Drug Discov. 8:355-356). In addition, an antibody targeting the IL-12 and IL-23 pathways underwent clinical trials for treating Crohn's Disease (Mannon et al., 2004, N. Engl. J. Med. 351:2069-79).

There exists a need in the art for additional or alternative treatments of conditions mediated by JAK kinases, such as those described above.

### SUMMARY OF THE INVENTION

Provided herein are JAK kinase inhibitory compounds. Accordingly, one aspect of the invention includes a compound of Formula (I) or (II): 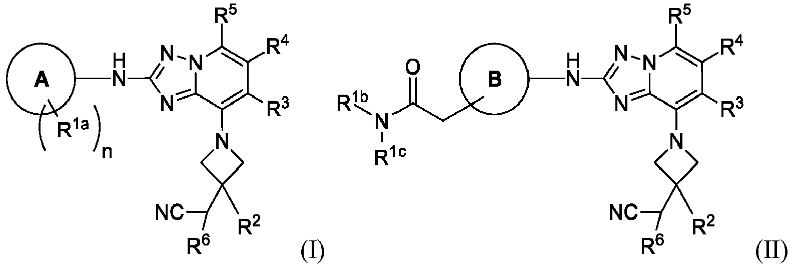 or a stereoisomer, tautomer, solvate, prodrug or salt thereof, wherein:
Ring A is phenyl, pyridinyl, pyrazolyl or isoquinolinyl;
Ring B is phenyl or 5-6 membered heteroaryl;
n is 0, 1 or 2;
R² is selected from
   (i) -(C0-C6 alkylene)-R^{c},
   (ii) -C(O)-NH-(C1-C6 alkyl optionally substituted by halogen, OH or CN) or
   (iii) -C(O)-(azetidinyl optionally substituted by C1-C6 alkyl or C1-C6 haloalkyl);
R³, R⁴ and R⁵ are each independently selected from the group consisting of hydrogen, CH₃, CH₂CH₃, OCH₃, CF₃, F and Cl;
R⁶ is H or C1-C3 alkyl;
R^{1a} independently at each occurrence is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or - C(O)-(3-7 membered heterocycloalkyl optionally substituted by C1-C6 alkyl);
R^{1b} and R^{1c} taken together form a 3-10 membered heterocycloalkyl optionally substituted by C1-C6 alkyl optionally substituted by halogen, CN, OH or C1-C6 alkoxy, -(C0-C6 alkylene)-(3-7 membered heterocycloalkyl optionally substituted by C1-C6 alkyl, -C(O)-(C1-C6 alkyl), -C(O)O-(C1-C6 alkyl) or - (C0-C6 alkylene)-C(O)-NR^{a}R^{b}) or -(C0-C6 alkylene)-NR^{a}R^{b};
R^{a} and R^{b} are independently selected from a group consisting of hydrogen, -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) and 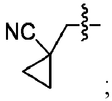 and
R^{c} is 3-10 membered cycloalkyl, 3-10 membered heterocycloalkyl, phenyl or 5-6 membered heteroaryl, wherein R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, - (C0-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).

Also provided are pharmaceutical compositions comprising a compound of Formula (I) or (II), or a stereoisomer, tautomer, solvate, prodrug or salt thereof, and a pharmaceutically acceptable carrier, diluent or excipient.

The present invention also provides, in some embodiments, use a compound of Formula (I) or (II) or a stereoisomer, tautomer, solvate, prodrug or salt thereof, in therapy, such as in the treatment of an inflammatory disease. Also provided are uses of a compound of Formula (I) or (II), or a stereoisomer, tautomer, solvate, prodrug or salt thereof, for the preparation of a medicament for the treatment of an inflammatory disease, such as asthma. Also provided is a method of preventing, treating or lessening the severity of a disease or condition responsive to the inhibition of a Janus kinase activity in a patient, comprising administering to the patient a therapeutically effective amount of a compound of Formula (I) or (II) or a stereoisomer, tautomer, solvate, prodrug or salt thereof.

### DETAILED DESCRIPTION OF THE INVENTION

### DEFINITIONS

"Halogen" or "halo" refers to F, Cl, Br or I. Additionally, terms such as "haloalkyl," are meant to include monohaloalkyl and polyhaloalkyl.

The term "alkyl" refers to a saturated linear or branched-chain monovalent hydrocarbon radical, wherein the alkyl radical may be optionally substituted. In one example, the alkyl radical is one to eighteen carbon atoms (C₁-C₁₈). In other examples, the alkyl radical is C₀-C₆, C₀-C₅, C₀-C₃, C₁-C₁₂, C₁-C₁₀, C₁-C₈, C₁-C₆, C₁-C₅, C₁-C₄, or C₁-C₃. C₀ alkyl refers to a bond. Examples of alkyl groups include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl and 1-octyl. In some embodiments, substituents for "optionally substituted alkyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

The term "alkenyl" refers to linear or branched-chain monovalent hydrocarbon radical with at least one site of unsaturation, i.e., a carbon-carbon double bond, wherein the alkenyl radical may be optionally substituted, and includes radicals having "cis" and "trans" orientations, or alternatively, "E" and "Z" orientations. In one example, the alkenyl radical is two to eighteen carbon atoms (C₂-C₁₈). In other examples, the alkenyl radical is C₂-C₁₂, C₂-C₁₀, C₂-C₈, C₂-C₆ or C₂-C₃. Examples include, but are not limited to, ethenyl or vinyl (-CH=CH₂), prop-1-enyl (-CH=CHCH₃), prop-2-enyl (-CH₂CH=CH₂), 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl and hexa-1,3-dienyl. In some embodiments, substituents for "optionally substituted alkenyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

The term "alkynyl" refers to a linear or branched monovalent hydrocarbon radical with at least one site of unsaturation, i.e., a carbon-carbon, triple bond, wherein the alkynyl radical may be optionally substituted. In one example, the alkynyl radical is two to eighteen carbon atoms (C₂-C₁₈). In other examples, the alkynyl radical is C₂-C₁₂, C₂-C₁₀, C₂-C₈, C₂-C₆ or C₂-C₃. Examples include, but are not limited to, ethynyl (-C---CH), prop-1-ynyl (-C≡CCH₃), prop-2-ynyl (propargyl, -CH₂C≡CH), but-1-ynyl, but-2-ynyl and but-3-ynyl. In some embodiments, substituents for "optionally substituted alkynyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

"Alkylene" refers to a saturated, branched or straight chain hydrocarbon group having two monovalent radical centers derived by the removal of two hydrogen atoms from the same or two different carbon atoms of a parent alkane. In one example, the divalent alkylene group is one to eighteen carbon atoms (C₁-C₁₈). In other examples, the divalent alkylene group is C₀-C₆, C₀-C₅, C₀-C₃, C₁-C₁₂, C₁-C₁₀, C₁-C₈, C₁-C₆, C₁-C₅, C₁-C₄, or C₁-C₃. The group C₀ alkylene refers to a bond. Example alkylene groups include methylene (-CH₂-), 1,1-ethyl (-CH(CH₃)-), (1,2-ethyl (-CH₂CH₂-), 1,1-propyl (-CH(CH₂CH₃)-), 2,2-propyl (-C(CH₃)₂-), 1,2-propyl (-CH(CH₃)CH₂-), 1,3-propyl (-CH₂CH₂CH₂-), 1,1-dimethyleth-1,2-yl (-C(CH₃)₂CH₂-), 1,4-butyl (-CH₂CH₂CH₂CH₂-), and the like.

The term "alkoxy" refers to a linear or branched monovalent radical represented by the formula -OR in which R is alkyl, as defined herein.

The term "thioalkyl" refers to a linear or branched monovalent radical represented by the formula -SR in which R is alkyl, as defined herein.

"Amino" means primary (i.e., -NH₂), secondary (i.e., -NRH), tertiary (i.e., - NRR) and quaternary (i.e., -N(+)RRR) amines, that are optionally substituted, in which each R is the same or different and selected from alkyl, cycloalkyl, aryl, and heterocyclyl, wherein the alkyl, cycloalkyl, aryl and heterocyclyl groups are as defined herein. Particular secondary and tertiary amines are alkylamine, dialkylamine, arylamine, diarylamine, aralkylamine and diaralkylamine, wherein the alkyl and aryl portions can be optionally substituted. Particular secondary and tertiary amines are methylamine, ethylamine, propylamine, isopropylamine, phenylamine, benzylamine, dimethylamine, diethylamine, dipropylamine and diisopropylamine. In some embodiments, R groups of a quarternary amine are each independently optionally substituted alkyl groups.

"Aryl" refers to a carbocyclic aromatic group, whether or not fused to one or more groups, having the number of carbon atoms designated, or if no number is designated, up to 14 carbon atoms. One example includes aryl groups having 6-14 carbon atoms. Another example includes aryl groups having 6-10 carbon atoms. Examples of aryl groups include phenyl, naphthyl, biphenyl, phenanthrenyl, naphthacenyl, 1,2,3,4-tetrahydronaphthalenyl, 1H-indenyl, 2,3-dihydro-1H-indenyl, and the like (see, e.g., Lang's Handbook of Chemistry (Dean, J. A., ed.) 13th ed. Table 7-2 [1985]). A particular aryl is phenyl. Substituted phenyl or substituted aryl means a phenyl group or aryl group substituted with one, two, three, four or five substituents, for example, 1-2, 1-3 or 1-4 substituents, such as chosen from groups specified herein (see "optionally substituted" definition), such as F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list. Examples of the term "substituted phenyl" include a mono- or di(halo)phenyl group such as 2-chlorophenyl, 2-bromophenyl, 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl, 2,4-difluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(alkyl)phenyl group such as 4-methylphenyl, 2,4-dimethylphenyl, 2-methylphenyl, 4-(isopropyl)phenyl, 4-ethylphenyl, 3-(n-propyl)phenyl and the like; a mono or di(alkoxy)phenyl group, for example, 3,4-dimethoxyphenyl, 3-methoxy-4-benzyloxyphenyl, 3-ethoxyphenyl, 4-(isopropoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy)phenyl group such 4-carboxyphenyl, a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups where the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl, 2-chloro-5-difluoromethoxy and the like, as well as trisubstituted phenyl groups where the substituents are different, for example 3-methoxy-4-benzyloxy-6-methyl sulfonylamino, 3-methoxy-4-benzyloxy-6-phenyl sulfonylamino, and tetrasubstituted phenyl groups where the substituents are different such as 3-methoxy-4-benzyloxy-5-methyl-6-phenyl sulfonylamino. In some embodiments, a substituent of an aryl, such as phenyl, comprises an amide. For example, an aryl (e.g., phenyl) substituent may be -(CH₂)₀₋₄CONR'R", wherein R' and R" each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo orNR'R".

"Cycloalkyl" refers to a non-aromatic, saturated or partially unsaturated hydrocarbon ring group wherein the cycloalkyl group may be optionally substituted independently with one or more substituents described herein. In one example, the cycloalkyl group is 3 to 12 carbon atoms (C₃-C₁₂). In other examples, cycloalkyl is C₃-C₈, C₃-C₁₀ or C₅-C₁₀. In other examples, the cycloalkyl group, as a monocycle, is C₃-C₈, C₃-C₆ or C₅-C₆. In another example, the cycloalkyl group, as a bicycle, is C₇-C₁₂. In another example, the cycloalkyl group, as a spiro system, is C₅-C₁₂. Examples of monocyclic cycloalkyl include cyclopropyl, cyclobutyl, cyclopentyl, 1-cyclopent-1-enyl, 1-cyclopent-2-enyl, 1-cyclopent-3-enyl, cyclohexyl, perdeuteriocyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl and cyclododecyl. Exemplary arrangements of bicyclic cycloalkyls having 7 to 12 ring atoms include, but are not limited to, [4,4], [4,5], [5,5], [5,6] or [6,6] ring systems. Exemplary bridged bicyclic cycloalkyls include, but are not limited to, bicyclo[2.2.1]heptane, bicyclo[2.2.2]octane and bicyclo[3.2.2]nonane. Examples of spiro cycloalkyl include, spiro[2.2]pentane, spiro[2.3]hexane, spiro[2.4]heptane, spiro[2.5]octane and spiro[4.5]decane. In some embodiments, substituents for "optionally substituted cycloalkyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, aryl and heterocyclic portions thereof may be optionally substituted, such as by one to four instances of substituents selected from this same list. In some embodiments, a substituent of a cycloalkyl comprises an amide. For example, a cycloalkyl substituent may be -(CH₂)₀₋₄CONR'R", wherein R' and R" each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo orNR'R".

"Guanidine" or "guanidinyl" means the group -NH-C(NH)-NHR in which R is hydrogen, alkyl, cycloalkyl, aryl or heterocyclyl, wherein the alkyl, cycloalkyl, aryl and heterocyclyl groups are as defined herein. A particular guanidine is the group -NH-C(NH)-NH₂.

"Heterocyclic group", "heterocyclic", "heterocycle", "heterocyclyl", or "heterocyclo" are used interchangeably and refer to any mono-, bi-, tricyclic or spiro, saturated or unsaturated, aromatic (heteroaryl) or non-aromatic (e.g., heterocycloalkyl), ring system, having 3 to 20 ring atoms, where the ring atoms are carbon, and at least one atom in the ring or ring system is a heteroatom selected from nitrogen, sulfur or oxygen. If any ring atom of a cyclic system is a heteroatom, that system is a heterocycle, regardless of the point of attachment of the cyclic system to the rest of the molecule. In one example, heterocyclyl includes 3-11 ring atoms ("members") and includes monocycles, bicycles, tricycles and spiro ring systems, wherein the ring atoms are carbon, where at least one atom in the ring or ring system is a heteroatom selected from nitrogen, sulfur or oxygen. In one example, heterocyclyl includes 1 to 4 heteroatoms. In one example, heterocyclyl includes 1 to 3 heteroatoms. In another example, heterocyclyl includes 3- to 7-membered monocycles having 1-2, 1-3 or 1-4 heteroatoms selected from nitrogen, sulfur or oxygen. In another example, heterocyclyl includes 4- to 6-membered monocycles having 1-2, 1-3 or 1-4 heteroatoms selected from nitrogen, sulfur or oxygen. In another example, heterocyclyl includes 3-membered monocycles. In another example, heterocyclyl includes 4-membered monocycles. In another example, heterocyclyl includes 5-6 membered monocycles, e.g., 5-6 membered heteroaryl. In another example, heterocyclyl includes 3-11 membered heterocycloyalkyls, such as 4-11 membered heterocycloalkyls. In some embodiments, a heterocycloalkyl includes at least one nitrogen. In one example, the heterocyclyl group includes 0 to 3 double bonds. Any nitrogen or sulfur heteroatom may optionally be oxidized (e.g., NO, SO, SO₂), and any nitrogen heteroatom may optionally be quaternized (e.g., [NR₄]⁺Cl⁻, [NR₄]⁺OH⁻). Example heterocycles are oxiranyl, aziridinyl, thiiranyl, azetidinyl, oxetanyl, thietanyl, 1,2-dithietanyl, 1,3-dithietanyl, pyrrolidinyl, dihydro-1H-pyrrolyl, dihydrofuranyl, tetrahydrofuranyl, dihydrothienyl, tetrahydrothienyl, imidazolidinyl, piperidinyl, piperazinyl, isoquinolinyl, tetrahydroisoquinolinyl, morpholinyl, thiomorpholinyl, 1,1-dioxo-thiomorpholinyl, dihydropyranyl, tetrahydropyranyl, hexahydrothiopyranyl, hexahydropyrimidinyl, oxazinanyl, thiazinanyl, thioxanyl, homopiperazinyl, homopiperidinyl, azepanyl, oxepanyl, thiepanyl, oxazepinyl, oxazepanyl, diazepanyl, 1,4-diazepanyl, diazepinyl, thiazepinyl, thiazepanyl, tetrahydrothiopyranyl, oxazolidinyl, thiazolidinyl, isothiazolidinyl, 1,1-dioxoisothiazolidinonyl, oxazolidinonyl, imidazolidinonyl, 4,5,6,7-tetrahydro[2H]indazolyl, tetrahydrobenzoimidazolyl, 4,5,6,7-tetrahydrobenzo[d]imidazolyl, 1,6-dihydroimidazol[4,5-d]pyrrolo[2,3-b]pyridinyl, thiazinyl, oxazinyl, thiadiazinyl, oxadiazinyl, dithiazinyl, dioxazinyl, oxathiazinyl, thiatriazinyl, oxatriazinyl, dithiadiazinyl, imidazolinyl, dihydropyrimidyl, tetrahydropyrimidyl, 1-pyrrolinyl, 2-pyrrolinyl, 3-pyrrolinyl, indolinyl, thiapyranyl, 2H-pyranyl, 4H-pyranyl, dioxanyl, 1,3-dioxolanyl, pyrazolinyl, pyrazolidinyl, dithianyl, dithiolanyl, pyrimidinonyl, pyrimidindionyl, pyrimidin-2,4-dionyl, piperazinonyl, piperazindionyl, pyrazolidinylimidazolinyl, 3-azabicyclo[3.1.0]hexanyl, 3,6-diazabicyclo[3.1.1]heptanyl, 6-azabicyclo[3.1.1]heptanyl, 3-azabicyclo[3.1.1]heptanyl, 3-azabicyclo[4.1.0]heptanyl, azabicyclo[2.2.2]hexanyl, 2-azabicyclo[3.2.1]octanyl, 8-azabicyclo[3.2.1]octanyl, 2-azabicyclo[2.2.2]octanyl, 8-azabicyclo[2.2.2]octanyl, 7-oxabicyclo[2.2.1]heptane, azaspiro[3.5]nonanyl, azaspiro [2.5]octanyl, azaspiro [4.5]decanyl, 1-azaspiro [4.5]decan-2-only, azaspiro[5.5]undecanyl, tetrahydroindolyl, octahydroindolyl, tetrahydroisoindolyl, tetrahydroindazolyl, 1,1-dioxohexahydrothiopyranyl. Examples of 5-membered heterocycles containing a sulfur or oxygen atom and one to three nitrogen atoms are thiazolyl, including thiazol-2-yl and thiazol-2-yl N-oxide, thiadiazolyl, including 1,3,4-thiadiazol-5-yl and 1,2,4-thiadiazol-5-yl, oxazolyl, for example oxazol-2-yl, and oxadiazolyl, such as 1,3,4-oxadiazol-5-yl, and 1,2,4-oxadiazol-5-yl. Example 5-membered ring heterocycles containing 2 to 4 nitrogen atoms include imidazolyl, such as imidazol-2-yl; triazolyl, such as 1,3,4-triazol-5-yl; 1,2,3-triazol-5-yl, 1,2,4-triazol-5-yl, and tetrazolyl, such as 1H-tetrazol-5-yl. Example benzo-fused 5-membered heterocycles are benzoxazol-2-yl, benzthiazol-2-yl and benzimidazol-2-yl. Example 6-membered heterocycles contain one to three nitrogen atoms and optionally a sulfur or oxygen atom, for example pyridyl, such as pyrid-2-yl, pyrid-3-yl, and pyrid-4-yl; pyrimidyl, such as pyrimid-2-yl and pyrimid-4-yl; triazinyl, such as 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl groups, are other example heterocycle groups. Heterocycles may be optionally substituted. For example, substituents for "optionally substituted heterocycles" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, aryl and heterocyclic portions thereof may be optionally substituted, such as by one to four instances of substituents selected from this same list. In some embodiments, a substituent of a heterocyclic group, such as a heteroaryl or heterocycloalkyl, comprises an amide. For example, a heterocyclic (e.g., heteroaryl or heterocycloalkyl) substituent may be -(CH₂)₀₋₄CONR'R", wherein R' and R" each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo or NR'R".

"Heteroaryl" refers to any mono-, bi-, or tricyclic ring system where at least one ring is a 5- or 6-membered aromatic ring containing from 1 to 4 heteroatoms selected from nitrogen, oxygen, and sulfur, and in an example embodiment, at least one heteroatom is nitrogen. See, for example, Lang's Handbook of Chemistry (Dean, J. A., ed.) 13th ed. Table 7-2 [1985]. Included in the definition are any bicyclic groups where any of the above heteroaryl rings are fused to an aryl ring, wherein the aryl ring or the heteroaryl ring is joined to the remainder of the molecule. In one embodiment, heteroaryl includes 5-6 membered monocyclic aromatic groups where one or more ring atoms is nitrogen, sulfur or oxygen. Example heteroaryl groups include thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, triazinyl, tetrazinyl, tetrazolo[1,5-b]pyridazinyl, imidazol[1,2-a]pyrimidinyl and purinyl, as well as benzo-fused derivatives, for example benzoxazolyl, benzofuryl, benzothiazolyl, benzothiadiazolyl, benzotriazolyl, benzoimidazolyl and indolyl. Heteroaryl groups can be optionally substituted. In some embodiments, substituents for "optionally substituted heteroaryls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list. In some embodiments, a substituent of a heteroaryl comprises an amide. For example, a heteroaryl substituent may be -(CH₂)₀₋₄CONR'R", wherein R' and R" each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo orNR'R".

In particular embodiments, a heterocyclyl group is attached at a carbon atom of the heterocyclyl group. By way of example, carbon bonded heterocyclyl groups include bonding arrangements at position 2, 3, 4, 5, or 6 of a pyridine ring, position 3, 4, 5, or 6 of a pyridazine ring, position 2, 4, 5, or 6 of a pyrimidine ring, position 2, 3, 5, or 6 of a pyrazine ring, position 2, 3, 4, or 5 of a furan, tetrahydrofuran, thiofuran, thiophene, pyrrole or tetrahydropyrrole ring, position 2, 4, or 5 of an oxazole, imidazole or thiazole ring, position 3, 4, or 5 of an isoxazole, pyrazole, or isothiazole ring, position 2 or 3 of an aziridine ring, position 2, 3, or 4 of an azetidine ring, position 2, 3, 4, 5, 6, 7, or 8 of a quinoline ring or position 1, 3, 4, 5, 6, 7, or 8 of an isoquinoline ring.

In certain embodiments, the heterocyclyl group is N-attached. By way of example, nitrogen bonded heterocyclyl or heteroaryl groups include bonding arrangements at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

The term "alkoxy" refers to a linear or branched monovalent radical represented by the formula -OR in which R is alkyl, as defined herein. Alkoxy groups include methoxy, ethoxy, propoxy, isopropoxy, mono-, di- and tri-fluoromethoxy and cyclopropoxy.

"Acyl" means a carbonyl containing substituent represented by the formula - C(O)-R in which R is hydrogen, alkyl, cycloalkyl, aryl or heterocyclyl, wherein the alkyl, cycloalkyl, aryl and heterocyclyl are as defined herein. Acyl groups include alkanoyl (e.g., acetyl), aroyl (e.g., benzoyl), and heteroaroyl (e.g., pyridinoyl).

"Optionally substituted" unless otherwise specified means that a group may be unsubstituted or substituted by one or more (e.g., 0, 1, 2, 3, 4, or 5 or more, or any range derivable therein) of the substituents listed for that group in which said substituents may be the same or different. In an embodiment, an optionally substituted group has 1 substituent. In another embodiment an optionally substituted group has 2 substituents. In another embodiment an optionally substituted group has 3 substituents. In another embodiment an optionally substituted group has 4 substituents. In another embodiment an optionally substituted group has 5 substituents.

Optional substituents for alkyl radicals, alone or as part of another substituent (e.g., alkoxy), as well as alkylenyl, alkenyl, alkynyl, heteroalkyl, heterocycloalkyl, and cycloalkyl, also each alone or as part of another substituent, can be a variety of groups, such as those described herein, as well as selected from the group consisting of halogen; oxo; CN; NO_{;} N₃; -OR'; perfluoro-C₁₋C₄ alkoxy; unsubstituted C₃-C₇ cycloalkyl; C₃-C₇ cycloalkyl substituted by halogen, OH, CN, unsubstituted C₁-C₆ alkyl, unsubstituted C₁-C₆ alkoxy, oxo or NR'R"; unsubstituted C₆-C₁₀ aryl (e.g., phenyl); C₆-C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; -NR'R"; -SR'; -SiR'R"R‴; -OC(O)R'; -C(O)R'; -CO₂R'; -CONR'R"; -OC(O)N R'R"; -NR"C(O)R'; -NR‴C(O)NR'R"; -NR"C(O)₂R'; -S(O)₂R'; -S(O)₂NR'R"; -NR'S(O )₂R"; -NR‴S(O)₂NR'R"; amidinyl; guanidinyl; -(CH₂)₁₋₄-OR'; -(CH₂)₁₋₄-NR'R"; -(CH₂)₁₋₄-SR'; -(CH₂)₁₋₄-SiR'R"R‴; -(CH₂)₁₋₄-OC(O)R'; -(CH₂)₁₋₄-C(O)R'; -(CH₂)₁₋₄-CO₂R'; and -(CH₂)₁₋₄CONR'R", or combinations thereof, in a number ranging from zero to (2m'+1), where m' is the total number of carbon atoms in such radical. R', R" and R‴ each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R". When R' and R" are attached to the same nitrogen atom, they can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo or NR'R". For example, -NR'R" is meant to include 1-pyrrolidinyl and 4-morpholinyl.

Similarly, optional substituents for the aryl and heteroaryl groups are varied. In some embodiments, substituents for aryl and heteroaryl groups are selected from the group consisting of halogen; CN; NO_{;} N₃; -OR'; perfluoro-C₁₋C₄ alkoxy; unsubstituted C₃-C₇ cycloalkyl; C₃-C₇ cycloalkyl substituted by halogen, OH, CN, unsubstituted C₁-C₆ alkyl, unsubstituted C₁-C₆ alkoxy, oxo or NR'R"; unsubstituted C₆-C₁₀ aryl (e.g., phenyl); C₆-C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R"; -NR'R"; -SR'; -SiR'R"R‴; -OC(O)R'; -C(O)R'; -CO₂R'; -CONR'R"; -OC(O)N R'R"; -NR"C(O)R'; -NR‴C(O)NR'R"; -NR"C(O)₂R'; -S(O)₂R'; -S(O)₂NR'R"; -NR'S(O )₂R"; -NR‴S(O)₂NR'R"; amidinyl; guanidinyl; -(CH₂)₁₋₄-OR'; -(CH₂)₁₋₄-NR'R"; -(CH₂)₁₋₄-SR'; -(CH₂)₁₋₄-SiR'R"R‴; -(CH₂)₁₋₄-OC(O)R'; -(CH₂)₁₋₄-C(O)R'; -(CH₂)₁₋₄-CO₂R'; and -(CH₂)₁₋₄CONR'R", or combinations thereof, in a number ranging from zero to (2m'+1), where m' is the total number of carbon atoms in such radical. R', R" and R‴ each independently refer to groups including, for example, hydrogen; unsubstituted C₁₋C₆ alkyl; C₁₋C₆ alkyl substituted by halogen, OH, CN, unsubstituted C₁-C₆ alkyl, unsubstituted C₁-C₆ alkoxy, oxo or NR'R"; unsubstituted C₁₋C₆ heteroalkyl; C₁₋C₆ heteroalkyl substituted by halogen, OH, CN, unsubstituted C₁-C₆ alkyl, unsubstituted C₁-C₆ alkoxy, oxo or NR'R"; unsubstituted C₆₋C₁₀ aryl; C₆₋C₁₀ aryl substituted by halogen, OH, CN, unsubstituted C₁-C₆ alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆ alkyl, unsubstituted C₁-C₆ alkoxy, oxo or NR'R". When R' and R" are attached to the same nitrogen atom, they can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆ alkyl, unsubstituted C₁-C₆ alkoxy, oxo or NR'R". For example, -NR'R" is meant to include 1-pyrrolidinyl and 4-morpholinyl.

The term "oxo" refers to =O or (=O)₂.

As used herein a wavy line " 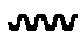 " that intersects a bond in a chemical structure indicate the point of attachment of the atom to which the wavy bond is connected in the chemical structure to the remainder of a molecule, or to the remainder of a fragment of a molecule. In some embodiments, an arrow together with an asterisk is used in the manner of a wavy line to indicate a point of attachment.

In certain embodiments, divalent groups are described generically without specific bonding configurations. It is understood that the generic description is meant to include both bonding configurations, unless specified otherwise. For example, in the group R¹-R²-R³, if the group R² is described as -CH₂C(O)-, then it is understood that this group can be bonded both as R¹-CH₂C(O)-R³, and as R¹-C(O)CH₂-R³, unless specified otherwise.

The phrase "pharmaceutically acceptable" refers to molecular entities and compositions that do not produce an adverse, allergic or other untoward reaction when administered to an animal, such as, for example, a human, as appropriate.

Compounds of the present invention may be in the form of a salt, such as a pharmaceutically acceptable salt. "Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, benzenesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particular base addition salts are the ammonium, potassium, sodium, calcium and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particular organic non-toxic bases include isopropylamine, diethylamine, ethanolamine, tromethamine, dicyclohexylamine, choline, and caffeine.

In some embodiments, a salt is selected from a hydrochloride, hydrobromide, trifluoroacetate, sulphate, phosphate, acetate, fumarate, maleate, tartrate, lactate, citrate, pyruvate, succinate, oxalate, methanesulphonate, p-toluenesulphonate, bisulphate, benzenesulphonate, ethane sulphonate, malonate, xinafoate, ascorbate, oleate, nicotinate, saccharinate, adipate, formate, glycolate, palmitate, L-lactate, D-lactate, aspartate, malate, L-tartrate, D-tartrate, stearate, furoate (e.g., 2-furoate or 3-furoate), napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), isethionate (2-hydroxyethylsulfonate), 2-mesitylenesulphonate, 2-naphthalenesulphonate, 2,5-dichlorobenzenesulphonate, D-mandelate, L-mandelate, cinnamate, benzoate, adipate, esylate, malonate, mesitylate (2-mesitylenesulphonate), napsylate (2-naphthalenesulfonate), camsylate (camphor-10-sulphonate, for example (1S)-(+)-10-camphorsulfonic acid salt), glutamate, glutarate, hippurate (2-(benzoylamino)acetate), orotate, xylate (p-xylene-2-sulphonate), and pamoic (2,2'-dihydroxy-1,1'-dinaphthylmethane-3,3'-dicarboxylate).

A "sterile" formulation is aseptic or free from all living microorganisms and their spores.

"Stereoisomers" refer to compounds that have identical chemical constitution, but differ with regard to the arrangement of the atoms or groups in space. Stereoisomers include diastereomers, enantiomers, conformers and the like.

"Chiral" refers to molecules which have the property of non-superimposability of the mirror image partner, while the term "achiral" refers to molecules which are superimposable on their mirror image partner.

"Diastereomer" refers to a stereoisomer with two or more centers of chirality and whose molecules are not mirror images of one another. Diastereomers have different physical properties, e.g., melting points, boiling points, spectral properties or biological activities. Mixtures of diastereomers may separate under high resolution analytical procedures such as electrophoresis and chromatography such as HPLC.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active forms, i.e., they have the ability to rotate the plane of plane-polarized light. In describing an optically active compound, the prefixes D and L, or *R* and S, are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Certain compounds of the present invention can exist in unsolvated forms as well as solvated forms, including hydrated forms. A "solvate" refers to an association or complex of one or more solvent molecules and a compound of the present invention. Examples of solvents that form solvates include water, isopropanol, ethanol, methanol, DMSO, ethyl acetate, acetic acid, and ethanolamine. Certain compounds of the present invention can exist in multiple crystalline or amorphous forms. In general, all physical forms are intended to be within the scope of the present invention. The term "hydrate" refers to the complex where the solvent molecule is water.

A "metabolite" refers to a product produced through metabolism in the body of a specified compound or salt thereof. Such products can result, for example, from the oxidation, reduction, hydrolysis, amidation, deamidation, esterification, deesterification, enzymatic cleavage, and the like, of the administered compound.

Metabolite products typically are identified by preparing a radiolabelled (e.g., ¹⁴C or ³H) isotope of a compound of the invention, administering it in a detectable dose (e.g., greater than about 0.5 mg/kg) to an animal such as rat, mouse, guinea pig, monkey, or to a human, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS, LC/MS or NMR analysis. In general, analysis of metabolites is done in the same way as conventional drug metabolism studies well known to those skilled in the art. The metabolite products, so long as they are not otherwise found in vivo, are useful in diagnostic assays for therapeutic dosing of the compounds of the invention.

"Amino-protecting group" as used herein refers to a derivative of the groups commonly employed to block or protect an amino group while reactions are carried out on other functional groups on the compound. Examples of such protecting groups include carbamates, amides, alkyl and aryl groups, and imines, as well as many N-heteroatom derivatives which can be removed to regenerate the desired amine group. Particular amino protecting groups are Pmb (p-Methoxybenzyl), Boc (tert-Butyloxycarbonyl), Fmoc (9-Fluorenylmethyloxycarbonyl) and Cbz (Carbobenzyloxy). Further examples of these groups are found in T. W. Greene and P. G. M. Wuts, "Protecting Groups in Organic Synthesis, 3rd ed., John Wiley & Sons, Inc., 1999. The term "protected amino" refers to an amino group substituted with one of the above amino-protecting groups.

"Carboxy-protecting group" as used herein refers to those groups that are stable to the conditions of subsequent reaction(s) at other positions of the molecule, which may be removed at the appropriate point without disrupting the remainder of the molecule, to give the unprotected carboxy-group. Examples of carboxy protecting groups include, ester groups and heterocyclyl groups. Ester derivatives of the carboxylic acid group may be employed to block or protect the carboxylic acid group while reactions are carried out on other functional groups on the compound. Examples of such ester groups include substituted arylalkyl, including substituted benzyls, such as 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, alkyl or substituted alkyl esters such as methyl, ethyl, t-butyl allyl or t-amyl, triphenylmethyl (trityl), 4-methoxytrityl, 4,4'-dimethoxytrityl, 4,4',4"-trimethoxytrityl, 2-phenylprop-2-yl, thioesters such as t-butyl thioester, silyl esters such as trimethylsilyl, t-butyldimethylsilyl esters, phenacyl, 2,2,2-trichloroethyl, beta-(trimethylsilyl)ethyl, beta-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. Another example of carboxy-protecting groups are heterocyclyl groups such as 1,3-oxazolinyl. Further examples of these groups are found in T. W. Greene and P. G. M. Wuts, "Protecting Groups in Organic Synthesis, 3rd ed., John Wiley & Sons, Inc., 1999. The term "protected carboxy" refers to a carboxy group substituted with one of the above carboxy-protecting groups.

"Hydroxy-protecting group" as used herein refers to a derivative of the hydroxy group commonly employed to block or protect the hydroxy group while reactions are carried out on other functional groups on the compound. Examples of such protecting groups include tetrahydropyranyloxy, benzoyl, acetoxy, carbamoyloxy, benzyl, and silylethers (e.g., TBS, TBDPS) groups. Further examples of these groups are found in T. W. Greene and P. G. M. Wuts, "Protecting Groups in Organic Synthesis, 3rd ed., John Wiley & Sons, Inc., 1999. The term "protected hydroxy" refers to a hydroxy group substituted with one of the above hydroxy-protecting groups.

A "subject," "individual," or "patient" is a vertebrate. In certain embodiments, the vertebrate is a mammal. Mammals include, but are not limited to, farm animals (such as cows), sport animals, pets (such as guinea pigs, cats, dogs, rabbits and horses), primates, mice and rats. In certain embodiments, a mammal is a human. In embodiments comprising administration of a compound of Formula (I) or (II) or a stereoisomer, tautomer, solvate, prodrug or salt thereof, to a patient, the patient is typically in need thereof.

The term "Janus kinase" refers to JAK1, JAK2, JAK3 and TYK2 protein kinases. In some embodiments, a Janus kinase may be further defined as one of JAK1, JAK2, JAK3 or TYK2. In any embodiment, any one of JAK1, JAK2, JAK3 and TYK2 may be specifically excluded as a Janus kinase. In some embodiments, a Janus kinase is JAK1. In some embodiments, a Janus kinase is a combination of JAK1 and JAK2.

The terms "inhibiting" and "reducing," or any variation of these terms, includes any measurable decrease or complete inhibition to achieve a desired result. For example, there may be a decrease of about, at most about, or at least about 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, reduction of activity (e.g., JAK1 activity) compared to normal.

In some embodiments, a compound of Formula (I) or (II) or a stereoisomer, tautomer, solvate, prodrug or salt thereof is selective for inhibition of JAK1 over JAK3 and TYK2. In some embodiments, a compound of Formula (I) or (II) is selective for inhibition of JAK1 over JAK2, JAK3, or TYK2, or any combination of JAK2, JAK3, or TYK2. In some embodiments, a compound of Formula (I) or (II) or a stereoisomer, tautomer, solvate, prodrug or salt thereof is selective for inhibition of JAK1 and JAK2 over JAK3 and TYK2. In some embodiments, a compound of Formula (I) or (II) or a stereoisomer, tautomer, solvate, prodrug or salt thereof is selective for inhibition of JAK1 over JAK3. By "selective for inhibition" it is meant that the compound is at least a 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, better inhibitor of a particular Janus kinase (e.g., JAK1) activity compared to another particular Janus kinase (e.g., JAK1) activity, or is at least a 2-, 3-, 4-, 5-, 10-, 25-, 50-, 100-, 250-, or 500-fold better inhibitor of a particular Janus kinase (e.g., JAK1) activity compared to another particular Janus kinase (e.g., JAK1) activity.

"Therapeutically effective amount" means an amount of a compound of the present invention, such as a compound of Formula (I) or (II) or a stereoisomer, tautomer, solvate, prodrug or salt thereof, that (i) treats or prevents the particular disease, condition or disorder, or (ii) attenuates, ameliorates or eliminates one or more symptoms of the particular disease, condition, or disorder, and optionally (iii) prevents or delays the onset of one or more symptoms of the particular disease, condition or disorder described herein. In some embodiments, the therapeutically effective amount is an amount sufficient to decrease or alleviate the symptoms of an autoimmune or inflammatory disease (e.g., asthma). In some embodiments, a therapeutically effective amount is an amount of a chemical entity described herein sufficient to significantly decrease the activity or number of B-cells. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; or relieve to some extent one or more of the symptoms associated with the cancer. To the extent the drug may prevent growth or kill existing cancer cells, it may be cytostatic or cytotoxic. For cancer therapy, efficacy can, for example, be measured by assessing the time to disease progression (TTP) or determining the response rate (RR).

"Treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, stabilized (i.e., not worsening) state of disease, decreasing the rate of disease progression, amelioration or palliation of the disease state, prolonging survival as compared to expected survival if not receiving treatment and remission or improved prognosis. In some embodiments, compounds of the invention, such as a compound of Formula (I) or (II), are used to delay development of a disease or disorder or to slow the progression of a disease or disorder. Those in need of treatment include those already with the condition or disorder as well as those prone to have the condition or disorder, (for example, through a genetic mutation) or those in which the condition or disorder is to be prevented.

"Inflammatory disorder" refers to any disease, disorder or syndrome in which an excessive or unregulated inflammatory response leads to excessive inflammatory symptoms, host tissue damage, or loss of tissue function. "Inflammatory disorder" also refers to a pathological state mediated by influx of leukocytes or neutrophil chemotaxis.

"Inflammation" refers to a localized, protective response elicited by injury or destruction of tissues, which serves to destroy, dilute, or wall off (sequester) both the injurious agent and the injured tissue. Inflammation is notably associated with influx of leukocytes or neutrophil chemotaxis. Inflammation can result from infection with pathogenic organisms and viruses and from noninfectious means such as trauma or reperfusion following myocardial infarction or stroke, immune responses to foreign antigens, and autoimmune responses. Accordingly, inflammatory disorders amenable to treatment with a compound of the present invention, such as a compound of Formula (I) or (II), encompass disorders associated with reactions of the specific defense system as well as with reactions of the nonspecific defense system.

"Specific defense system" refers to the component of the immune system that reacts to the presence of specific antigens. Examples of inflammation resulting from a response of the specific defense system include the classical response to foreign antigens, autoimmune diseases, and delayed type hypersensitivity responses mediated by T-cells. Chronic inflammatory diseases, the rejection of solid transplanted tissue and organs, e.g., kidney and bone marrow transplants, and graft versus host disease (GVHD), are further examples of inflammatory reactions of the specific defense system.

The term "nonspecific defense system" refers to inflammatory disorders that are mediated by leukocytes that are incapable of immunological memory (e.g., granulocytes, and macrophages). Examples of inflammation that result, at least in part, from a reaction of the nonspecific defense system include inflammation associated with conditions such as adult (acute) respiratory distress syndrome (ARDS) or multiple organ injury syndromes; reperfusion injury; acute glomerulonephritis; reactive arthritis; dermatoses with acute inflammatory components; acute purulent meningitis or other central nervous system inflammatory disorders such as stroke; thermal injury; inflammatory bowel disease; granulocyte transfusion associated syndromes; and cytokine-induced toxicity.

"Autoimmune disease" refers to any group of disorders in which tissue injury is associated with humoral or cell-mediated responses to the body's own constituents. Non-limiting examples of autoimmune diseases include rheumatoid arthritis, lupus and multiple sclerosis.

"Allergic disease" as used herein refers to any symptoms, tissue damage, or loss of tissue function resulting from allergy. "Arthritic disease" as used herein refers to any disease that is characterized by inflammatory lesions of the joints attributable to a variety of etiologies. "Dermatitis" as used herein refers to any of a large family of diseases of the skin that are characterized by inflammation of the skin attributable to a variety of etiologies. "Transplant rejection" as used herein refers to any immune reaction directed against grafted tissue, such as organs or cells (e.g., bone marrow), characterized by a loss of function of the grafted and surrounding tissues, pain, swelling, leukocytosis, and thrombocytopenia. The therapeutic methods of the present invention include methods for the treatment of disorders associated with inflammatory cell activation.

"Inflammatory cell activation" refers to the induction by a stimulus (including, but not limited to, cytokines, antigens or auto-antibodies) of a proliferative cellular response, the production of soluble mediators (including but not limited to cytokines, oxygen radicals, enzymes, prostanoids, or vasoactive amines), or cell surface expression of new or increased numbers of mediators (including, but not limited to, major histocompatability antigens or cell adhesion molecules) in inflammatory cells (including but not limited to monocytes, macrophages, T lymphocytes, B lymphocytes, granulocytes (i.e., polymorphonuclear leukocytes such as neutrophils, basophils, and eosinophils), mast cells, dendritic cells, Langerhans cells, and endothelial cells). It will be appreciated by persons skilled in the art that the activation of one or a combination of these phenotypes in these cells can contribute to the initiation, perpetuation, or exacerbation of an inflammatory disorder.

In some embodiments, inflammatory disorders which can be treated according to the methods of this invention include, but are not limited to, asthma, rhinitis (e.g., allergic rhinitis), allergic airway syndrome, atopic dermatitis, bronchitis, rheumatoid arthritis, psoriasis, contact dermatitis, chronic obstructive pulmonary disease and delayed hypersensitivity reactions.

The terms "cancer" and "cancerous", "neoplasm", and "tumor" and related terms refer to or describe the physiological condition in mammals that is typically characterized by unregulated cell growth. A "tumor" comprises one or more cancerous cells. Examples of cancer include carcinoma, blastoma, sarcoma, seminoma, glioblastoma, melanoma, leukemia, and myeloid or lymphoid malignancies. More particular examples of such cancers include squamous cell cancer (e.g., epithelial squamous cell cancer) and lung cancer including small-cell lung cancer, non-small cell lung cancer ("NSCLC"), adenocarcinoma of the lung and squamous carcinoma of the lung. Other cancers include skin, keratoacanthoma, follicular carcinoma, hairy cell leukemia, buccal cavity, pharynx (oral), lip, tongue, mouth, salivary gland, esophageal, larynx, hepatocellular, gastric, stomach, gastrointestinal, small intestine, large intestine, pancreatic, cervical, ovarian, liver, bladder, hepatoma, breast, colon, rectal, colorectal, genitourinary, biliary passage, thyroid, papillary, hepatic, endometrial, uterine, salivary gland, kidney or renal, prostate, testis, vulval, peritoneum, anal, penile, bone, multiple myeloma, B-cell lymphoma, central nervous system, brain, head and neck, Hodgkin's, and associated metastases. Examples of neoplastic disorders include myeloproliferative disorders, such as polycythemia vera, essential thrombocytosis, myelofibrosis, such as primary myelofibrosis, and chronic myelogenous leukemia (CML).

A "chemotherapeutic agent" is an agent useful in the treatment of a given disorder, for example, cancer or inflammatory disorders. Examples of chemotherapeutic agents are well-known in the art and include examples such as those disclosed in U.S. Publ. Appl. No. 2010/0048557, incorporated herein by reference. Additionally, chemotherapeutic agents include pharmaceutically acceptable salts, acids or derivatives of any of chemotherapeutic agents, as well as combinations of two or more of them.

"Package insert" is used to refer to instructions customarily included in commercial packages of therapeutic products that contain information about the indications, usage, dosage, administration, contraindications or warnings concerning the use of such therapeutic products.

The terms "compound(s) of this invention," and "compound(s) of the present invention" and the like, unless otherwise indicated, include compounds of Formula (I) or (II), and stereoisomers (including atropisomers), geometric isomers, tautomers, solvates, metabolites, isotopes, salts (e.g., pharmaceutically acceptable salts), and prodrugs thereof. In some embodiments, solvates, metabolites, isotopes or prodrugs are excluded, or any combination thereof.

Unless otherwise stated, structures depicted herein are also meant to include compounds that differ only in the presence of one or more isotopically enriched atoms. Exemplary isotopes that can be incorporated into compounds of the present invention, such as a compound of Formula (I) or (II), include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I, respectively. Isotopically-labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, in compounds of Formula (I) or (II), one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Isotopically labeled compounds can generally be prepared by following procedures analogous to those disclosed in the Schemes or in the Examples herein, by substituting an isotopically labeled reagent for a non-isotopically labeled reagent.

It is specifically contemplated that any limitation discussed with respect to one embodiment of the invention may apply to any other embodiment of the invention. Furthermore, any compound or composition of the invention may be used in any method of the invention, and any method of the invention may be used to produce or to utilize any compound or composition of the invention.

The use of the term "or" is used to mean "and/or" unless explicitly indicated to refer to alternatives only or the alternative are mutually exclusive, although the disclosure supports a definition that refers to only alternatives and "and/or."

Throughout this application, the term "about" is used to indicate that a value includes the standard deviation of error for the device or method being employed to determine the value.

As used herein, "a" or "an" means one or more, unless clearly indicated otherwise. As used herein, "another" means at least a second or more.

Headings used herein are intended only for organizational purposes.

### INHIBITORS OF JANUS KINASES

Accordingly, one aspect of the invention includes a compound of Formula (I) or (II): 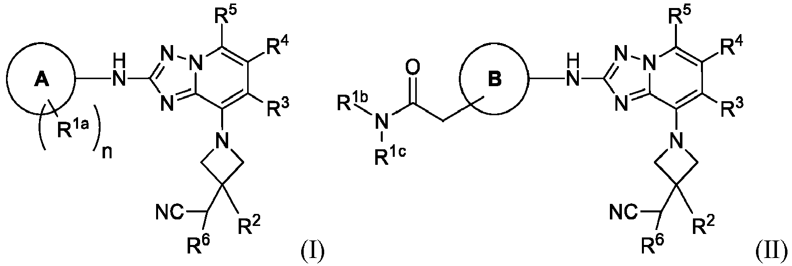 or a stereoisomer, tautomer, solvate, prodrug or salt thereof, wherein:
Ring A is phenyl, pyridinyl, pyrazolyl or isoquinolinyl;
Ring B is phenyl or 5-6 membered heteroaryl;
n is 0, 1 or 2;
R² is selected from
   (i) -(C0-C6 alkylene)-R^{c},
   (ii) -C(O)-NH-(C1-C6 alkyl optionally substituted by halogen, OH or CN) or
   (iii) -C(O)-(azetidinyl optionally substituted by C1-C6 alkyl or C1-C6 haloalkyl);
R³, R⁴ and R⁵ are each independently selected from the group consisting of hydrogen, CH₃, CH₂CH₃, OCH₃, CF₃, F and Cl;
R⁶ is H or C1-C3 alkyl;
R^{1a} independently at each occurrence is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or - C(O)-(3-7 membered heterocycloalkyl optionally substituted by C1-C6 alkyl);
R^{1b} and R^{1c} taken together form a 3-10 membered heterocycloalkyl optionally substituted by C1-C6 alkyl optionally substituted by halogen, CN, OH or C1-C6 alkoxy, -(C0-C6 alkylene)-(3-7 membered heterocycloalkyl optionally substituted by C1-C6 alkyl, -C(O)-(C1-C6 alkyl), -C(O)O-(C1-C6 alkyl) or - (C0-C6 alkylene)-C(O)-NR^{a}R^{b}) or -(C0-C6 alkylene)-NR^{a}R^{b};
R^{a} and R^{b} are independently selected from a group consisting of hydrogen, -C1-C6 alkyl optionally substituted by halogen, OH, CN or C1-C6 alkoxy, -C(0)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) and 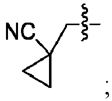 and
R^{c} is 3-10 membered cycloalkyl, 3-10 membered heterocycloalkyl, phenyl or 5-6 membered heteroaryl, wherein R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(C0-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).

Provided in some embodiments is a compound of Formula (I) or a stereoisomer, tautomer, solvate, prodrug or salt thereof. Provided in some embodiments is a compound of Formula (II) or a stereoisomer, tautomer, solvate, prodrug or salt thereof.

In some embodiments, R² is -C(O)-NH-(C1-C6 alkyl optionally substituted by halogen, OH or CN) or -C(O)-(azetidinyl optionally substituted by C1-C6 alkyl or C1-C6 haloalkyl). In some embodiments, R² is -(C0-C6 alkylene)-R^{c}.

In some embodiments, R^{c} is 3-10 membered cycloalkyl, 3-10 membered heterocycloalkyl or phenyl, where in R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(C0-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).

In some embodiments, R^{c} is 5-6 membered heteroaryl optionally substituted by halogen, CN, OH, C1-C6 alkoxy optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, - (C0-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).

In some embodiments, wherein n is 0. In some embodiments, n is 1 and R^{1a} is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or -C(O)-(3-7 membered heterocycloalkyl optionally substituted by C1-C6 alkyl). In some embodiments, n is 1 and R^{1a} independently at each occurrence is halogen, C1-C6 alkyl optionally substituted by halogen, CN or OH, C1-C6 alkoxy, 3-10 membered cycloalkyl or 3-10 membered heterocycloalkyl.

In some embodiments, R^{a} is hydrogen or C1-C3 alkyl, and R^{b} is -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) or

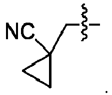

In some embodiments, Ring A is phenyl, pyridinyl or pyrazolyl. In some embodiments, Ring A is pyrazolyl. In some embodiments, Ring B is phenyl. In some embodiments, Ring B is 5-6 membered heteroaryl. In some embodiments, Ring B is pyrazolyl.

In some embodiments, R³, R⁴ and R⁵ are each independently selected from the group consisting of hydrogen, CH₃, CH₂CH₃, CF₃, F and Cl. In some embodiments, R³, R⁴ and R⁵ are each hydrogen. In some embodiments, R⁶ is hydrogen.

In some embodiments, a compound is selected from Table 1 or of the Examples, or a stereoisomer, tautomer, solvate, prodrug or salt thereof.

Also provided is a pharmaceutical composition comprising a compound of Formula (I) or (II), or a stereoisomer, tautomer, solvate, prodrug or salt thereof, and a pharmaceutically acceptable carrier, diluent or excipient.

Use of a compound of Formula (I) or (II) or a stereoisomer, tautomer, solvate, prodrug or salt thereof in therapy is also provided.

Use of a compound of Formula (I) or (II) or a stereoisomer, tautomer, solvate, prodrug or salt thereof in the treatment of an inflammatory disease, such as asthma, is also provided.

Use of a compound of Formula (I) or (II) or a stereoisomer, tautomer, solvate, prodrug or salt thereof for the preparation of a medicament for the treatment of an inflammatory disease, such as asthma, is also provided.

Also provided is a compound of Formula (I) or (II) or a stereoisomer, tautomer, solvate, prodrug or salt thereof for use in the treatment of an inflammatory disease, such as asthma.

Also provided is a method of preventing, treating or lessening the severity of a disease or condition responsive to the inhibition of a Janus kinase activity in a patient, comprising administering to the patient a therapeutically effective amount of a compound of Formula (I) or (II) or a stereoisomer, tautomer, solvate, prodrug or salt thereof. In some embodiments, the disease or condition is asthma. In some embodiments, the Janus kinase is JAK1. In some embodiments, a compound is administered via inhalation.

Also provided is a compound selected from Table 1, or a stereoisomer, tautomer, solvate, prodrug or salt thereof, or any combination thereof.

**Table 1: Exemplary Compounds of the Present Invention**

| **Ex.** | **Structure** | **Name** |
|---|---|---|
| 1 | 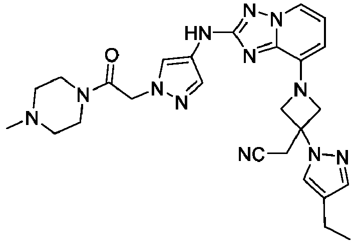 | 2-[3-(4-ethylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **2** | 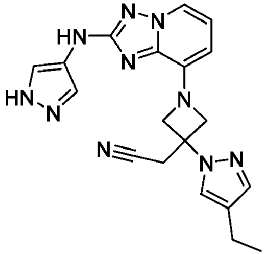 | 2-[3-(4-ethylpyrazol-1-yl)-1-[2-(1H-pyrazol-4-ylamino)-[1,2,4]triazolo[1,5-a]pyridin-8-yl]azetidin-3-yl]acetonitrile |
| **3** | 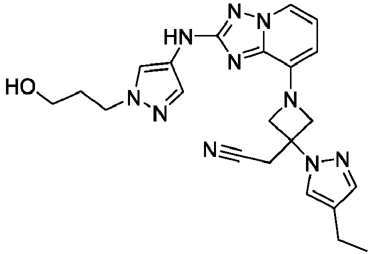 | 2-[3-(4-ethylpyrazol-1-yl)-1-[2-[[1-(3-hydroxypropyl)pyrazol-4-yl]amino]-[1,2,4]triazolo[1,5-a]pyridin-8-yl]azetidin-3-yl]acetonitrile |
| **4** | 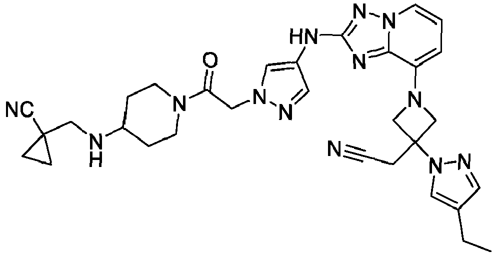 | 1-[[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1- yl]acetyl]-4-piperidyl]amino]methyl]c yclopropanecarbonitrile |
| **5** | 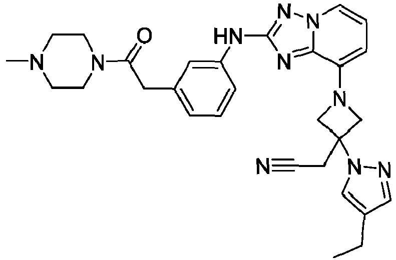 | 2-[3-(4-ethylpyrazol-1-yl)-1-[2-[3-[2-(4-methylpiperazin-1-yl)-2-oxo-ethyl]anilino]-[1,2,4]triazolo[1,5-a]pyridin-8-yl]azetidin-3-yl]acetonitrile |
| **6** | 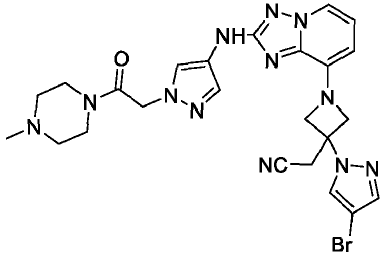 | 2-[3-(4-bromopyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **7** | 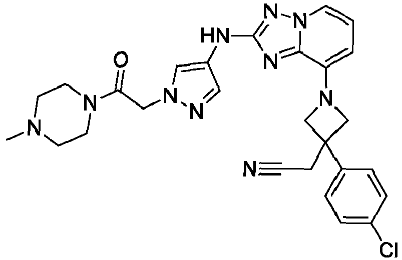 | 2-[3-(4-chlorophenyl)-1- [2-[[1-[2-(4-methylpiperazin-1-yl)-2-oxo-ethyl]pyrazol-4-yl]amino]-[1,2,4]triazolo[1,5-a]pyridin-8-yl]azetidin-3- yl]acetonitrile |
| **8** | 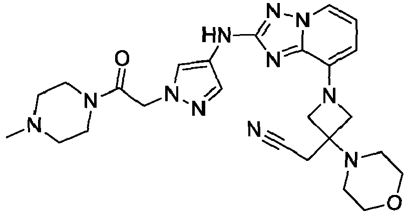 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-morpholino-azetidin-3- yl]acetonitrile |
| **9** | 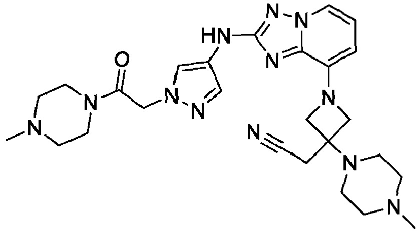 | 2-[3-(4-methylpiperazin-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3- yl]acetonitrile |
| **10** | 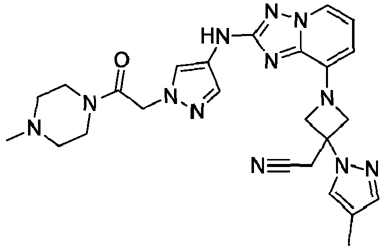 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(4-methylpyrazol-1-yl)azetidin-3-yl]acetonitrile |
| **11** | 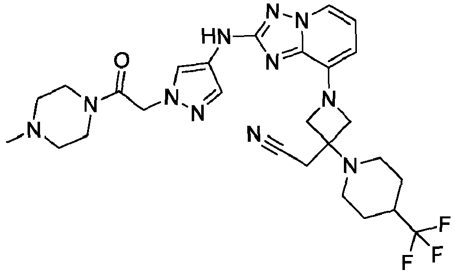 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(trifluoromethyl)-1-piperidyl]azetidin-3-yl]acetonitrile |
| **12** | 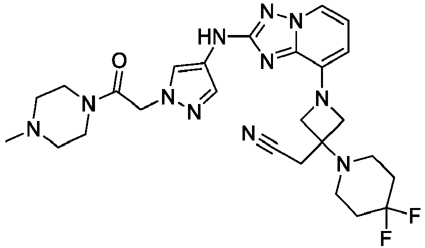 | 2-[3-(4,4-difluoro-1-piperidyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **13** | 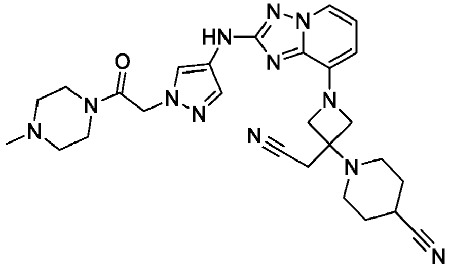 | 1-[3-(cyanomethyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]piperidine-4-carbonitrile |
| **14** | 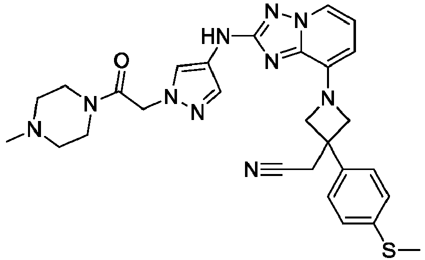 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(4-methylsulfanylphenyl)aze tidin-3-yl]acetonitrile |
| **15** | 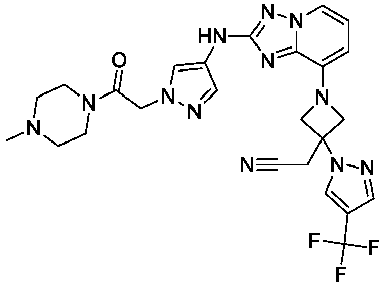 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(trifluoromethyl)pyrazol-1-yl]azetidin-3-yl]acetonitrile |
| **16** | 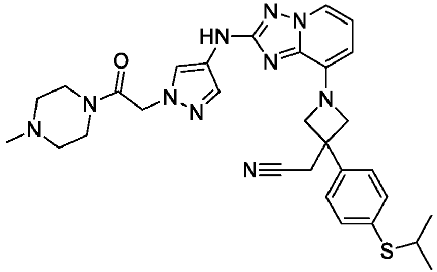 | 2-[3-(4-isopropylsulfanylphenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **17** | 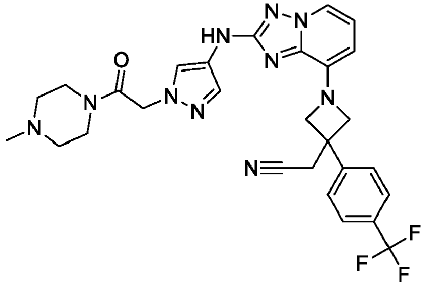 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(trifluoromethyl)phenyl]a zetidin-3-yl]acetonitrile |
| **18** | 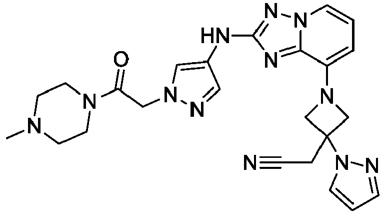 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-pyrazol-1-yl-azetidin-3-yl]acetonitrile |
| **19** | 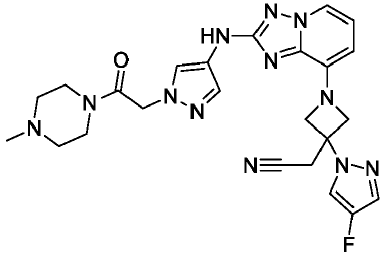 | 2-[3-(4-fluoropyrazol-1- yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **20** | 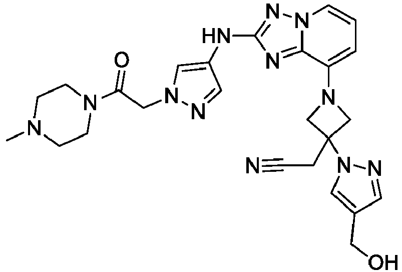 | 2-[3-[4-(hydroxymethyl)pyrazol-1-yl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **21** | 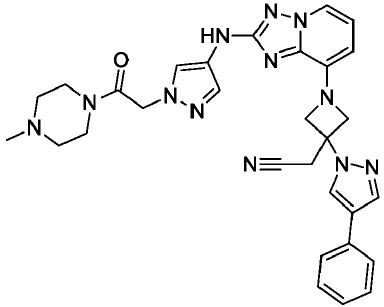 | formicacid;2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(4-phenylpyrazol-1-yl)azetidin-3-yl]acetonitrile |
| **22** | 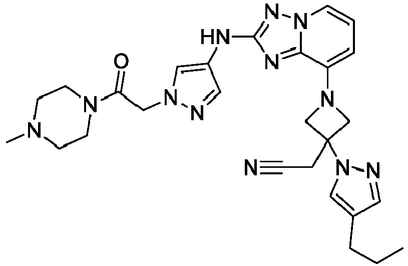 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(4-propylpyrazol-1-yl)azetidin-3-yl]acetonitrile |
| **23** | 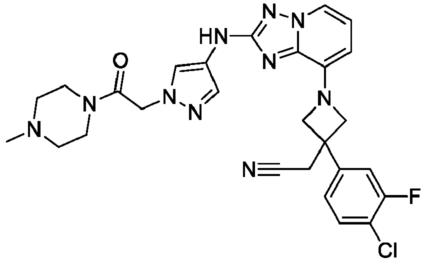 | 2-[3-(4-chloro-3-fluorophenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **24** | 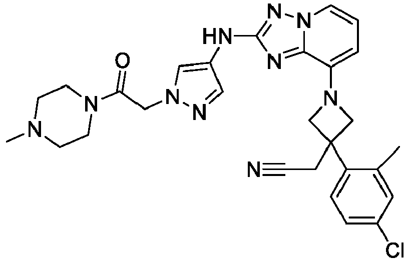 | 2-[3-(4-chloro-2-methylphenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **25** | 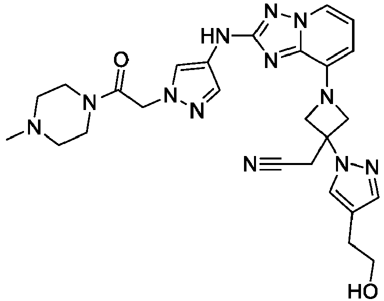 | 2-[3-[4-(2-hydroxyethyl)pyrazol-1- yl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **26** | 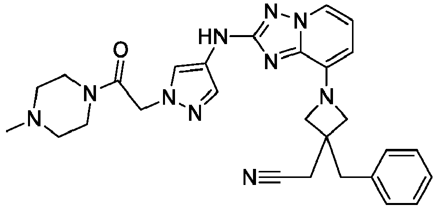 | 2-[3-benzyl-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **27** | 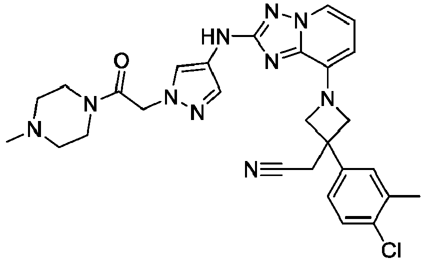 | 2-[3-(4-chloro-3-methylphenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **28** | 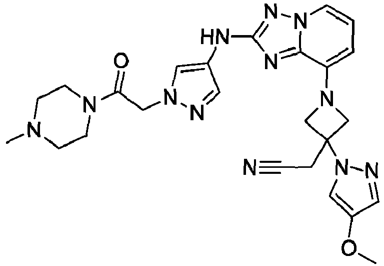 | 2-[3-(4-methoxypyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **29** | 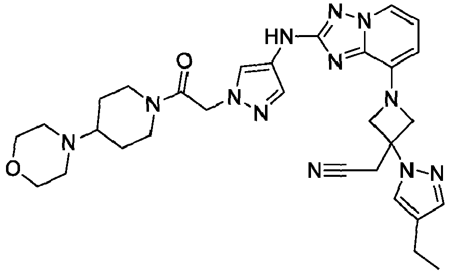 | 2-[3-(4-ethylpyrazol-1- yl)-1-[2-[[1-[2-(4-morpholino-1-piperidyl)-2-oxo-ethyl]pyrazol-4-yl]amino]-[1,2,4]triazolo[1,5-a]pyridin-8-yl]azetidin-3-yl]acetonitrile |
| **30** | 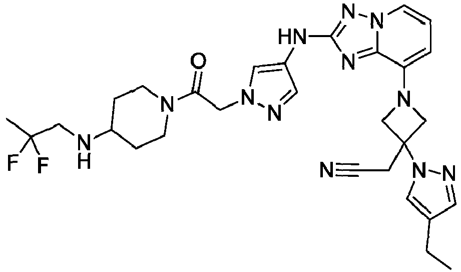 | 2-[1-[2-[[1-[2-[4-(2,2-difluoropropylamino)-1-piperidyl]-2-oxoethyl]pyrazol-4-yl]amino]-[1,2,4]triazolo[1,5-a]pyridin-8-yl]-3-(4-ethylpyrazol-1-yl)azetidin-3-yl]acetonitrile |
| **31** | 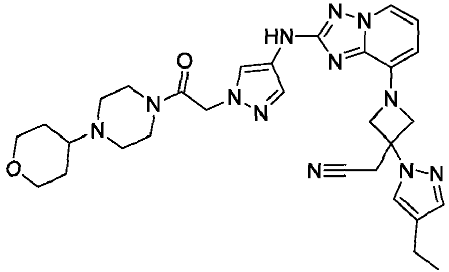 | 2-[3-(4-ethylpyrazol-1- yl)-1-[2-[[1-[2-oxo-2-(4-tetrahydropyran-4-ylpiperazin-1-yl)ethyl]pyrazol-4-yl]amino]-[1,2,4]triazolo[1,5-a]pyridin-8-yl]azetidin-3- yl]acetonitrile |
| **32** | 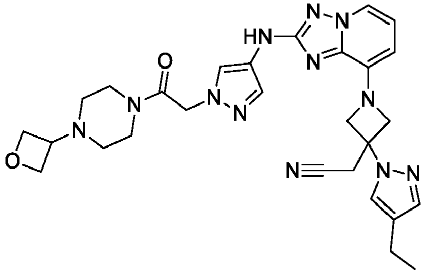 | 2-[3-(4-ethylpyrazol-1-yl)-1-[2-[[1-[2-[4-(oxetan-3-yl)piperazin-1- yl]-2-oxo-ethyl]pyrazol-4-yl]amino]-[1,2,4]triazolo[1,5-a]pyridin-8-yl]azetidin-3-yl]acetonitrile;formicacid |
| **33** | 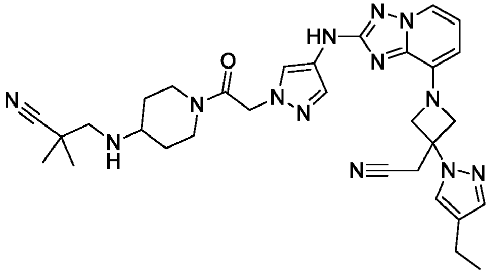 | 3-[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]-4-piperidyl]amino]-2,2-dimethyl-propanenitrile |
| **34** | 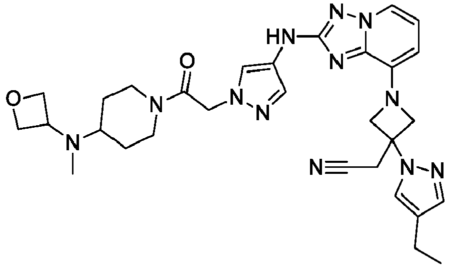 | 2-[3-(4-ethylpyrazol-1- yl)-1-[2-[[1-[2-[4-[methyl(oxetan-3-yl)amino]-1-piperidyl]-2-oxo-ethyl]pyrazol-4-yl]amino]-[1,2,4]triazolo[1,5-a]pyridin-8-yl]azetidin-3-yl]acetonitrile |
| **35** | 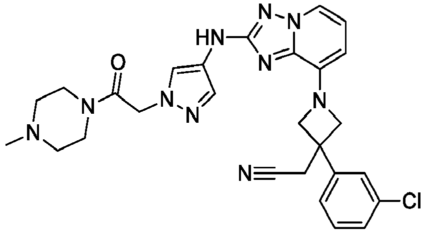 | 2-[3-(3-chlorophenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **36** | 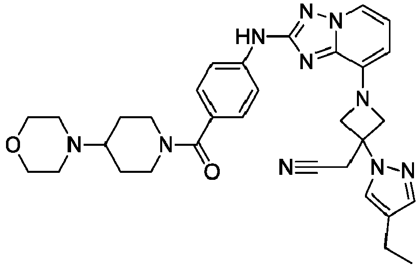 | 2-[3-(4-ethylpyrazol-1- yl)-1-[2-[4-(4-morpholinopiperidine-1-carbonyl)anilino]-[1,2,4]triazolo[1,5-a]pyridin-8-yl]azetidin-3-yl]acetonitrile |
| **37** | 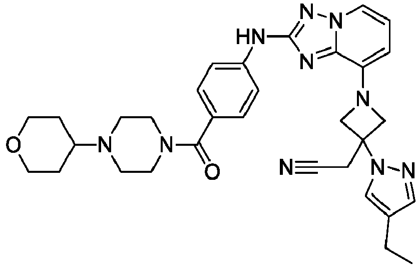 | 2-[3-(4-ethylpyrazol-1-yl)-1-[2-[4-(4-tetrahydropyran-4-ylpiperazine-1-carbonyl)anilino]-[1,2,4]triazolo[1,5-a]pyridin-8-yl]azetidin-3-yl]acetonitrile |
| **38** | 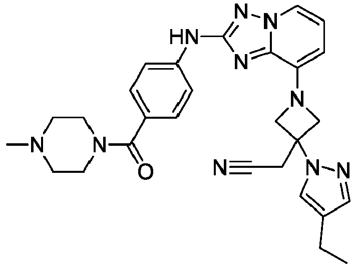 | 2-(3-(4-ethyl-1H-pyrazol-1-yl)-1-(2-((4-(4-methylpiperazine-1-carbonyl)phenyl)amino)-[1,2,4]triazolo[1,5-a]pyridin-8-yl)azetidin-3- yl)acetonitrile |
| **39** | 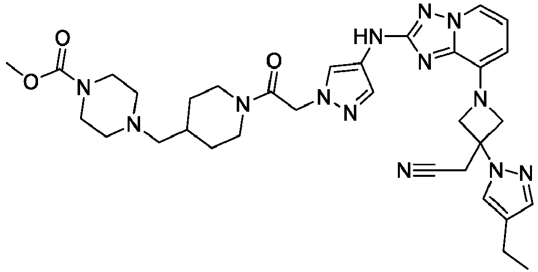 | methyl4-[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]-4-piperidyl]methyl]piperazi ne-1-carboxylate |
| **40** | 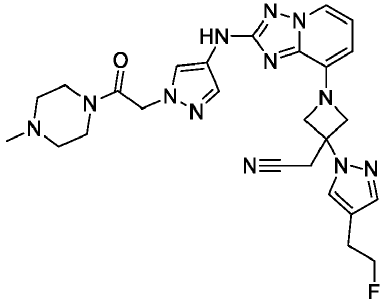 | 2-[3-[4-(2-fluoroethyl)pyrazol-1-yl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **41** | 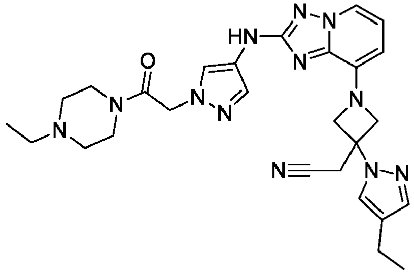 | 2-[1-[2-[[1-[2-(4-ethylpiperazin-1-yl)-2-oxo-ethyl]pyrazol-4-yl]amino]-[1,2,4]triazolo[1,5-a]pyridin-8-yl]-3-(4-ethylpyrazol-1-yl)azetidin-3-yl]acetonitrile |
| **42** | 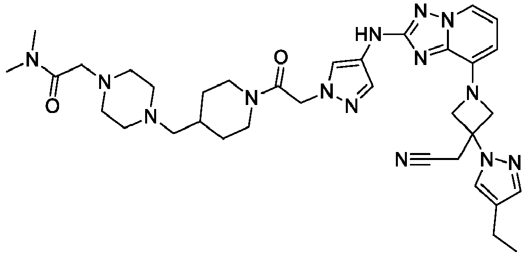 | 2-[4-[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]-4-piperidyl]methyl]piperazi n-1-yl]-N,N-dimethylacetamide |
| **43** | 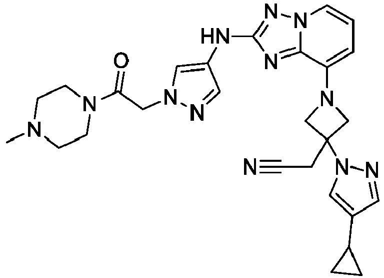 | 2-[3-(4-cyclopropylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **44** | 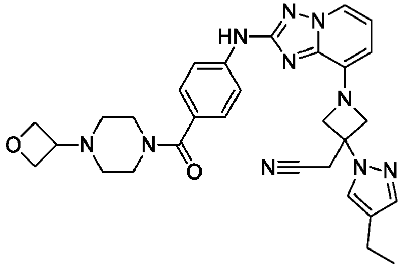 | 2-[3-(4-ethylpyrazol-1- yl)-1-[2-[4-[4-(oxetan-3- yl)piperazine-1-carbonyl]anilino]-[1,2,4]triazolo[1,5-a]pyridin-8-yl]azetidin-3-yl]acetonitrile |
| **45** | 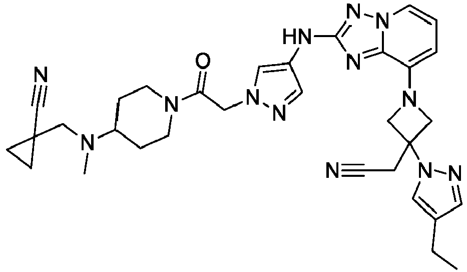 | 1-[[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]-4-piperidyl]-methyl-amino]methyl]cyclopropa necarbonitrile |
| **46** | 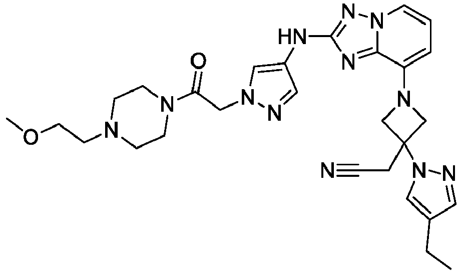 | 2-[3-(4-ethylpyrazol-1- yl)-1-[2-[[1-[2-[4-(2-methoxyethyl)piperazin-1-yl]-2-oxoethyl]pyrazol-4-yl]amino]-[1,2,4]triazolo[1,5-a]pyridin-8-yl]azetidin-3-yl]acetonitrile |
| **47** | 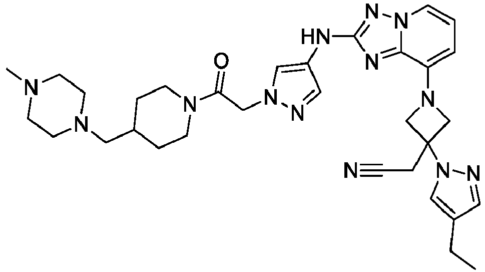 | 2-[3-(4-ethylpyrazol-1-yl)-1-[2-[[1-[2-[4-[(4-methylpiperazin-1- yl)methyl]-1-piperidyl]-2-oxo-ethyl]pyrazol-4-yl]amino]-[1,2,4]triazolo[1,5-a]pyridin-8-yl]azetidin-3-yl]acetonitrile |
| **48** | 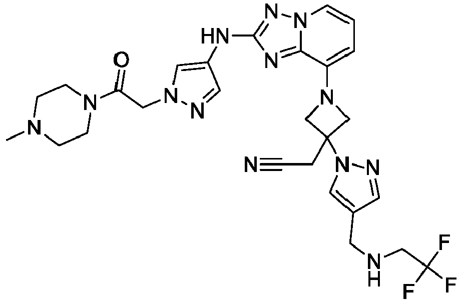 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-[(2,2,2-trifluoroethylamino)meth yl]pyrazol-1-yl]azetidin-3-yl]acetonitrile |
| **49** | 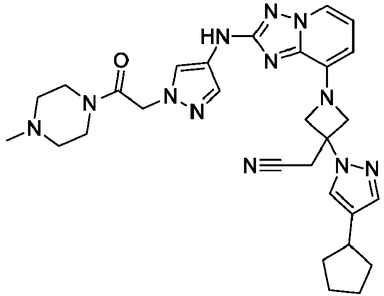 | 2-[3-(4-cyclopentylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **50** | 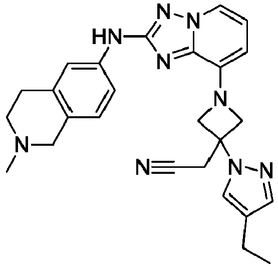 | 2-[3-(4-ethylpyrazol-1-yl)-1-[2-[(2-methyl-3,4-dihydro-1H-isoquinolin-6-yl)amino]-[1,2,4]triazolo[1,5-a]pyridin-8-yl]azetidin-3-yl]acetonitrile |
| **51** | 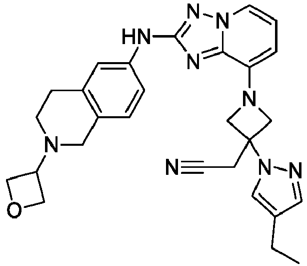 | 2-[3-(4-ethylpyrazol-1-yl)-1-[2-[[2-(oxetan-3-yl)-3,4-dihydro-1H-isoquinolin-6-yl]amino]-[1,2,4]triazolo[1,5-a]pyridin-8-yl]azetidin-3-yl]acetonitrile |
| **52** | 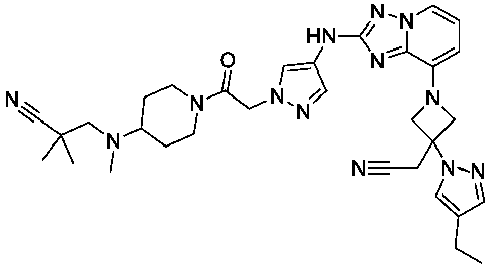 | 3-[[1-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]-4-piperidyl]-methyl-amino]-2,2-dimethyl-propanenitrile |
| **53** | 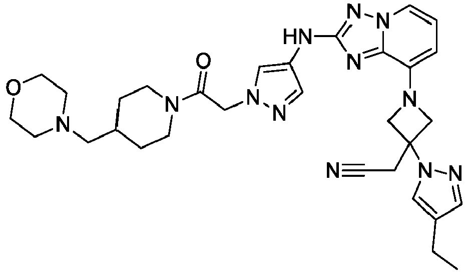 | 2-[3-(4-ethylpyrazol-1 - yl)-1-[2-[[1-[2-[4-(morpholinomethyl)-1 - piperidyl]-2-oxoethyl]pyrazol-4-yl]amino]-[1,2,4]triazolo[1,5-a]pyridin-8-yl]azetidin-3-yl]acetonitrile |
| **54** | 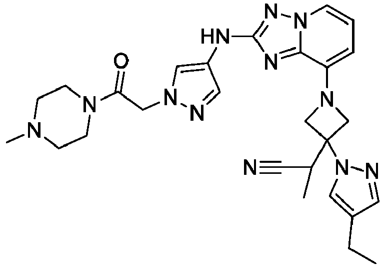 | 2-[3-(4-ethylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]propanenitrile |
| **55** | 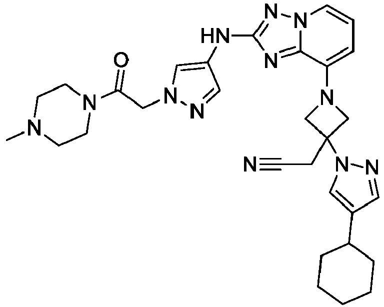 | 2-[3-(4-cyclohexylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **56** | 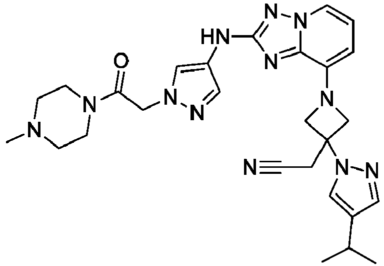 | formic acid;2-[3-(4-isopropylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **57** | 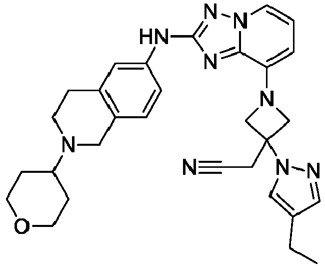 | 2-[3-(4-ethylpyrazol-1-yl)-1-[2-[(2-tetrahydropyran-4-yl-3,4-dihydro-1H-isoquinolin-6-yl)amino]-[1,2,4]triazolo[1,5-a]pyridin-8-yl]azetidin-3-yl]acetonitrile |
| **58** | 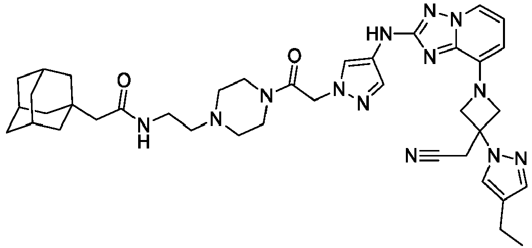 | 2-(1-adamantyl)-N-[2-[4-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]piperazin-1-yl]ethyl]acetamide |
| **59** | 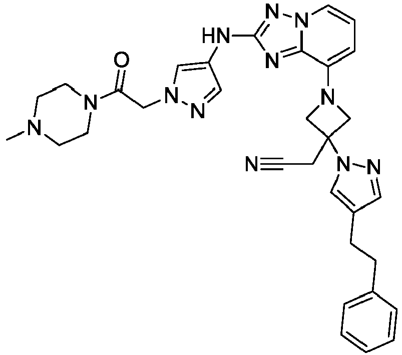 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(2-phenylethyl)pyrazol-1-yl]azetidin-3-yl]acetonitrile |
| **60** | 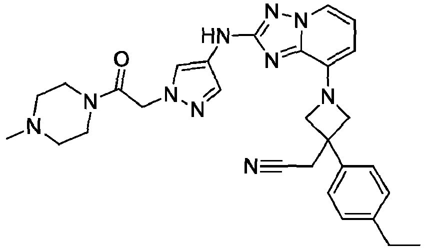 | 2-[3-(4-ethylphenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **61** | 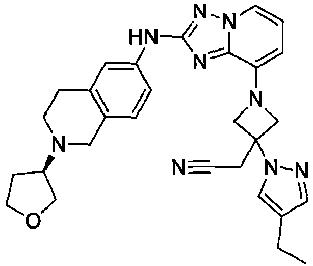 | 2-[3-(4-ethylpyrazol-1- yl)-1-[2-[[2-[rac-(3R)-tetrahydrofuran-3-yl]-3,4-dihydro-1H-isoquinolin-6-yl]amino]-[1,2,4]triazolo[1,5-a]pyridin-8-yl]azetidin-3- yl]acetonitrile |
| **62** | 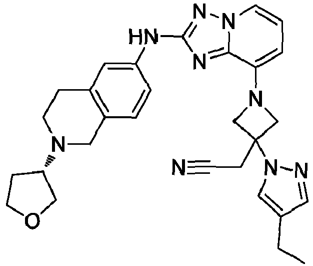 | 2-[3-(4-ethylpyrazol-1- yl)-1-[2-[[2-[rac-(3S)-tetrahydrofuran-3-yl]-3,4-dihydro-1H-isoquinolin-6-yl]amino]-[1,2,4]triazolo[1,5-a]pyridin-8-yl]azetidin-3- yl]acetonitrile |
| **63** | 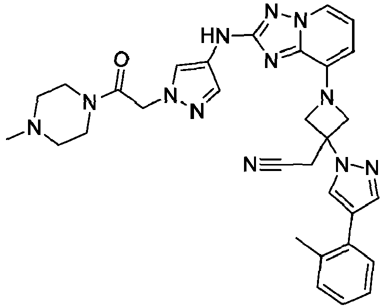 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(o-tolyl)pyrazol-1-yl]azetidin-3-yl]acetonitrile |
| **64** | 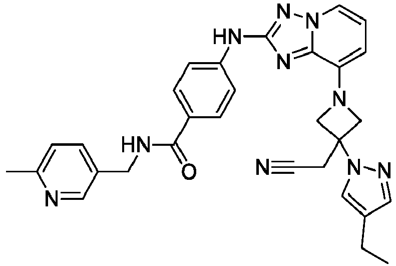 | 4-[[8-[3-(cyanomethyl)-3-(4-ethylpyrazol-1-yl)azetidin-1-yl]-[1,2,4]triazolo[1,5-a]pyridin-2-yl]amino]-N-[(6-methyl-3-pyridyl)methyl]benzamid e |
| **65** | 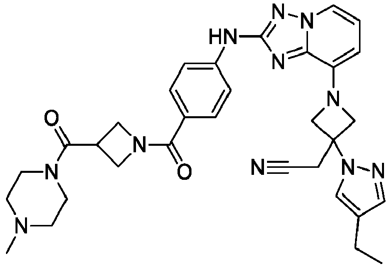 | 2-[3-(4-ethylpyrazol-1-yl)-1-[2-[4-[3-(4-methylpiperazine-1- carbonyl)azetidine-1-carbonyl]anilino]-[1,2,4]triazolo[1,5-a]pyridin-8-yl]azetidin-3-yl]acetonitrile |
| **66** | 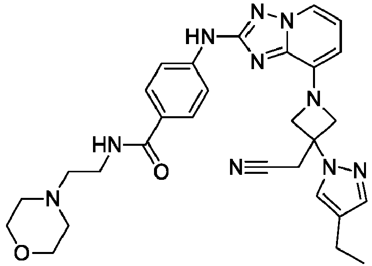 | 4-[[8-[3-(cyanomethyl)-3-(4-ethylpyrazol-1-yl)azetidin-1-yl]-[1,2,4]triazolo[1,5-a]pyridin-2-yl]amino]-N-(2-morpholinoethyl)benzami de |
| **67** | 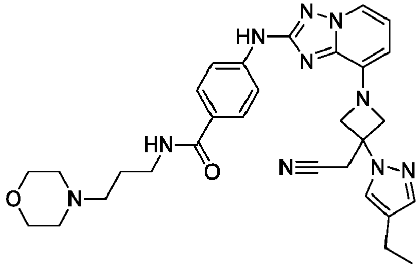 | 4-[[8-[3-(cyanomethyl)-3-(4-ethylpyrazol-1-yl)azetidin-1-yl]-[1,2,4]triazolo[1,5-a]pyridin-2-yl]amino]-N-(3-morpholinopropyl)benza mide |
| **68** | 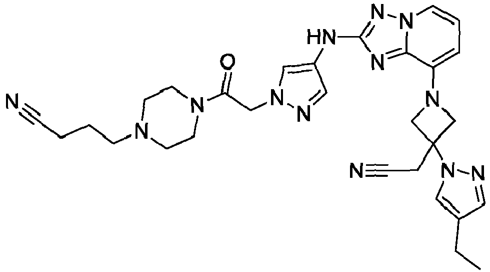 | 4-[4-[2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]acetyl]piperazin-1- yl]butanenitrile |
| **69** | 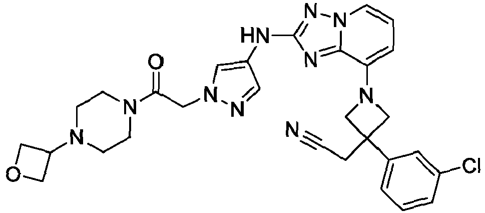 | 2-[3-(3-chlorophenyl)-1-[2-[[1-[2-[4-(oxetan-3-yl)piperazin-1-yl]-2-oxoethyl]pyrazol-4-yl]amino]-[1,2,4]triazolo[1,5-a]pyridin-8-yl]azetidin-3-yl]acetonitrile |
| **70** | 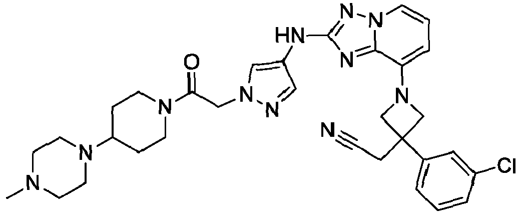 | 2-[3-(3-chlorophenyl)-1-[2-[[1-[2-[4-(4-methylpiperazin-1-yl)-1-piperidyl]-2-oxoethyl]pyrazol-4-yl]amino]-[1,2,4]triazolo[1,5-a]pyridin-8-yl]azetidin-3- yl]acetonitrile |
| **71** | 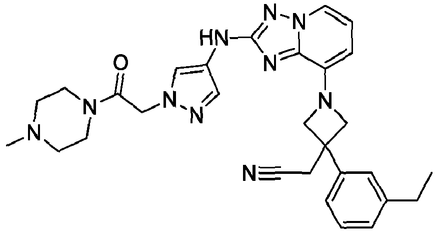 | 2-[3-(3-ethylphenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3- yl]acetonitrile |
| **72** | 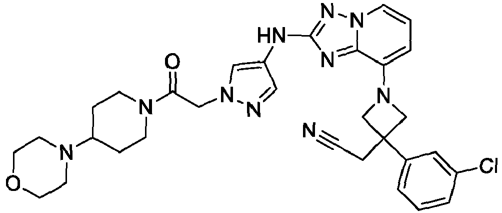 | 2-[3-(3-chlorophenyl)-1-[2-[[1-[2-(4-morpholino-1-piperidyl)-2-oxoethyl]pyrazol-4-yl]amino]-[1,2,4]triazolo[1,5-a]pyridin-8-yl]azetidin-3- yl]acetonitrile |
| **73** | 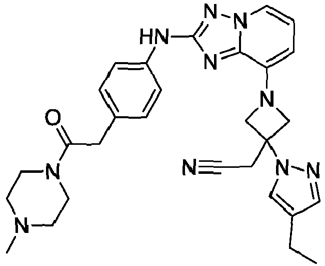 | 2-[3-(4-ethylpyrazol-1-yl)-1-[2-[4-[2-(4-methylpiperazin-1-yl)-2-oxo-ethyl]anilino]-[1,2,4]triazolo[1,5-a]pyridin-8-yl]azetidin-3-yl]acetonitrile |
| **74** | 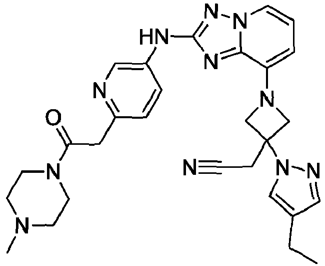 | 2-[3-(4-ethylpyrazol-1- yl)-1-[2-[[6-[2-(4-methylpiperazin-1-yl)-2-oxo-ethyl]-3-pyridyl]amino]-[1,2,4]triazolo[1,5-a]pyridin-8-yl]azetidin-3-yl]acetonitrile |
| **75** | 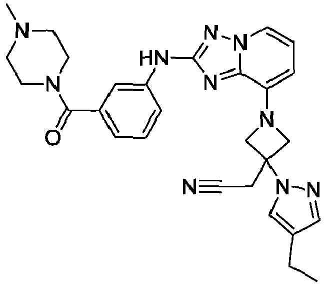 | 2-[3-(4-ethylpyrazol-1- yl)-1-[2-[3-(4-methylpiperazine-1-carbonyl)anilino]-[1,2,4]triazolo[1,5-a]pyridin-8-yl]azetidin-3- yl]acetonitrile |
| **76** | 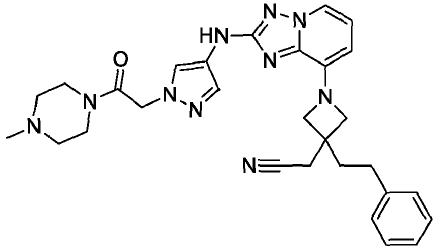 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(2-phenylethyl)azetidin-3- yl]acetonitrile |
| **77** | 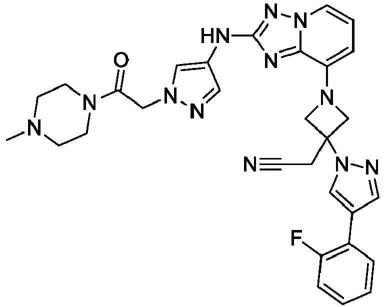 | 2-[3-[4-(2-fluorophenyl)pyrazol-1- yl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **78** | 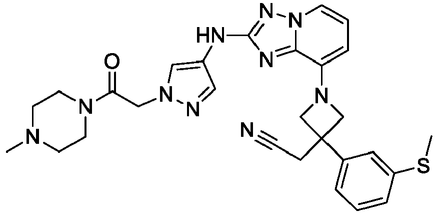 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(3- methylsulfanylphenyl)aze tidin-3-yl]acetonitrile |
| **79** | 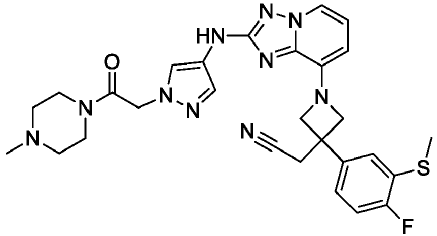 | 2-[3-(4-fluoro-3-methylsulfanyl-phenyl)-1- [2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **80** | 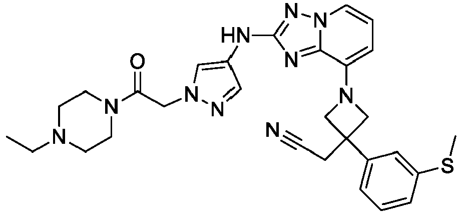 | 2-[1-[2-[[1-[2-(4-ethylpiperazin-1-yl)-2-oxo-ethyl]pyrazol-4-yl]amino]-[1,2,4]triazolo[1,5-a]pyridin-8-yl]-3-(3- methylsulfanylphenyl)aze tidin-3-yl]acetonitrile |
| **81** | 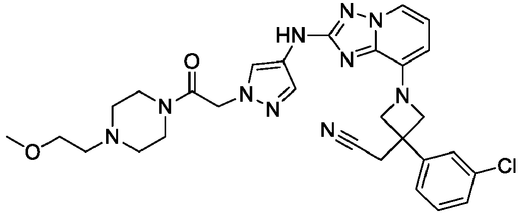 | 2-[3-(3-chlorophenyl)-1-[2-[[1-[2-[4-(2-methoxyethyl)piperazin-1-yl]-2-oxoethyl]pyrazol-4-yl]amino]-[1,2,4]triazolo[1,5-a]pyridin-8-yl]azetidin-3-yl] acetonitrile |
| **82** | 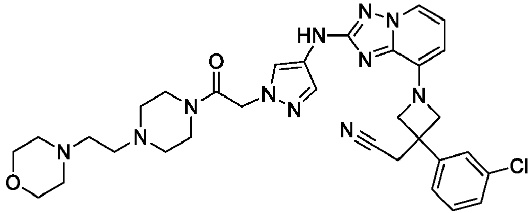 | 2-[3-(3-chlorophenyl)-1-[2-[[1-[2-[4-(2-morpholinoethyl)piperazi n-1-yl]-2-oxo-ethyl]pyrazol-4-yl]amino]-[1,2,4]triazolo[1,5-a]pyridin-8-yl]azetidin-3-yl] acetonitrile |
| **83** | 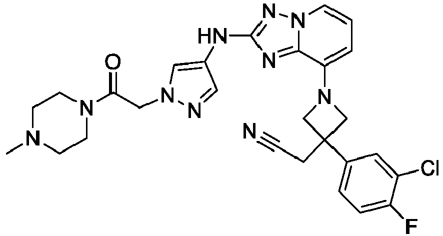 | 2-[3 -(3 -chloro-4-fluorophenyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl] acetonitrile |
| **84** | 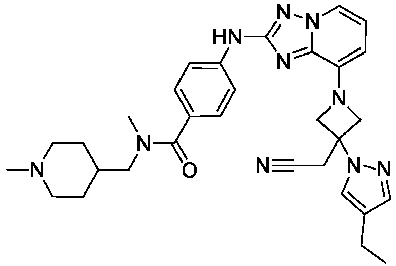 | 4-[[8-[3-(cyanomethyl)-3-(4-ethylpyrazol-1-yl)azetidin-1-yl]-[1,2,4]triazolo[1,5-a]pyridin-2-yl]amino]-N-methyl-N-[(1-methyl-4-piperidyl)methyl]benzami de |
| **85** | 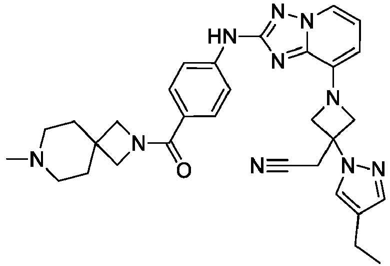 | 2-[3-(4-ethylpyrazol-1-yl)-1-[2-[4-(7-methyl-2,7-diazaspiro[3.5]nonane-2-carbonyl)anilino]-[1,2,4]triazolo[1,5-a]pyridin-8-yl]azetidin-3-yl] acetonitrile |
| **86** | 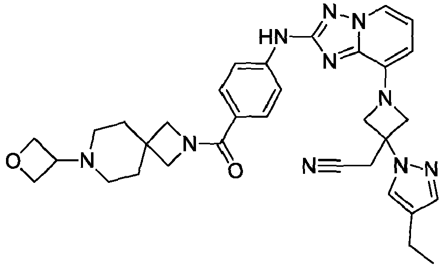 | 2-[3-(4-ethylpyrazol-1-yl)-1-[2-[4-[7-(oxetan-3-yl)-2,7-diazaspiro[3.5]nonane-2-carbonyl]anilino]-[1,2,4]triazolo[1,5-a]pyridin-8-yl]azetidin-3-yl]acetonitrile |
| **87** | 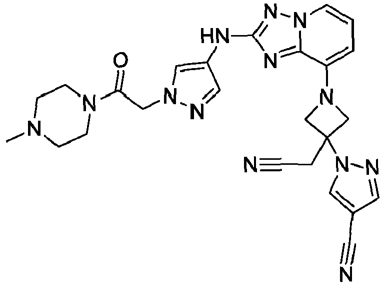 | 1-[3-(cyanomethyl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]pyrazole-4-carbonitrile |
| **88** | 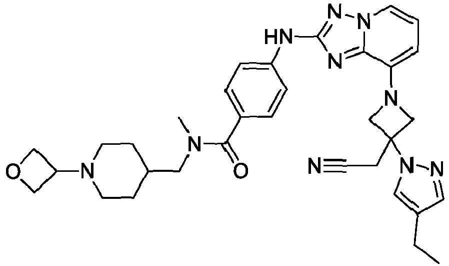 | 4-[[8-[3-(cyanomethyl)-3-(4-ethylpyrazol-1-yl)azetidin-1-yl]-[1,2,4]triazolo[1,5-a]pyridin-2-yl]amino]-N-methyl-N-[[1-(oxetan-3-yl)-4-piperidyl]methyl]benzami de |
| **89** | 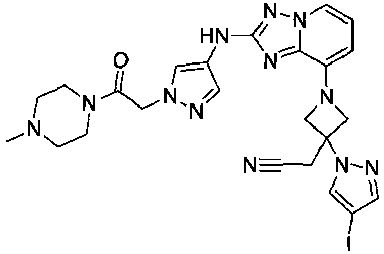 | 2-[3-(4-iodopyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **90** | 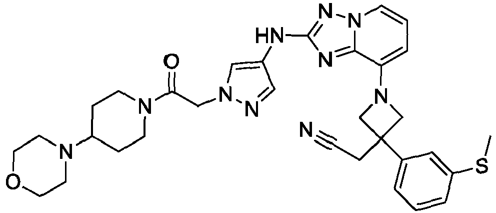 | 2-[3-(3-methylsulfanylphenyl)-1-[2-[[1-[2-(4-morpholino-1-piperidyl)-2-oxo-ethyl]pyrazol-4-yl]amino]-[1,2,4]triazolo[1,5-a]pyridin-8-yl]azetidin-3-yl]acetonitrile |
| **91** | 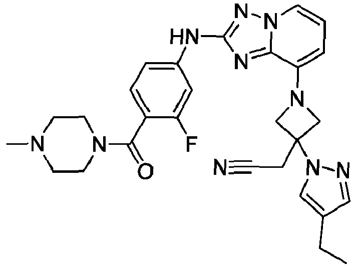 | 2-[3-(4-ethylpyrazol-1-yl)-1-[2-[3-fluoro-4-(4-methylpiperazine-1-carbonyl)anilino]-[1,2,4]triazolo[1,5-a]pyridin-8-yl]azetidin-3-yl]acetonitrile |
| **92** | 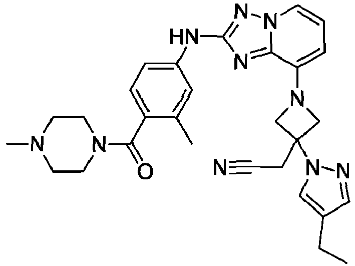 | 2-[3-(4-ethylpyrazol-1-yl)-1-[2-[3-methyl-4-(4-methylpiperazine-1-carbonyl)anilino]-[1,2,4]triazolo[1,5-a]pyridin-8-yl]azetidin-3-yl]acetonitrile |
| **93** | 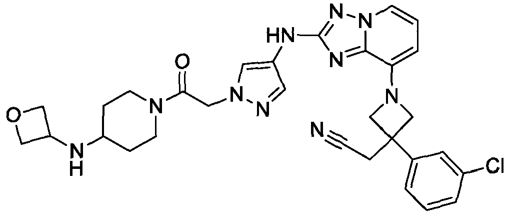 | 2-[3-(3-chlorophenyl)-1-[2-[[1-[2-[4-(oxetan-3-ylamino)-1-piperidyl]-2-oxo-ethyl]pyrazol-4-yl]amino]-[1,2,4]triazolo[1,5-a]pyridin-8-yl]azetidin-3-yl]acetonitrile |
| **94** | 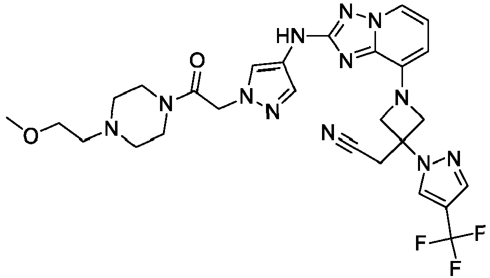 | 2-[1-[2-[[1-[2-[4-(2-methoxyethyl)piperazin-1-yl]-2-oxo-ethyl]pyrazol-4-yl]amino]-[1,2,4]triazolo[1,5-a]pyridin-8-yl]-3-[4-(trifluoromethyl)pyrazol-1-yl]azetidin-3-yl]acetonitrile |
| **95** | 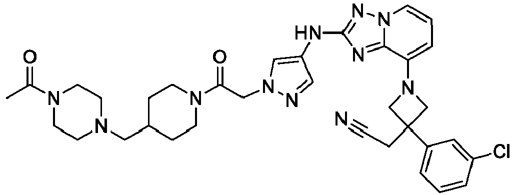 | 2-[1-[2-[[1-[2-[4-[(4-acetylpiperazin-1-yl)methyl]-1-piperidyl]-2-oxo-ethyl]pyrazol-4-yl]amino]-[1,2,4]triazolo[1,5-a]pyridin-8-yl]-3-(3-chlorophenyl)azetidin-3-yl]acetonitrile |
| **96** | 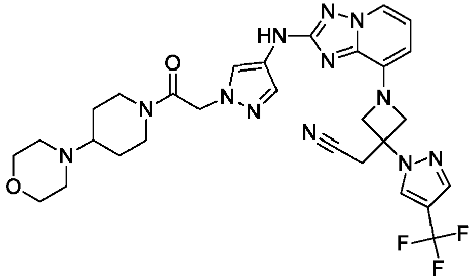 | 2-[1-[2-[[1-[2-(4-morpholino-1-piperidyl)-2-oxo-ethyl]pyrazol-4-yl]amino]-[1,2,4]triazolo[1,5-a]pyridin-8-yl]-3-[4-(trifluoromethyl)pyrazol-1-yl]azetidin-3-yl]acetonitrile |
| **97** | 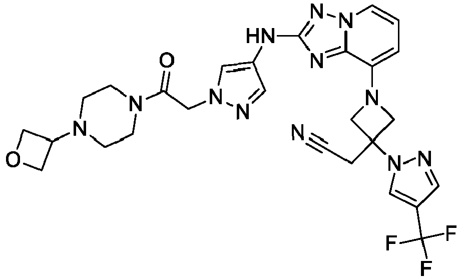 | 2-[1-[2-[[1-[2-[4-(oxetan-3-yl)piperazin-1-yl]-2-oxo-ethyl]pyrazol-4-yl]amino]-[1,2,4]triazolo[1,5-a]pyridin-8-yl]-3-[4-(trifluoromethyl)pyrazol-1-yl]azetidin-3-yl]acetonitrile |
| **98** | 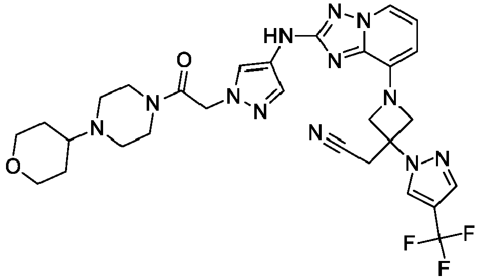 | 2-[1-[2-[[1-[2-oxo-2-(4-tetrahydropyran-4-ylpiperazin-1-yl)ethyl]pyrazol-4-yl]amino]-[1,2,4]triazolo[1,5-a]pyridin-8-yl]-3-[4-(trifluoromethyl)pyrazol-1-yl]azetidin-3-yl]acetonitrile |
| **99** | 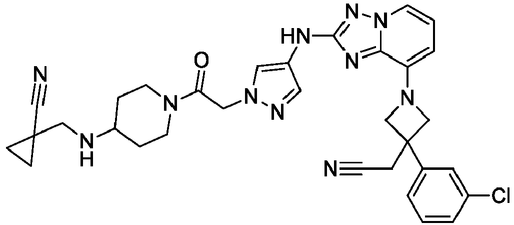 | 1-[[[1-[2-[4-[[8-[3-(3-chlorophenyl)-3-(cyanomethyl)azetidin-1-yl]-[1,2,4]triazolo[1,5-a]pyridin-2-yl]amino]pyrazol-1-yl]acetyl]-4-piperidyl]amino]methyl]c yclopropanecarbonitrile |
| **100** | 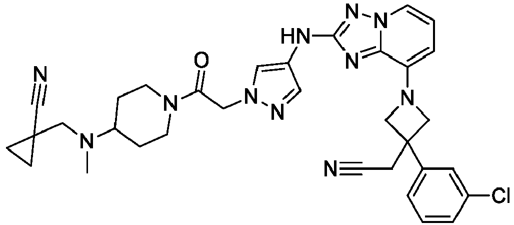 | 1-[[[1-[2-[4-[[8-[3-(3-chlorophenyl)-3-(cyanomethyl)azetidin-1-yl]-[1,2,4]triazolo[1,5-a]pyridin-2-yl]amino]pyrazol-1-yl]acetyl]-4-piperidyl]-methyl-amino]methyl]cyclopropa necarbonitrile |
| **101** | 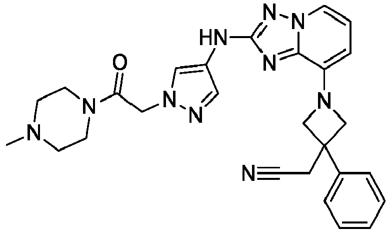 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-phenyl-azetidin-3-yl]acetonitrile |
| **102** | 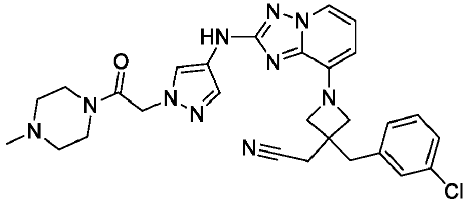 | 2-[3-[(3-chlorophenyl)methyl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **103** | 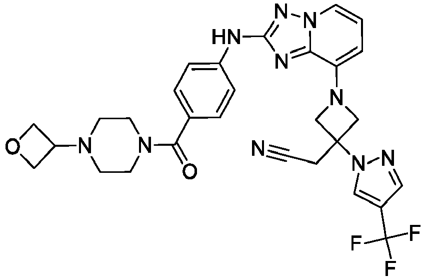 | 2-[1-[2-[4-[4-(oxetan-3-yl)piperazine-1-carbonyl]anilino]-[1,2,4]triazolo[1,5-a]pyridin-8-yl]-3-[4-(trifluoromethyl)pyrazol-1-yl]azetidin-3-yl]acetonitrile |
| **104** | 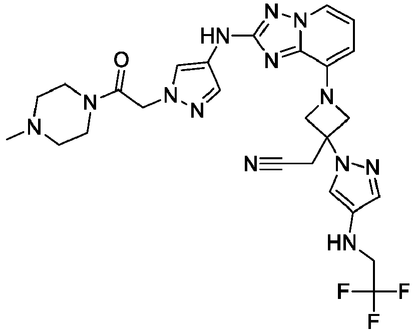 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[4-(2,2,2-trifluoroethylamino)pyraz ol-1-yl]azetidin-3-yl]acetonitrile |
| **105** | 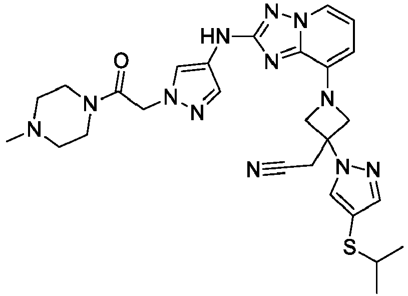 | 2-[3-(4-isopropylsulfanylpyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **106** | 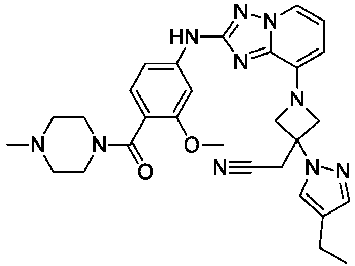 | 2-[3-(4-ethylpyrazol-1-yl)-1-[2-[3-methoxy-4-(4-methylpiperazine-1-carbonyl)anilino]-[1,2,4]triazolo[1,5-a]pyridin-8-yl]azetidin-3-yl]acetonitrile |
| **107** | 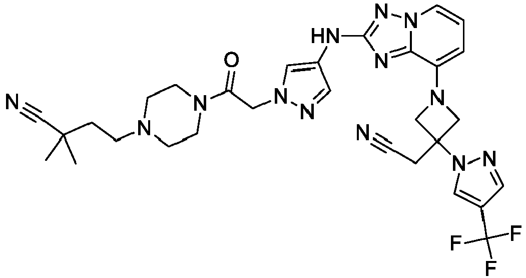 | 4-[4-[2-[4-[[8-[3-(cyanomethyl)-3-[4-(trifluoromethyl)pyrazol-1-yl]azetidin-1-yl]-[1,2,4]triazolo[1,5-a]pyridin-2-yl]amino]pyrazol-1-yl]acetyl]piperazin-1-yl]-2,2-dimethyl-butanenitrile |
| **108** | 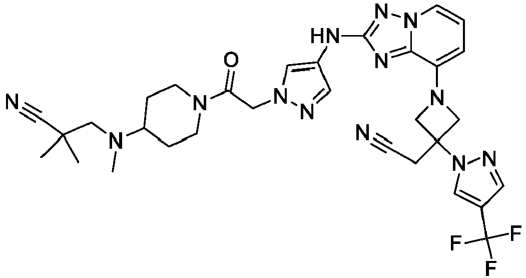 | 3-[[1-[2-[4-[[8-[3-(cyanomethyl)-3-[4-(trifluoromethyl)pyrazol-1-yl]azetidin-1-yl]-[1,2,4]triazolo[1,5-a]pyridin-2-yl]amino]pyrazol-1-yl]acetyl]-4-piperidyl]-methyl-amino]-2,2-dimethyl-propanenitrile;formic acid |
| **109** | 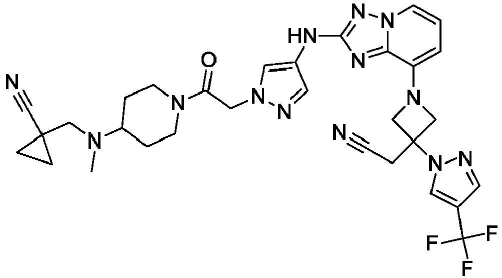 | 1-[[[1-[2-[4-[[8-[3-(cyanomethyl)-3-[4-(trifluoromethyl)pyrazol-1-yl]azetidin-1-yl]-[1,2,4]triazolo[1,5-a]pyridin-2-yl]amino]pyrazol-1-yl]acetyl]-4-piperidyl]-methyl-amino]methyl]cyclopropa necarbonitrile |
| **110** | 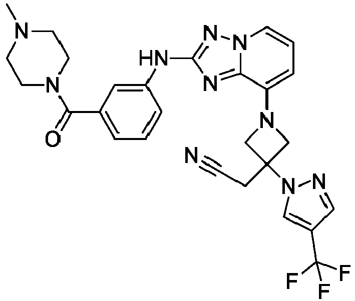 | formicacid;2-[1-[2-[3-(4-methylpiperazine-1-carbonyl)anilino]-[1,2,4]triazolo[1,5-a]pyridin-8-yl]-3-[4-(trifluoromethyl)pyrazol-1-yl]azetidin-3-yl]acetonitrile |
| **111** | 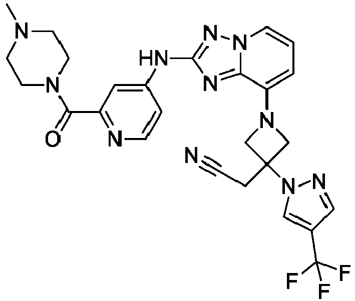 | 2-[1-[2-[[2-(4-methylpiperazine-1-carbonyl)-4-pyridyl]amino]-[1,2,4]triazolo[1,5-a]pyridin-8-yl]-3-[4-(trifluoromethyl)pyrazol-1-yl]azetidin-3-yl] acetonitrile |
| **112** | 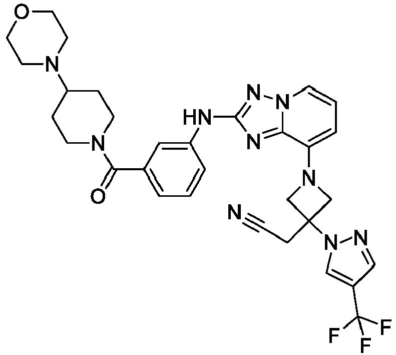 | formic acid;2-[1-[2-[3-(4-morpholinopiperidine-1-carbonyl)anilino]-[1,2,4]triazolo[1,5-a]pyridin-8-yl]-3-[4-(trifluoromethyl)pyrazol-1-yl]azetidin-3-yl] acetonitrile |
| **113** | 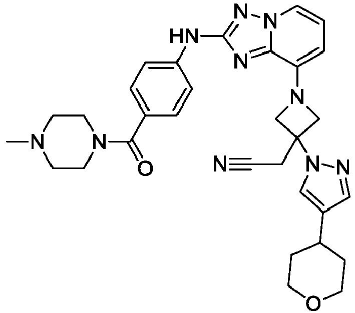 | 2-[1-[2-[4-(4-methylpiperazine-1-carbonyl)anilino]-[1,2,4]triazolo[1,5-a]pyridin-8-yl]-3-(4-tetrahydropyran-4-ylpyrazol-1-yl)azetidin-3-yl] acetonitrile |
| **114** | 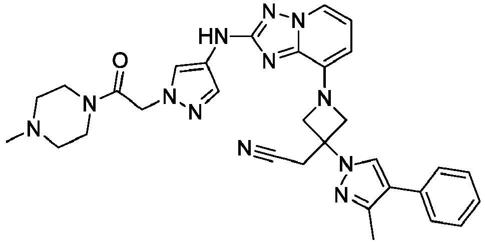 | formic acid;2-[3-(3-methyl-4-phenyl-pyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl] acetonitrile |
| **115** | 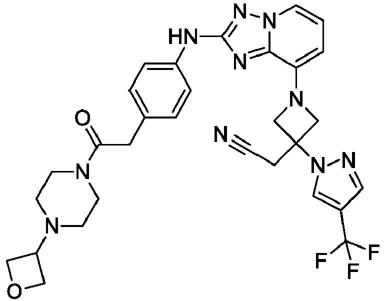 | 2-[1-[2-[4-[2-[4-(oxetan-3-yl)piperazin-1-yl]-2-oxo-ethyl]anilino]-[1,2,4]triazolo[1,5-a]pyridin-8-yl]-3-[4-(trifluoromethyl)pyrazol-1-yl]azetidin-3-yl] acetonitrile |
| **116** | 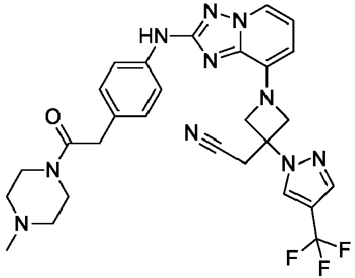 | formic acid;2-[1-[2-[4-[2-(4-methylpiperazin-1-yl)-2-oxo-ethyl]anilino]-[1,2,4]triazolo[1,5-a]pyridin-8-yl]-3-[4-(trifluoromethyl)pyrazol-1-yl]azetidin-3-yl] acetonitrile |
| **117** | 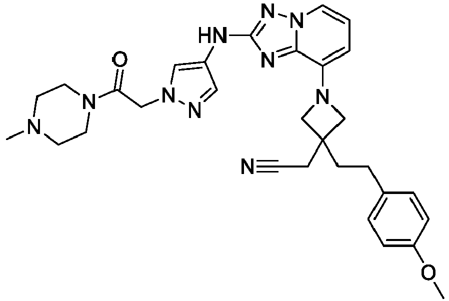 | 2-[3-[2-(4-methoxyphenyl)ethyl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl] acetonitrile |
| **118** | 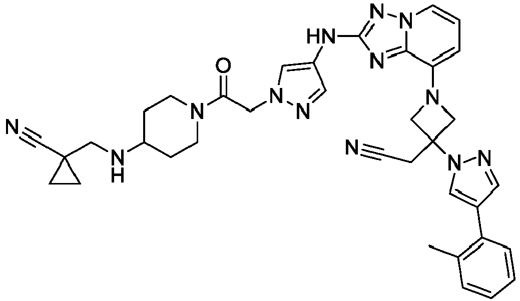 | 1-[[[1-[2-[4-[[8-[3-(cyanomethyl)-3-[4-(o-tolyl)pyrazol-1-yl]azetidin-1-yl]-[1,2,4]triazolo[1,5-a]pyridin-2-yl]amino]pyrazol-1-yl]acetyl]-4-piperidyl]amino]methyl]c yclopropanecarbonitrile |
| **119** | 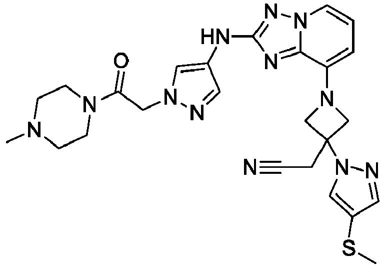 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-(4-methylsulfanylpyrazol-1-yl)azetidin-3-yl] acetonitrile |
| **120** | 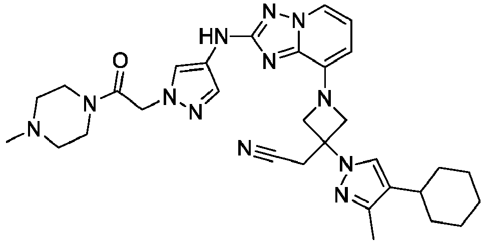 | 2-[3-(4-cyclohexyl-3-methyl-pyrazol-1-yl)-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **121** | 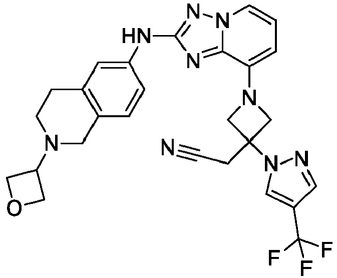 | 2-[1-[2-[[2-(oxetan-3-yl)-3,4-dihydro-1H-isoquinolin-6-yl]amino]-[1,2,4]triazolo[1,5-a]pyridin-8-yl]-3-[4-(trifluoromethyl)pyrazol-1-yl]azetidin-3-yl] acetonitrile |
| **122** | 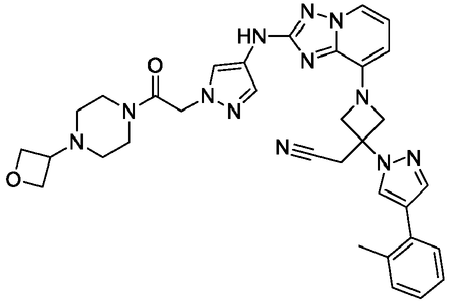 | formic acid;2-[3-[4-(o-tolyl)pyrazol-1-yl]-1-[2-[[1-[2-[4-(oxetan-3-yl)piperazin-1-yl]-2-oxo-ethyl]pyrazol-4-yl]amino]-[1,2,4]triazolo[1,5-a]pyridin-8-yl]azetidin-3-yl] acetonitrile |
| **123** | 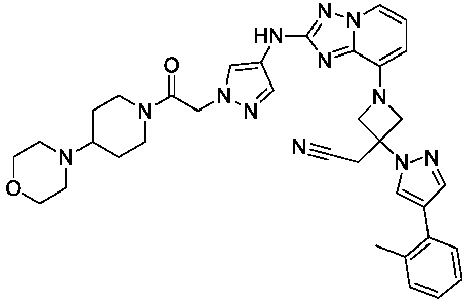 | 2-[1-[2-[[1-[2-(4-morpholino-1-piperidyl)-2-oxo-ethyl]pyrazol-4-yl]amino]-[1,2,4]triazolo[1,5-a]pyridin-8-yl]-3-[4-(o-tolyl)pyrazol-1-yl]azetidin-3-yl]acetonitrile |
| **124** | 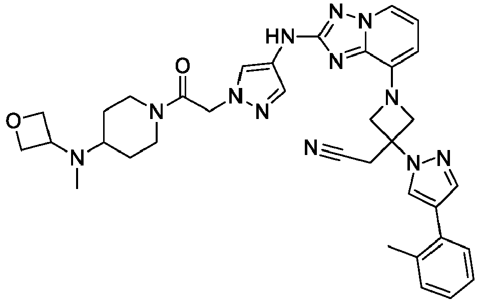 | 2-[1-[2-[[1-[2-[4-[methyl(oxetan-3-yl)amino]-1-piperidyl]-2-oxo-ethyl]pyrazol-4-yl]amino]-[1,2,4]triazolo[1,5-a]pyridin-8-yl]-3-[4-(o-tolyl)pyrazol-1-yl]azetidin-3-yl] acetonitrile |
| **125** | 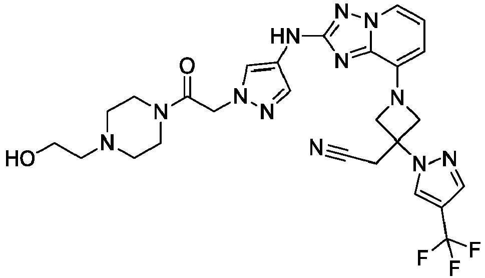 | formic acid;2-[1-[2-[[1-[2-[4-(2-hydroxyethyl)piperazin-1-yl]-2-oxo-ethyl]pyrazol-4-yl]amino]-[1,2,4]triazolo[1,5-a]pyridin-8-yl]-3-[4-(trifluoromethyl)pyrazol-1-yl]azetidin-3-yl] acetonitrile |
| **126** | 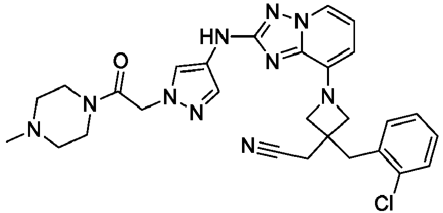 | 2-[3-[(2-chlorophenyl)methyl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl] acetonitrile |
| **127** | 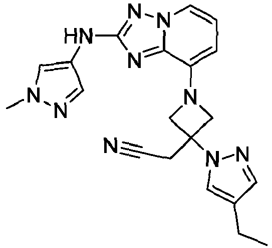 | 2-[3-(4-ethylpyrazol-1-yl)-1-[2-[(1-methylpyrazol-4-yl)amino]-[1,2,4]triazolo[1,5-a]pyridin-8-yl]azetidin-3-yl]acetonitrile |
| **128** | 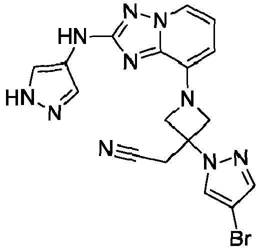 | 2-[3-(4-bromopyrazol-1-yl)-1-[2-(1H-pyrazol-4-ylamino)-[1,2,4]triazolo[1,5-a]pyridin-8-yl]azetidin-3-yl]acetonitrile |
| **129** | 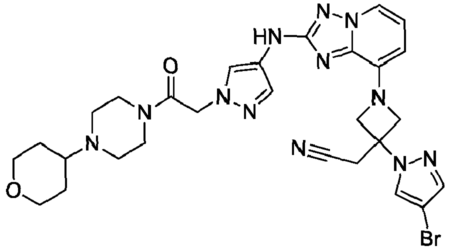 | 2-[3-(4-bromopyrazol-1-yl)-1-[2-[[1-[2-oxo-2-(4-tetrahydropyran-4-ylpiperazin-1-yl)ethyl]pyrazol-4-yl]amino]-[1,2,4]triazolo[1,5-a]pyridin-8-yl]azetidin-3-yl] acetonitrile |
| **130** | 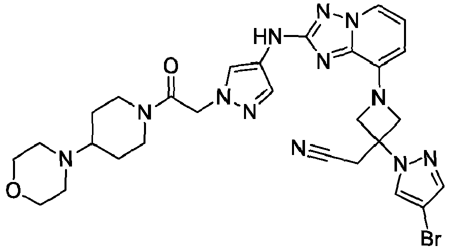 | 2-[3-(4-bromopyrazol-1-yl)-1-[2-[[1-[2-(4-morpholino-1-piperidyl)-2-oxo-ethyl]pyrazol-4-yl]amino]-[1,2,4]triazolo[1,5-a]pyridin-8-yl]azetidin-3-yl] acetonitrile |
| **131** | 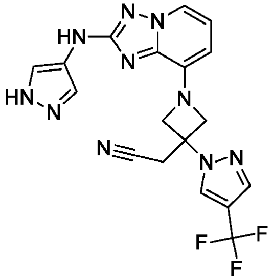 | 2-[1-[2-(1H-pyrazol-4-ylamino)-[1,2,4]triazolo[1,5-a]pyridin-8-yl]-3-[4-(trifluoromethyl)pyrazol-1-yl]azetidin-3-yl] acetonitrile |
| **132** | 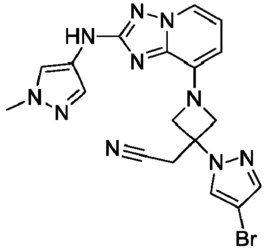 | 2-[3-(4-bromopyrazol-1-yl)-1-[2-[(1-methylpyrazol-4-yl)amino]-[1,2,4]triazolo[1,5-a]pyridin-8-yl]azetidin-3-yl] acetonitrile |
| **133** | 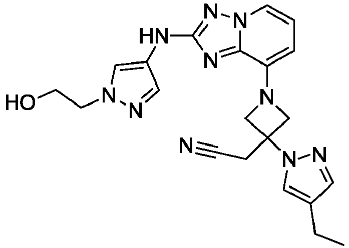 | 2-[3-(4-ethylpyrazol-1-yl)-1-[2-[[1-(2-hydroxyethyl)pyrazol-4-yl]amino]-[1,2,4]triazolo[1,5-a]pyridin-8-yl]azetidin-3-yl] acetonitrile |
| **134** | 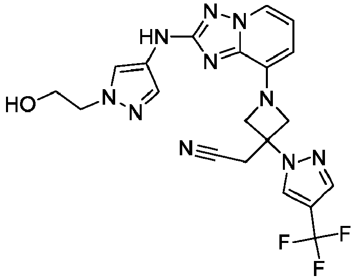 | 2-[1-[2-[[1-(2-hydroxyethyl)pyrazol-4-yl]amino]-[1,2,4]triazolo[1,5-a]pyridin-8-yl]-3-[4-(trifluoromethyl)pyrazol-1-yl]azetidin-3-yl] acetonitrile |
| **135** | 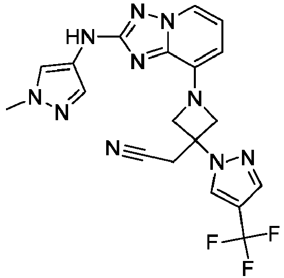 | 2-[1-[2-[(1-methylpyrazol-4-yl)amino]-[1,2,4]triazolo[1,5-a]pyridin-8-yl]-3-[4-(trifluoromethyl)pyrazol-1-yl]azetidin-3-yl] acetonitrile |
| **136** | 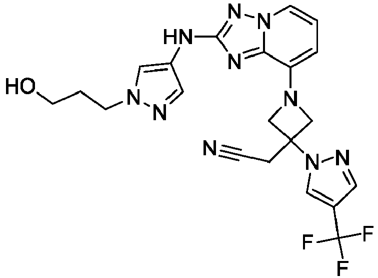 | 2-[1-[2-[[1-(3-hydroxypropyl)pyrazol-4-yl]amino]-[1,2,4]triazolo[1,5-a]pyridin-8-yl]-3-[4-(trifluoromethyl)pyrazol-1-yl]azetidin-3-yl] acetonitrile |
| **137** | 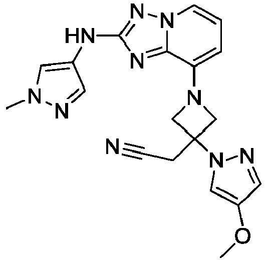 | 2-[3-(4-methoxypyrazol-1-yl)-1-[2-[(1-methylpyrazol-4-yl)amino]-[1,2,4]triazolo[1,5-a]pyridin-8-yl]azetidin-3-yl] acetonitrile |
| **138** | 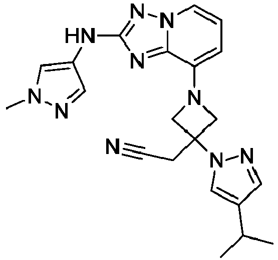 | 2-[3-(4-isopropylpyrazol-1-yl)-1-[2-[(1-methylpyrazol-4-yl)amino]-[1,2,4]triazolo[1,5-a]pyridin-8-yl]azetidin-3-yl] acetonitrile |
| **139** | 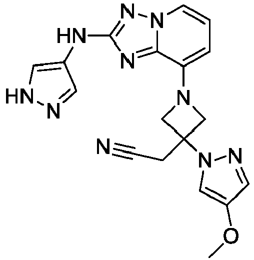 | 2-[3-(4-methoxypyrazol-1-yl)-1-[2-(1H-pyrazol-4-ylamino)-[1,2,4]triazolo[1,5-a]pyridin-8-yl]azetidin-3-yl]acetonitrile |
| **140** | 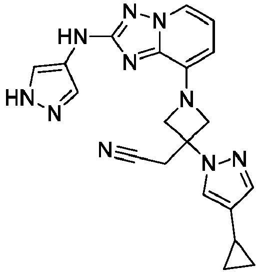 | 2-[3-(4-cyclopropylpyrazol-1-yl)-1-[2-(1H-pyrazol-4-ylamino)-[1,2,4]triazolo[1,5-a]pyridin-8-yl]azetidin-3-yl]acetonitrile |
| **141** | 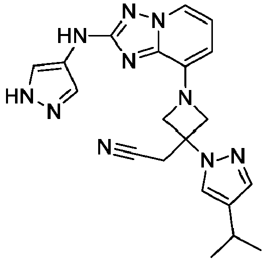 | 2-[3-(4-isopropylpyrazol-1-yl)-1-[2-(1H-pyrazol-4-ylamino)-[1,2,4]triazolo[1,5-a]pyridin-8-yl]azetidin-3-yl]acetonitrile |
| **142** | 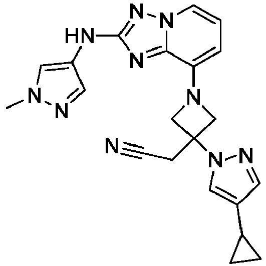 | 2-[3-(4-cyclopropylpyrazol-1-yl)-1-[2-[(1-methylpyrazol-4-yl)amino]-[1,2,4]triazolo[1,5-a]pyridin-8-yl]azetidin-3-yl]acetonitrile |
| **143** | 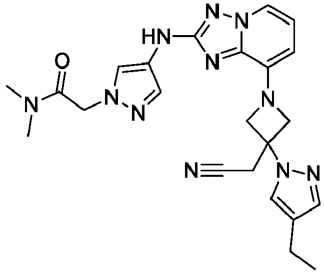 | 2-[4-[[8-[3-(cyanomethyl)-3-(4-ethylpyrazol-1-yl)azetidin-1-yl]-[1,2,4]triazolo[1,5-a]pyridin-2-yl]amino]pyrazol-1-yl]-N,N-dimethyl-acetamide |
| **144** | 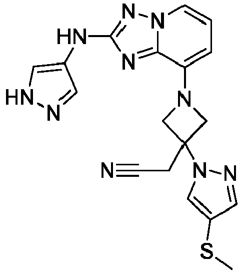 | 2-[3-(4-methylsulfanylpyrazol-1-yl)-1-[2-(1H-pyrazol-4-ylamino)-[1,2,4]triazolo[1,5-a]pyridin-8-yl]azetidin-3-yl]acetonitrile |
| **145** | 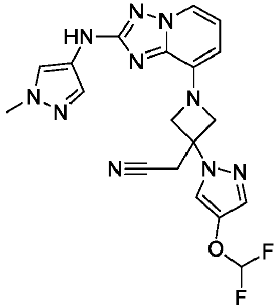 | 2-[3-[4-(difluoromethoxy)pyrazol -1-yl]-1-[2-[(1-methylpyrazol-4-yl)amino]-[1,2,4]triazolo[1,5-a]pyridin-8-yl]azetidin-3-yl]acetonitrile |
| **146** | 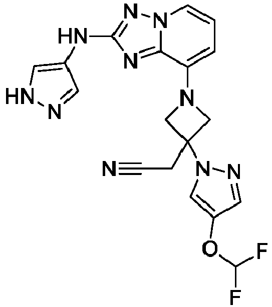 | 2-[3-[4-(difluoromethoxy)pyrazol -1-yl]-1-[2-(1H-pyrazol-4-ylamino)-[1,2,4]triazolo[1,5-a]pyridin-8-yl]azetidin-3-yl]acetonitrile |
| **147** | 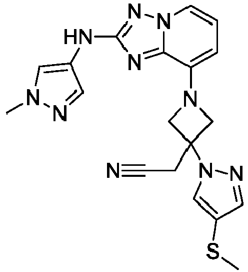 | 2-[1-[2-[(1-methylpyrazol-4-yl)amino]-[1,2,4]triazolo[1,5-a]pyridin-8-yl]-3-(4-methylsulfanylpyrazol-1-yl)azetidin-3-yl] acetonitrile |
| **148** | 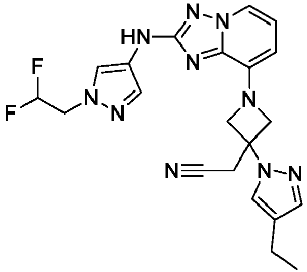 | 2-[1-[2-[[1-(2,2-difluoroethyl)pyrazol-4-yl]amino]-[1,2,4]triazolo[1,5-a]pyridin-8-yl]-3-(4-ethylpyrazol-1-yl)azetidin-3-yl] acetonitrile |
| **149** | 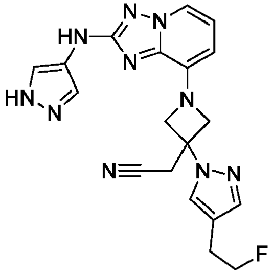 | 2-[3-[4-(2-fluoroethyl)pyrazol-1-yl]-1-[2-(1H-pyrazol-4-ylamino)-[1,2,4]triazolo[1,5-a]pyridin-8-yl]azetidin-3-yl] acetonitrile |
| **150** | 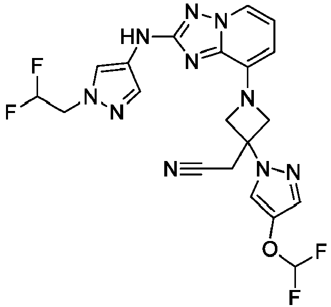 | 2-[1-[2-[[1-(2,2-difluoroethyl)pyrazol-4-yl]amino]-[1,2,4]triazolo[1,5-a]pyridin-8-yl]-3-[4-(difluoromethoxy)pyrazol -1-yl]azetidin-3-yl] acetonitrile |
| **151** | 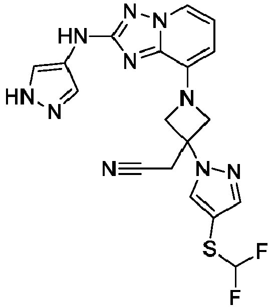 | 2-[3-[4-(difluoromethylsulfanyl)p yrazol-1-yl]-1-[2-(1H-pyrazol-4-ylamino)-[1,2,4]triazolo[1,5-a]pyridin-8-yl]azetidin-3-yl]acetonitrile |
| **152** | 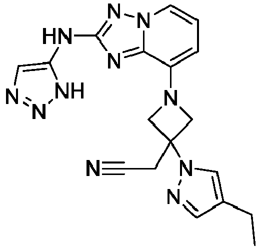 | 2-[3-(4-ethylpyrazol-1-yl)-1-[2-(1H-triazol-5-ylamino)-[1,2,4]triazolo[1,5-a]pyridin-8-yl]azetidin-3-yl]acetonitrile |
| **153** | 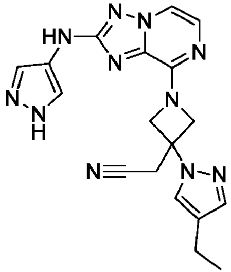 | 2-[3-(4-ethylpyrazol-1-yl)-1-[2-(1H-pyrazol-4-ylamino)-[1,2,4]triazolo[1,5-a]pyrazin-8-yl]azetidin-3-yl]acetonitrile |
| **154** | 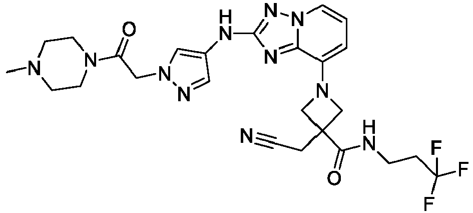 | 3-(cyanomethyl)-1-[2-[[1-[2-(4-methylpiperazin-1-yl)-2-oxo-ethyl]pyrazol-4-yl]amino]-[1,2,4]triazolo[1,5-a]pyridin-8-yl]-N-(3,3,3-trifluoropropyl)azetidine-3-carboxamide |
| **155** | 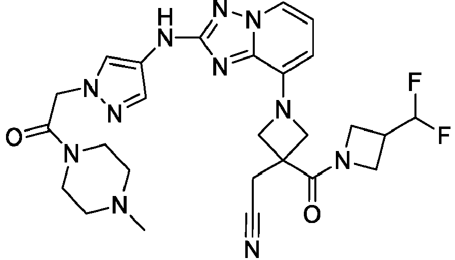 | 2-[3-[3-(difluoromethyl)azetidine-1-carbonyl]-1-[2-[[1-[2-(4-methylpiperazin-1-yl)-2-oxo-ethyl]pyrazol-4-yl]amino]-[1,2,4]triazolo[1,5-a]pyridin-8-yl]azetidin-3-yl]acetonitrile |
| **156** | 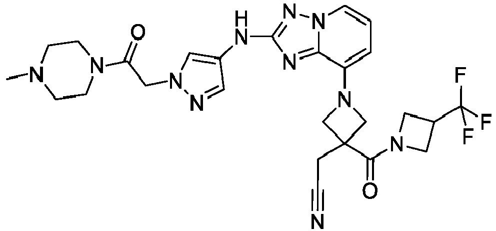 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[3-(trifluoromethyl)azetidine-1-carbonyl]azetidin-3-yl]acetonitrile |
| **157** | 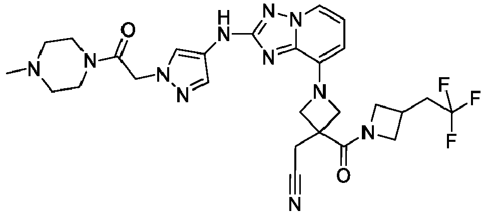 | 2-[1-[2-[[1-[2-(4-methylpiperazin-1-yl)-2-oxo-ethyl]pyrazol-4-yl]amino]-[1,2,4]triazolo[1,5-a]pyridin-8-yl]-3-[3-(2,2,2-trifluoroethyl)azetidine-1-carbonyl]azetidin-3-yl]acetonitrile |

Compounds of the invention may contain one or more asymmetric carbon atoms. Accordingly, the compounds may exist as diastereomers, enantiomers or mixtures thereof. The syntheses of the compounds may employ racemates, diastereomers or enantiomers as starting materials or as intermediates. Mixtures of particular diastereomeric compounds may be separated, or enriched in one or more particular diastereomers, by chromatographic or crystallization methods. Similarly, enantiomeric mixtures may be separated, or enantiomerically enriched, using the same techniques or others known in the art. Each of the asymmetric carbon or nitrogen atoms may be in the R or S configuration and both of these configurations are within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined. Unless otherwise specified, if solid wedges or dashed lines are used, relative stereochemistry is intended.

Another aspect includes prodrugs of the compounds of the present invention, such as a compound of Formula (I) or (II), or a stereoisomer, tautomer, solvate, prodrug or salt thereof, including known amino-protecting and carboxy-protecting groups which are released, for example hydrolyzed, to yield the compound of the present invention under physiologic conditions.

The term "prodrug" refers to a precursor or derivative form of a pharmaceutically active substance that is less efficacious to the patient compared to the parent drug and is capable of being enzymatically or hydrolytically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, and 5-fluorocytosine and 5-fluorouridine prodrugs.

A particular class of prodrugs are compounds in which a nitrogen atom in an amino, amidino, aminoalkyleneamino, iminoalkyleneamino or guanidino group is substituted with a hydroxy group, an alkylcarbonyl (-CO-R) group, an alkoxycarbonyl (-CO-OR), or an acyloxyalkyl-alkoxycarbonyl (-CO-O-R-O-CO-R) group where R is a monovalent or divalent group, for example alkyl, alkylene or aryl, or a group having the Formula -C(O)-O-CP1P2-haloalkyl, where P1 and P2 are the same or different and are hydrogen, alkyl, alkoxy, cyano, halogen, alkyl or aryl. In a particular embodiment, the nitrogen atom is one of the nitrogen atoms of the amidino group of the compounds of Formula (I) or (II) or a subformula thereof. Prodrugs may be prepared by reacting a compound of the present invention, such as a compound of Formula (I) or (II), or a stereoisomer, tautomer, solvate, prodrug or salt thereof, with an activated group, such as acyl groups, to bond, for example, a nitrogen atom in the compound to the exemplary carbonyl of the activated acyl group. Examples of activated carbonyl compounds are those containing a leaving group bonded to the carbonyl group, and include, for example, acyl halides, acyl amines, acyl pyridinium salts, acyl alkoxides, acyl phenoxides such as p-nitrophenoxy acyl, dinitrophenoxy acyl, fluorophenoxy acyl, and difluorophenoxy acyl. The reactions are generally carried out in inert solvents at reduced temperatures such as -78 to about 50°C. The reactions may also be carried out in the presence of an inorganic base, for example potassium carbonate or sodium bicarbonate, or an organic base such as an amine, including pyridine, trimethylamine, triethylamine, triethanolamine, or the like.

Additional types of prodrugs are also encompassed. For instance, a free carboxyl group of a compound of the invention, such as a compound of Formula (I) or (II), can be derivatized as an amide or alkyl ester. As another example, compounds of the present invention comprising free hydroxy groups can be derivatized as prodrugs by converting the hydroxy group into a group such as, but not limited to, a phosphate ester, hemisuccinate, dimethylaminoacetate, or phosphoryloxymethyloxycarbonyl group, as outlined in Fleisher, D. et al., (1996) Improved oral drug delivery: solubility limitations overcome by the use of prodrugs Advanced Drug Delivery Reviews, 19:115. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers, wherein the acyl group can be an alkyl ester optionally substituted with groups including, but not limited to, ether, amine and carboxylic acid functionalities, or where the acyl group is an amino acid ester as described above, are also encompassed. Prodrugs of this type are described in J. Med. Chem., (1996), 39: 10. More specific examples include replacement of the hydrogen atom of the alcohol group with a group such as (C₁₋C₆)alkanoyloxymethyl, 1-((C₁₋C₆)alkanoyloxy)ethyl, 1-methyl-1-((C₁₋C₆)alkanoyloxy)ethyl, (C₁₋C₆)alkoxycarbonyloxymethyl, N-(C₁₋C₆)alkoxycarbonylaminomethyl, succinoyl, (C₁₋C₆)alkanoyl, alpha-amino(C₁₋C₄)alkanoyl, arylacyl and alpha-aminoacyl, or alpha-aminoacyl-alpha-aminoacyl, where each alpha-aminoacyl group is independently selected from the naturally occurring L-amino acids, P(O)(OH)₂, -P(O)(O(C₁₋C₆)alkyl)₂ or glycosyl (the radical resulting from the removal of a hydroxyl group of the hemiacetal form of a carbohydrate).

"Leaving group" refers to a portion of a first reactant in a chemical reaction that is displaced from the first reactant in the chemical reaction. Examples of leaving groups include, but are not limited to, halogen atoms, alkoxy and sulfonyloxy groups. Example sulfonyloxy groups include, but are not limited to, alkylsulfonyloxy groups (for example methyl sulfonyloxy (mesylate group) and trifluoromethylsulfonyloxy (triflate group)) and arylsulfonyloxy groups (for example p-toluenesulfonyloxy (tosylate group) and p-nitrosulfonyloxy (nosylate group)).

### SYNTHESIS OF JANUS KINASE INHIBITOR COMPOUNDS

Compounds of the present invention may be synthesized by synthetic routes described herein. In certain embodiments, processes well-known in the chemical arts can be used, in addition to, or in light of, the description contained herein. The starting materials are generally available from commercial sources such as Aldrich Chemicals (Milwaukee, Wis.) or are readily prepared using methods well known to those skilled in the art (e.g., prepared by methods generally described in Louis F. Fieser and Mary Fieser, Reagents for Organic Synthesis, v. 1-19, Wiley, N.Y. (1967-1999 ed.), Beilsteins Handbuch der organischen Chemie, 4, Aufl. ed. Springer-Verlag, Berlin, including supplements (also available via the Beilstein online database)), or Comprehensive Heterocyclic Chemistry, Editors Katrizky and Rees, Pergamon Press, 1984.

Compounds may be prepared singly or as compound libraries comprising at least 2, for example 5 to 1,000 compounds, or 10 to 100 compounds. Libraries of compounds may be prepared by a combinatorial 'split and mix' approach or by multiple parallel syntheses using either solution phase or solid phase chemistry, by procedures known to those skilled in the art. Thus according to a further aspect of the invention there is provided a compound library comprising at least 2 compounds of the present invention, such as a compound of Formula (I) or (II).

For illustrative purposes, reaction Schemes depicted below provide routes for synthesizing the compounds of the present invention as well as key intermediates. For a more detailed description of the individual reaction steps, see the Examples section below. Those skilled in the art will appreciate that other synthetic routes may be used. Although some specific starting materials and reagents are depicted in the Schemes and discussed below, other starting materials and reagents can be substituted to provide a variety of derivatives or reaction conditions. In addition, many of the compounds prepared by the methods described below can be further modified in light of this disclosure using conventional chemistry well known to those skilled in the art.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups include acetyl, trifluoroacetyl, benzyl, phenylsulfonyl, t-butoxycarbonyl (BOC), benzyloxycarbonyl (CBz) and 9-fluorenylmethyleneoxycarbonyl (Fmoc). The need for such protection is readily determined by one skilled in the art. For a general description of protecting groups and their use, see T. W. Greene, Protective Groups in Organic Synthesis, John Wiley & Sons, New York, 1991.

Other conversions commonly used in the synthesis of compounds of the present invention, and which can be carried out using a variety of reagents and conditions, include the following:
(1) Reaction of a carboxylic acid with an amine to form an amide. Such a transformation can be achieved using various reagents known to those skilled in the art but a comprehensive review can be found in Tetrahedron, 2005, 61, 10827-10852.
(2) Reaction of a primary or secondary amine with an aryl halide or pseudo halide, e.g., a triflate, commonly known as a "Buchwald-Hartwig cross-coupling," can be achieved using a variety of catalysts, ligands and bases. A review of these methods is provided in Comprehensive Organic Name Reactions and Reagents, 2010, 575-581.
(3) A palladium cross-coupling reaction between an aryl halide and a vinyl boronic acid or boronate ester. This transformation is a type of "Suzuki-Miyaura cross-coupling," a class of reaction that has been thoroughly reviewed in Chemical Reviews, 1995, 95(7), 2457-2483.
(4) The hydrolysis of an ester to give the corresponding carboxylic acid is well known to those skilled in the art and conditions include: for methyl and ethyl esters, the use of a strong aqueous base such as lithium, sodium or potassium hydroxide or a strong aqueous mineral acid such as HCl; for a tert-butyl ester, hydrolysis would be carried out using acid, for example, HCl in dioxane or trifluoroacetic acid (TFA) in dichloromethane (DCM).

It will be appreciated that where appropriate functional groups exist, compounds of various formulae or any intermediates used in their preparation may be further derivatised by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

In a further example, primary amine or secondary amine groups may be converted into amide groups (-NHCOR' or -NRCOR') by acylation. Acylation may be achieved by reaction with an appropriate acid chloride in the presence of a base, such as triethylamine, in a suitable solvent, such as dichloromethane, or by reaction with an appropriate carboxylic acid in the presence of a suitable coupling agent such HATU (O-(7-azabenzotriazol-1-yl)-N,N,N',N'-tetramethyluronium hexafluorophosphate) in a suitable solvent such as dichloromethane. Similarly, amine groups may be converted into sulphonamide groups (-NHSO₂R' or -NR"SO₂R') groups by reaction with an appropriate sulphonyl chloride in the presence of a suitable base, such as triethylamine, in a suitable solvent such as dichloromethane. Primary or secondary amine groups can be converted into urea groups (-NHCONR'R" or -NRCONR'R") by reaction with an appropriate isocyanate in the presence of a suitable base such as triethylamine, in a suitable solvent, such as dichloromethane.

An amine (-NH₂) may be obtained by reduction of a nitro (-NO₂) group, for example by catalytic hydrogenation, using for example hydrogen in the presence of a metal catalyst, for example palladium on a support such as carbon in a solvent such as ethyl acetate or an alcohol e.g. methanol. Alternatively, the transformation may be carried out by chemical reduction using for example a metal, e.g. tin or iron, in the presence of an acid such as hydrochloric acid.

In a further example, amine (-CH₂NH₂) groups may be obtained by reduction of nitriles (-CN), for example by catalytic hydrogenation using for example hydrogen in the presence of a metal catalyst, for example palladium on a support such as carbon, or Raney nickel, in a solvent such as an ether e.g. a cyclic ether such as tetrahydrofuran, at an appropriate temperature, for example from about -78 °C to the reflux temperature of the solvent.

In a further example, amine (-NH₂) groups may be obtained from carboxylic acid groups (-CO₂H) by conversion to the corresponding acyl azide (-CON₃), Curtius rearrangement and hydrolysis of the resultant isocyanate (-N=C=O).

Aldehyde groups (-CHO) may be converted to amine groups (-CH₂NR'R")) by reductive amination employing an amine and a borohydride, for example sodium triacetoxyborohydride or sodium cyanoborohydride, in a solvent such as a halogenated hydrocarbon, for example dichloromethane, or an alcohol such as ethanol, where necessary in the presence of an acid such as acetic acid at around ambient temperature.

In a further example, aldehyde groups may be converted into alkenyl groups (-CH=CHR') by the use of a Wittig or Wadsworth-Emmons reaction using an appropriate phosphorane or phosphonate under standard conditions known to those skilled in the art.

Aldehyde groups may be obtained by reduction of ester groups (such as - CO₂Et) or nitriles (-CN) using diisobutylaluminium hydride in a suitable solvent such as toluene. Alternatively, aldehyde groups may be obtained by the oxidation of alcohol groups using any suitable oxidising agent known to those skilled in the art.

Ester groups (-CO₂R') may be converted into the corresponding acid group (-CO₂H) by acid- or base-catalused hydrolysis, depending on the nature of R. If R is t-butyl, acid-catalysed hydrolysis can be achieved for example by treatment with an organic acid such as trifluoroacetic acid in an aqueous solvent, or by treatment with an inorganic acid such as hydrochloric acid in an aqueous solvent.

Carboxylic acid groups (-CO₂H) may be converted into amides (CONHR' or - CONR'R") by reaction with an appropriate amine in the presence of a suitable coupling agent, such as HATU, in a suitable solvent such as dichloromethane.

In a further example, carboxylic acids may be homologated by one carbon (i.e -CO₂H to -CH₂CO₂H) by conversion to the corresponding acid chloride (-COCI) followed by Arndt-Eistert synthesis.

In a further example, -OH groups may be generated from the corresponding ester (e.g. -CO₂R'), or aldehyde (-CHO) by reduction, using for example a complex metal hydride such as lithium aluminium hydride in diethyl ether or tetrahydrofuran, or sodium borohydride in a solvent such as methanol. Alternatively, an alcohol may be prepared by reduction of the corresponding acid (-CO₂H), using for example lithium aluminium hydride in a solvent such as tetrahydrofuran, or by using borane in a solvent such as tetrahydrofuran.

Alcohol groups may be converted into leaving groups, such as halogen atoms or sulfonyloxy groups such as an alkylsulfonyloxy, e.g. trifluoromethylsulfonyloxy or arylsulfonyloxy, e.g. *p*-toluenesulfonyloxy group using conditions known to those skilled in the art. For example, an alcohol may be reacted with thioyl chloride in a halogenated hydrocarbon (e.g. dichloromethane) to yield the corresponding chloride. A base (e.g. triethylamine) may also be used in the reaction.

In another example, alcohol, phenol or amide groups may be alkylated by coupling a phenol or amide with an alcohol in a solvent such as tetrahydrofuran in the presence of a phosphine, e.g. triphenylphosphine and an activator such as diethyl-, diisopropyl, or dimethylazodicarboxylate. Alternatively alkylation may be achieved by deprotonation using a suitable base e.g. sodium hydride followed by subsequent addition of an alkylating agent, such as an alkyl halide.

Aromatic halogen substituents in the compounds may be subjected to halogen-metal exchange by treatment with a base, for example a lithium base such as *n*-butyl or t-butyl lithium, optionally at a low temperature, e.g. around -78 °C, in a solvent such as tetrahydrofuran, and then quenched with an electrophile to introduce a desired substituent. Thus, for example, a formyl group may be introduced by using *N,N-*dimethylformamide as the electrophile. Aromatic halogen substituents may alternatively be subjected to metal (e.g. palladium or copper) catalysed reactions, to introduce, for example, acid, ester, cyano, amide, aryl, heteraryl, alkenyl, alkynyl, thio- or amino substituents. Suitable procedures which may be employed include those described by Heck, Suzuki, Stille, Buchwald or Hartwig.

Aromatic halogen substituents may also undergo nucleophilic displacement following reaction with an appropriate nucleophile such as an amine or an alcohol. Advantageously, such a reaction may be carried out at elevated temperature in the presence of microwave irradiation.

### METHODS OF SEPARATION

In each of the exemplary Schemes it may be advantageous to separate reaction products from one another or from starting materials. The desired products of each step or series of steps is separated or purified (hereinafter separated) to the desired degree of homogeneity by the techniques common in the art. Typically such separations involve multiphase extraction, crystallization or trituration from a solvent or solvent mixture, distillation, sublimation, or chromatography. Chromatography can involve any number of methods including, for example: reverse-phase and normal phase; size exclusion; ion exchange; supercritical fluid; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. Example separation methods include boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

Diastereomeric mixtures can be separated into their individual diastereoisomers on the basis of their physical chemical differences by methods well known to those skilled in the art, such as by chromatography or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereoisomers and converting (e.g., hydrolyzing) the individual diastereoisomers to the corresponding pure enantiomers. Also, some of the compounds of the present invention may be atropisomers (e.g., substituted biaryls) and are considered as part of this invention. Enantiomers can also be separated by use of a chiral HPLC column or supercritical fluid chromatography.

A single stereoisomer, e.g., an enantiomer, substantially free of its stereoisomer may be obtained by resolution of the racemic mixture using a method such as formation of diastereomers using optically active resolving agents (Eliel, E. and Wilen, S., Stereochemistry of Organic Compounds, John Wiley & Sons, Inc., New York, 1994; Lochmuller, C. H., J. Chromatogr., 113(3):283-302 (1975)). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Drug Stereochemistry, Analytical Methods and Pharmacology, Irving W. Wainer, Ed., Marcel Dekker, Inc., New York (1993).

Diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, the substrate to be resolved is reacted with one enantiomer of a chiral compound to form a diastereomeric pair (Eliel, E. and Wilen, S., Stereochemistry of Organic Compounds, John Wiley & Sons, Inc., New York, 1994, p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, such as a menthyl ester, e.g., (-) menthyl chloroformate in the presence of base, or Mosher ester, α-methoxy-α-(trifhioromethyl)phenyl acetate (Jacob, J. Org. Chem. 47:4165 (1982)), of the racemic mixture, and analyzing the NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111, incorporated herein by reference). By method (3), a racemic mixture of two enantiomers can be separated by chromatography using a chiral stationary phase (Chiral Liquid Chromatography W. J. Lough, Ed., Chapman and Hall, New York, (1989); Okamoto, J. of Chromatogr. 513:375-378 (1990)). Enriched or purified enantiomers can be distinguished by methods used to distinguish other chiral molecules with asymmetric carbon atoms, such as optical rotation and circular dichroism. The absolute stereochemistry of chiral centers and enatiomers can be determined by x-ray crystallography.

Positional isomers, for example E and Z forms, of compounds of Formula (I) or (II), and intermediates for their synthesis, may be observed by characterization methods such as NMR and analytical HPLC. For certain compounds where the energy barrier for interconversion is sufficiently high, the E and Z isomers may be separated, for example by preparatory HPLC.

### PHARMACEUTICAL COMPOSITIONS AND ADMINISTRATION

The compounds with which the invention is concerned are JAK kinase inhibitors, such as JAK1 inhibitors, and are useful in the treatment of several diseases, for example, inflammatory diseases, such as asthma.

Accordingly, another embodiment provides pharmaceutical compositions or medicaments containing a compound of the invention, such as a compound of Formula (I) or (II), or a stereoisomer, tautomer, solvate, prodrug or salt thereof, and a pharmaceutically acceptable carrier, diluent or excipient, as well as methods of using the compounds of the invention to prepare such compositions and medicaments.

In one example, a compound of Formula (I) or (II) or a stereoisomer, tautomer, solvate, prodrug or salt thereof may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but typically ranges anywhere from about 3 to about 8. In one example, a compound of Formula (I) or (II), or a stereoisomer, tautomer, solvate, prodrug or salt thereof, is formulated in an acetate buffer, at pH 5. In another embodiment, the compounds of the present invention, such as a compound of Formula (I) or (II) or a stereoisomer, tautomer, solvate, prodrug or salt thereof, are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial, as is required in the pharmaceutical art. In general, the daily dose range for oral administration will lie within the range of from about 0.001 mg to about 100 mg per kg body weight of a human, often 0.01 mg to about 50 mg per kg, for example 0.1 to 10 mg per kg, in single or divided doses. In general, the daily dose range for inhaled administration will lie within the range of from about 0.1 µg to about 1 mg per kg body weight of a human, preferably 0.1 µg to 50 µg per kg, in single or divided doses. On the other hand, it may be necessary to use dosages outside these limits in some cases.

The compounds of the invention, such as a compound of Formula (I) or (II) or a stereoisomer, tautomer, solvate, prodrug or salt thereof, may be administered by any suitable means, including oral, topical (including buccal and sublingual), rectal, vaginal, transdermal, parenteral, subcutaneous, intraperitoneal, intrapulmonary, intradermal, intrathecal, inhaled and epidural and intranasal, and, if desired for local treatment, intralesional administration. Parenteral infusions include intramuscular, intravenous, intraarterial, intraperitoneal, or subcutaneous administration. In some embodiments, inhaled administration is employed.

The compounds of the present invention, such as a compound of Formula (I) or (II) or a stereoisomer, tautomer, solvate, prodrug or salt thereof, may be administered in any convenient administrative form, e.g., tablets, powders, capsules, lozenges, granules, solutions, dispersions, suspensions, syrups, sprays, vapors, suppositories, gels, emulsions, patches, etc. Such compositions may contain components conventional in pharmaceutical preparations, e.g., diluents (e.g., glucose, lactose or mannitol), carriers, pH modifiers, buffers, sweeteners, bulking agents, stabilizing agents, surfactants, wetting agents, lubricating agents, emulsifiers, suspending agents, preservatives, antioxidants, opaquing agents, glidants, processing aids, colorants, perfuming agents, flavoring agents, other known additives as well as further active agents.

Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. For example, carriers include solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, pp 1289-1329, 1990). Except insofar as any conventional carrier is incompatible with the active ingredient, its use in the therapeutic or pharmaceutical compositions is contemplated. Exemplary excipients include dicalcium phosphate, mannitol, lactose, starch, magnesium stearate, sodium saccharine, cellulose, magnesium carbonate or combinations thereof. A pharmaceutical composition may comprise different types of carriers or excipients depending on whether it is to be administered in solid, liquid or aerosol form, and whether it need to be sterile for such routes of administration.

For example, tablets and capsules for oral administration may be in unit dose presentation form, and may contain conventional excipients such as binding agents, for example syrup, acacia, gelatin, sorbitol, tragacanth, or polyvinyl-pyrrolidone; fillers, for example, lactose, sugar, maize-starch, calcium phosphate, sorbitol or glycine; tabletting lubricant, for example, magnesium stearate, talc, polyethylene glycol or silica; disintegrants, for example, potato starch, or acceptable wetting agents such as sodium lauryl sulfate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example, sorbitol, syrup, methyl cellulose, glucose syrup, gelatin hydrogenated edible fats; emulsifying agents, for example, lecithin, sorbitan monooleate, or acacia; non-aqueous vehicles (which may include edible oils), for example, almond oil, fractionated coconut oil, oily esters such as glycerine, propylene glycol, or ethyl alcohol; preservatives, for example, methyl or propyl p-hydroxybenzoate or sorbic acid, and if desired conventional flavoring or coloring agents.

For topical application to the skin, a compound may be made up into a cream, lotion or ointment. Cream or ointment formulations which may be used for the drug are conventional formulations well known in the art, for example as described in standard textbooks of pharmaceutics such as the British Pharmacopoeia.

Compounds of the invention, such as a compound of Formula (I) or (II) or a stereoisomer, tautomer, solvate, prodrug or salt thereof, may also be formulated for inhalation, for example, as a nasal spray, or dry powder or aerosol inhalers. For delivery by inhalation, the compound is typically in the form of microparticles, which can be prepared by a variety of techniques, including spray-drying, freeze-drying and micronisation. Aerosol generation can be carried out using, for example, pressure-driven jet atomizers or ultrasonic atomizers, such as by using propellant-driven metered aerosols or propellant-free administration of micronized compounds from, for example, inhalation capsules or other "dry powder" delivery systems.

By way of example, a composition of the invention may be prepared as a suspension for delivery from a nebulizer or as an aerosol in a liquid propellant, for example, for use in a pressurized metered dose inhaler (PMDI). Propellants suitable for use in a PMDI are known to the skilled person, and include CFC-12, HFA-134a, HFA-227, HCFC-22 (CCl₂F₂) and HFA-152 (CH₄F₂ and isobutane).

In some embodiments, a composition of the invention is in dry powder form, for delivery using a dry powder inhaler (DPI). Many types of DPI are known.

Microparticles for delivery by administration may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of, for example, greater than 90 µm.

In the case of an aerosol-based formulation, an example is:

| | |
|---|---|
| Compound of the invention* | 24 mg / canister |
| Lecithin, NF Liq. Conc. | 1.2 mg / canister |
| Trichlorofluoromethane, NF | 4.025 g / canister |
| Dichlorodifluoromethane, NF | 12.15 g / canister. |

| | |
|---|---|
| * Such as a compound of Formula (I) or (II). | |

A compound, such as a compound of Formula (I) or (II) or a stereoisomer, tautomer, solvate, prodrug or salt thereof, may be dosed as described depending on the inhaler system used. In addition to the compound, the administration forms may additionally contain excipients as described above, or, for example, propellants (e.g., Frigen in the case of metered aerosols), surface-active substances, emulsifiers, stabilizers, preservatives, flavorings, fillers (e.g., lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g., Nebulator^{®}, Volumatic^{®}), and automatic devices emitting a puffer spray (Autohaler^{®}), for metered aerosols, in the case of powder inhalers in particular, a number of technical solutions are available (e.g., Diskhaler^{®}, Rotadisk^{®}, Turbohaler^{®} or the inhalers, for example, as described in U.S. Patent No. 5,263,475, incorporated herein by reference). Additionally, compounds of the invention, such as a compound of Formula (I) or (II) or a stereoisomer, tautomer, solvate, prodrug or salt thereof, may be delivered in multichamber devices thus allowing for delivery of combination agents.

The compound, such as a compound of Formula (I) or (II) or a stereoisomer, tautomer, solvate, prodrug or salt thereof, may also be administered parenterally in a sterile medium. Depending on the vehicle and concentration used, the compound can either be suspended or dissolved in the vehicle. Advantageously, adjuvants such as a local anaesthetic, preservative or buffering agents can be dissolved in the vehicle.

### TARGETED INHALED DRUG DELIVERY

Optimisation of drugs for delivery to the lung by topical (inhaled) administration has been recently reviewed (Cooper, A. E. et al. Curr. Drug Metab. 2012, 13, 457-473). Due to limitations in the delivery device, the dose of an inhaled drug is likely to be low (approximately <1mg/day) in humans which necessitates highly potent molecules. For compounds destined to be delivered via dry powder inhalation there is also a requirement to be able to generate crystalline forms of the compound that can be micronized to 1-5 µm in size. Additionally, the compound needs to maintain a sufficient concentration in the lung over a given time period so as to be able to exert a pharmacological effect of the desired duration, and for pharmacological targets where systemic inhibition of said target is undesired, to have a low systemic exposure. The lung has an inherently high permeability to both large molecules (proteins, peptides) as well as small molecules with concomitant short lung half-lives, thus it is necessary to attenuate the lung absorption rate through modification of one or more features of the compounds: minimizing membrane permeability, reducing dissolution rate, or introducing a degree of basicity into the compound to enhance binding to the phospholipid-rich lung tissue or through trapping in acidic sub-cellular compartments such as lysosomes (pH 5). Accordingly, in some embodiments, compounds of the present invention exhibit one or more of these features.

### METHODS OF TREATMENT WITH AND USES OF JANUS KINASE INHIBITORS

The compounds of the present invention, such as a compound of Formula (I) or (II) or a stereoisomer, tautomer, solvate, prodrug or salt thereof, inhibit the activity of a Janus kinase, such as JAK1 kinase. For example, a compound of the present invention, such as a compound of Formula (I) or (II) or a stereoisomer, tautomer, solvate, prodrug or salt thereof, inhibits the phosphorylation of signal transducers and activators of transcription (STATs) by JAK1 kinase as well as STAT mediated cytokine production. Compounds of the present invention, such as a compound of Formula (I) or (II) or a stereoisomer, tautomer, solvate, prodrug or salt thereof, are useful for inhibiting JAK1 kinase activity in cells through cytokine pathways, such as IL-6, IL-15, IL-7, IL-2, IL-4, IL-9, IL-10, IL-13, IL-21, G-CSF, IFNalpha, IFNbeta or IFNgamma pathways. Accordingly, in one embodiment is provided a method of contacting a cell with a compound of the present invention, such as a compound of Formula (I) or (II) or a stereoisomer, tautomer, solvate, prodrug or salt thereof, to inhibit a Janus kinase activity in the cell (e.g., JAK1 activity).

The compounds of the present invention, such as compounds of Formula (I) or (II) or a stereoisomer, tautomer, solvate, prodrug or salt thereof, can be used for the treatment of immunological disorders driven by aberrant IL-6, IL-15, IL-7, IL-2, IL-4, IL9, IL-10, IL-13, IL-21, G-CSF, IFNalpha, IFNbeta or IFNgamma cytokine signaling.

Accordingly, one embodiment includes compounds of the present invention, such as a compound of Formula (I) or (II) or a stereoisomer, tautomer, solvate, prodrug or salt thereof, for use in therapy.

In some embodiments, there is provided use a compound of the present invention, such as a compound of Formula (I) or (II) or a stereoisomer, tautomer, solvate, prodrug or salt thereof, in the treatment of an inflammatory disease. Further provided is use of a compound of the present invention, such as a compound of Formula (I) or (II) or a stereoisomer, tautomer, solvate, prodrug or salt thereof, for the preparation of a medicament for the treatment of an inflammatory disease, such as asthma. Also provided is a compound of the present invention, such as a compound of Formula (I) or (II) or a stereoisomer, tautomer, solvate, prodrug or salt thereof, for use in the treatment of an inflammatory disease, such as asthma.

Another embodiment includes a method of preventing, treating or lessening the severity of a disease or condition, such as asthma, responsive to the inhibition of a Janus kinase activity, such as JAK1 kinase activity, in a patient. The method can include the step of administering to a patient a therapeutically effective amount of a compound of the present invention, such as a compound of Formula (I) or (II) or a stereoisomer, tautomer, solvate, prodrug or salt thereof. In one embodiment, the disease or condition responsive to the inhibition of a Janus kinase, such as JAK1 kinase, is asthma.

In one embodiment, the disease or condition is cancer, stroke, diabetes, hepatomegaly, cardiovascular disease, multiple sclerosis, Alzheimer's disease, cystic fibrosis, viral disease, autoimmune diseases, atherosclerosis, restenosis, psoriasis, rheumatoid arthritis, inflammatory bowel disease, asthma, allergic disorders, inflammation, neurological disorders, a hormone-related disease, conditions associated with organ transplantation (e.g., transplant rejection), immunodeficiency disorders, destructive bone disorders, proliferative disorders, infectious diseases, conditions associated with cell death, thrombin-induced platelet aggregation, liver disease, pathologic immune conditions involving T cell activation, alopecia, CNS disorders or a myeloproliferative disorder.

In one embodiment, the inflammatory disease is rheumatoid arthritis, psoriasis, asthma, inflammatory bowel disease, contact dermatitis or delayed hypersensitivity reactions. In one embodiment, the autoimmune disease is rheumatoid arthritis, lupus or multiple sclerosis.

In one embodiment, the cancer is breast, ovary, cervix, prostate, testis, penile, genitourinary tract, seminoma, esophagus, larynx, gastric, stomach, gastrointestinal, skin, keratoacanthoma, follicular carcinoma, melanoma, lung, small cell lung carcinoma, non-small cell lung carcinoma (NSCLC), lung adenocarcinoma, squamous carcinoma of the lung, colon, pancreas, thyroid, papillary, bladder, liver, biliary passage, kidney, bone, myeloid disorders, lymphoid disorders, hairy cells, buccal cavity and pharynx (oral), lip, tongue, mouth, salivary gland, pharynx, small intestine, colon, rectum, anal, renal, prostate, vulval, thyroid, large intestine, endometrial, uterine, brain, central nervous system, cancer of the peritoneum, hepatocellular cancer, head cancer, neck cancer, Hodgkin's or leukemia.

In one embodiment, the disease is a myeloproliferative disorder. In one embodiment, the myeloproliferative disorder is polycythemia vera, essential thrombocytosis, myelofibrosis or chronic myelogenous leukemia (CML).

Another embodiment includes the use of a compound of the present invention, such as a compound of Formula (I) or (II) or a stereoisomer, tautomer, solvate, prodrug or salt thereof, for the manufacture of a medicament for the treatment of a disease described herein (e.g., an inflammatory disorder, an immunological disorder or cancer). In one embodiment, the invention provides a method of treating a disease or condition as described herein e.g., an inflammatory disorder, an immunological disorder or cancer) by targeting inhibition of a JAK kinase, such as JAK1.

### COMBINATION THERAPY

The compounds of the present invention, such as a compound of Formula (I) or (II) or a stereoisomer, tautomer, solvate, prodrug or salt thereof, may be employed alone or in combination with other agents for treatment. The second compound of a pharmaceutical composition or dosing regimen typically has complementary activities to the compound of this invention such that they do not adversely affect each other. Such agents are suitably present in combination in amounts that are effective for the purpose intended. The compounds may be administered together in a unitary pharmaceutical composition or separately and, when administered separately this may occur simultaneously or sequentially. Such sequential administration may be close or remote in time.

For example, other compounds may be combined with compounds with which the invention is concerned for the prevention or treatment of inflammatory diseases, such as asthma. Thus the present invention is also concerned with pharmaceutical compositions comprising a therapeutically effective amount of a compound of the invention and one or more other therapeutic agents. Suitable therapeutic agents for a combination therapy with compounds of the invention include, but are not limited to: an adenosine A2A receptor antagonist; an anti-infective; a non-steroidal Glucocorticoid Receptor (GR Receptor) agonist; an antioxidant; a β2 adrenoceptor agonist; a CCR1 antagonist; a chemokine antagonist (not CCR1); a corticosteroid; a CRTh2 antagonist; a DP1 antagonist; a formyl peptide receptor antagonist; a histone deacetylase activator; a chloride channel hCLCA1 blocker; an epithelial sodium channel blocker (ENAC blocker; an inter-cellular adhesion molecule 1 blocker (ICAM blocker); an IKK2 inhibitor; a JNK inhibitor; a cyclooxygenase inhibitor (COX inhibitor); a lipoxygenase inhibitor; a leukotriene receptor antagonist; a dual β2 adrenoceptor agonist/M3 receptor antagonist (MABA compound); a MEK-1 inhibitor; a myeloperoxidase inhibitor (MPO inhibitor); a muscarinic antagonist; a p38 MAPK inhibitor; a phosphodiesterase PDE4 inhibitor; a phosphatidylinositol 3-kinase δ inhibitor (PI3-kinase δ inhibitor); a phosphatidylinositol 3-kinase γ inhibitor (PI3-kinase γ inhibitor); a peroxisome proliferator activated receptor agonist (PPARy agonist); a protease inhibitor; a retinoic acid receptor modulator (RAR γ modulator); a statin; a thromboxane antagonist; a TLR7 receptor agonist; or a vasodilator.

In addition, compounds of the invention, such as a compound of Formula (I) or (II), may be combined with: (1) corticosteroids, such as alclometasone dipropionate, amelometasone, beclomethasone dipropionate, budesonide, butixocort propionate, biclesonide, blobetasol propionate, desisobutyrylciclesonide, dexamethasone, dtiprednol dicloacetate, fluocinolone acetonide, fluticasone furoate, fluticasone propionate, loteprednol etabonate (topical) or mometasone furoate; (2) β2-adrenoreceptor agonists such as salbutamol, albuterol, terbutaline, fenoterol, bitolterol, carbuterol, clenbuterol, pirbuterol, rimoterol, terbutaline, tretoquinol, tulobuterol and long acting β2-adrenoreceptor agonists such as metaproterenol, isoproterenol, isoprenaline, salmeterol, indacaterol, formoterol (including formoterol fumarate), arformoterol, carmoterol, abediterol, vilanterol trifenate, olodaterol; (3) corticosteroid/long acting β2 agonist combination products such as salmeterol/fluticasone propionate (Advair^{®}, also sold as Seretide^{®}), formoterol/budesonide (Symbicort^{®}), formoterol/fluticasone propionate (Flutiform^{®}), formoterol/ciclesonide, formoterol/mometasone furoate, indacaterol/mometasone furoate, vilanterol trifenate/fluticasone furoate, or arformoterol/ciclesonide; (4) anticholinergic agents, for example, muscarinic-3 (M3) receptor antagonists such as ipratropium bromide, tiotropium bromide, aclidinium (LAS-34273), glycopyrronium bromide, umeclidinium bromide; (5) M3-anticholinergic/β2-adrenoreceptor agonist combination products such as vilanterol /umeclidinium (Anoro^{®} Ellipta^{®}), olodaterol/tiotropium bromide, glycopyrronium bromide/indacaterol (Ultibro^{®}, also sold as Xoterna^{®}), fenoterol hydrobromide/ipratropium bromide (Berodual^{®}), albuterol sulfate/ipratropium bromide (Combivent^{®}), formoterol fumarate/glycopyrrolate, or aclidinium bromide/formoterol (6) dual pharmacology M3-anticholinergic/β2-adrenoreceptor agonists such as batefenterol succinate, AZD-2115 or LAS-190792; (7) leukotriene modulators, for example, leukotriene antagonists such as montelukast, zafirulast or pranlukast or leukotriene biosynthesis inhibitors such as zileuton, or LTB4 antagonists such as amelubant, or FLAP inhibitors such as fiboflapon, GSK-2190915; (8) phosphodiesterase-IV (PDE-IV) inhibitors (oral or inhaled), such as roflumilast, cilomilast, oglemilast, rolipram, tetomilast, AVE-8112, revamilast, CHF 6001; (9) antihistamines, for example, selective histamine-1 (H1) receptor antagonists such as fexofenadine, citirizine, loratidine or astemizole or dual H1/H3 receptor antagonists such as GSK 835726, or GSK 1004723; (10) antitussive agents, such as codeine or dextramorphan; (11) a mucolytic, for example, N-acetyl cysteine or fudostein; (12) a expectorant/mucokinetic modulator, for example, ambroxol, hypertonic solutions (e.g., saline or mannitol) or surfactant; (13) a peptide mucolytic, for example, recombinant human deoxyribonoclease I (dornase-alpha and rhDNase) or helicidin; (14) antibiotics, for example azithromycin, tobramycin or aztreonam; (15) non-selective COX-1/COX-2 inhibitors, such as ibuprofen or ketoprofen; (16) COX-2 inhibitors, such as celecoxib and rofecoxib; (17) VLA-4 antagonists, such as those described in WO97/03094 and WO97/02289, each incorporated herein by reference; (18) TACE inhibitors and TNF-α inhibitors, for example anti-TNF monoclonal antibodies, such as Remicade^{®} and CDP-870 and TNF receptor immunoglobulin molecules, such as Enbrel^{®}; (19) inhibitors of matrix metalloprotease, for example MMP-12; (20) human neutrophil elastase inhibitors, such as BAY-85-8501 or those described in WO2005/026124, WO2003/053930 and WO06/082412, each incorporated herein by reference; (21) A2b antagonists such as those described in WO2002/42298, incorporated herein by reference; (22) modulators of chemokine receptor function, for example antagonists of CCR3 and CCR8; (23) compounds which modulate the action of other prostanoid receptors, for example, a thromboxane A₂ antagonist; DP1 antagonists such as laropiprant or asapiprant CRTH2 antagonists such as OC000459, fevipiprant, ADC 3680 or ARRY 502; (24) PPAR agonists including PPAR alpha agonists (such as fenofibrate), PPAR delta agonists, PPAR gamma agonists such as pioglitazone, rosiglitazone and balaglitazone; (25) methylxanthines such as theophylline or aminophylline and methylxanthine/corticosteroid combinations such as theophylline/budesonide, theophylline/fluticasone propionate, theophylline/ciclesonide, theophylline/mometasone furoate and theophylline/beclometasone dipropionate; (26) A2a agonists such as those described in EP1052264 and EP1241176; (27) CXCR2 or IL-8 antagonists such as AZD-5069, AZD-4721, danirixin; (28) IL-R signalling modulators such as kineret and ACZ 885; (29) MCP-1 antagonists such as ABN-912; (30) a p38 MAPK inhibitor such as BCT197, JNJ49095397, losmapimod or PH-797804; (31) TLR7 receptor agonists such as AZD 8848; (32) PI3-kinase inhibitors such as RV1729 or GSK2269557.

In some embodiments, the compounds of the present invention, such as a compound of Formula (I) or (II) or a stereoisomer, tautomer, solvate, prodrug or salt thereof, can be used in combination with one or more additional drugs, for example anti-hyperproliferative, anti-cancer, cytostatic, cytotoxic, anti-inflammatory or chemotherapeutic agents, such as those agents disclosed in U.S. Publ. Appl. No. 2010/0048557, incorporated herein by reference. A compound of the present invention, such as a compound of Formula (I) or (II), can be also used in combination with radiation therapy or surgery, as is known in the art.

### ARTICLES OF MANUFACTURE

Another embodiment includes an article of manufacture (e.g., a kit) for treating a disease or disorder responsive to the inhibition of a Janus kinase, such as a JAK1 kinase. The kit can comprise:
(a) a first pharmaceutical composition comprising a compound of the present invention, such as a compound of Formula (I) or (II) or a stereoisomer, tautomer, solvate, prodrug or salt thereof; and
(b) instructions for use.

In another embodiment, the kit further comprises:
(c) a second pharmaceutical composition, such as a pharmaceutical composition comprising an agent for treatment as described above, such as an agent for treatment of an inflammatory disorder, or a chemotherapeutic agent.

In one embodiment, the instructions describe the simultaneous, sequential or separate administration of said first and second pharmaceutical compositions to a patient in need thereof.

In one embodiment, the first and second compositions are contained in separate containers. In another embodiment, the first and second compositions are contained in the same container.

Containers for use include, for example, bottles, vials, syringes, blister pack, etc. The containers may be formed from a variety of materials such as glass or plastic. The container includes a compound of the present invention, such as a compound of Formula (I) or (II), or composition thereof, which is effective for treating the condition and may have a sterile access port (for example the container may be an intravenous solution bag or a vial having a stopper pierceable by a hypodermic injection needle). The label or package insert indicates that the compound or composition is used for treating the condition of choice, such as asthma or cancer. In one embodiment, the label or package inserts indicates that the compound or composition can be used to treat a disorder. In addition, the label or package insert may indicate that the patient to be treated is one having a disorder characterized by overactive or irregular Janus kinase activity, such as overactive or irregular JAK1 activity. The label or package insert may also indicate that the compound or composition can be used to treat other disorders.

Alternatively, or additionally, the kit may further comprise a second (or third) container comprising a pharmaceutically acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

In order to illustrate the invention, the following examples are included. However, it is to be understood that these examples do not limit the invention and are only meant to suggest a method of practicing the invention. Persons skilled in the art will recognize that the chemical reactions described may be readily adapted to prepare other compounds of the present invention, and alternative methods for preparing the compounds are within the scope of this invention. For example, the synthesis of non-exemplified compounds according to the invention may be successfully performed by modifications apparent to those skilled in the art, e.g., by appropriately protecting interfering groups, by utilizing other suitable reagents known in the art other than those described, or by making routine modifications of reaction conditions. Alternatively, other reactions disclosed herein or known in the art will be recognized as having applicability for preparing other compounds of the invention.

### EXAMPLES

Although the invention has been described and illustrated with a certain degree of particularity, it is understood that the present disclosure has been made only by way of example, and that numerous changes in the combination and arrangement of parts can be resorted to by those skilled in the art without departing from the spirit and scope of the invention, as defined by the claims. 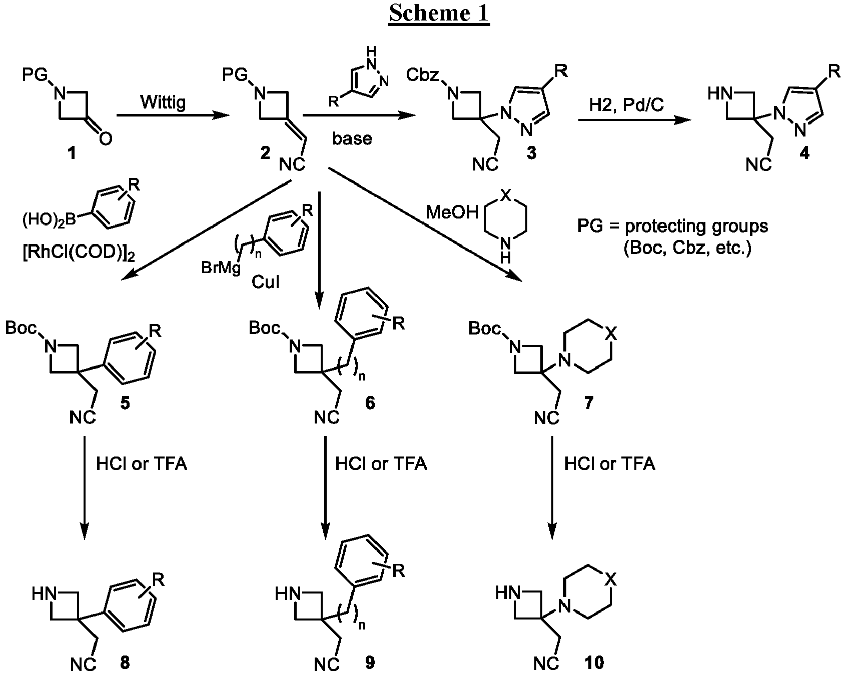 Compounds of formula **8-10** may be prepared by general synthetic methods as shown in Scheme 1.

Compound **2** can be synthesized by treatment of appropriately protected azetidinone **1.** A solution of azetidinone **1** in an organic solvent such as, but not limited to, THF was added to a solution of diethyl cyanomethyl phosphate and a base such as, but not limited to, potassium tert-butoxide, in an organic solvent such as, but not limited to, THF at a temperature of about -5 °C and for a time varying from 3-5 h afforded (cyanomethylidene)azetidine **2.** Compound **2** can be treated with an appropriately substituted pyrazole with a base such as, but not limited to, DBU, in an organic solvent such as, but not limited to, acetonitrile at a temperature of about 50 °C and for a time for about 3-24 h readily affords compounds of formula 3. Deprotection of the Cbz group with a catalyst such as, but not limited to, Pd/C and hydrogen gas at atmospheric pressure for a time varying from 12-24 h and for a temperature of about room temperature yielded compounds of formula **4.** Alternatively, (cyanomethylidene)azetidine **2** can be treated with an appropriately substituted boronic acid, with a catalyst such as, but not limited to, [Rh(COD)Cl]₂ and with a base such as, but not limited to potassium hydroxide in an organic solvent such as, but not limited to, 1,4-dioxane at a temperature for about 100 °C in the presence of microwave irradiation and for a time of about 1 h gives compounds of formula **5.** Compound **2** can also be treated with various Grignard reagents in the presence of CuI in an organic solvent such as, but not limited to, THF at a temperature of about room temperature and for a time varying from 12-24 h affords compounds of formula **6.** Lastly, compound **2** can be treated with an appropriately substituted amine in an organic solvent such as, but not limited to, MeOH at a temperature of about reflux temperature and for a time varying from 12-24 h affords compounds of formula **7.** Compounds of formula **8-10** can produced from treatment of compounds of formula **5-7** under acidic conditions such as, but not limited to, HCl or TFA at about room temperature and for a time varying from 12-24 h. 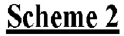 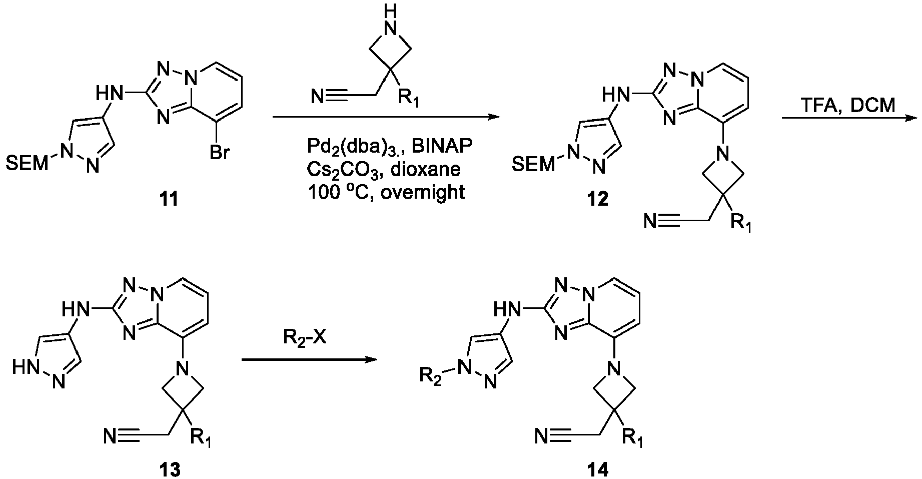 Compounds of formula **14** may be prepared by general synthetic methods as shown in Scheme 2.

Compounds of formula **12** can be synthesized by treatment of compound **11** with a catalyst such as, but not limited to, Pd₂(dba)₃, a ligand such as, but not limited to, BINAP and a base such as, but not limited to, Cs₂CO₃ at a temperature at about 100 °C and for a time varying from 12-24 h. Deprotection of the SEM group of **12** under acidic conditions followed by alkylation of an appropriately substituted alkyl halide in the presence of a base such as, but not limited to, DIPEA in an organic solvent such as, but not limited to DMF at a temperature of about room temperature and for a time varying from 12-24 h affords compounds of formula **14.** 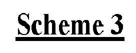 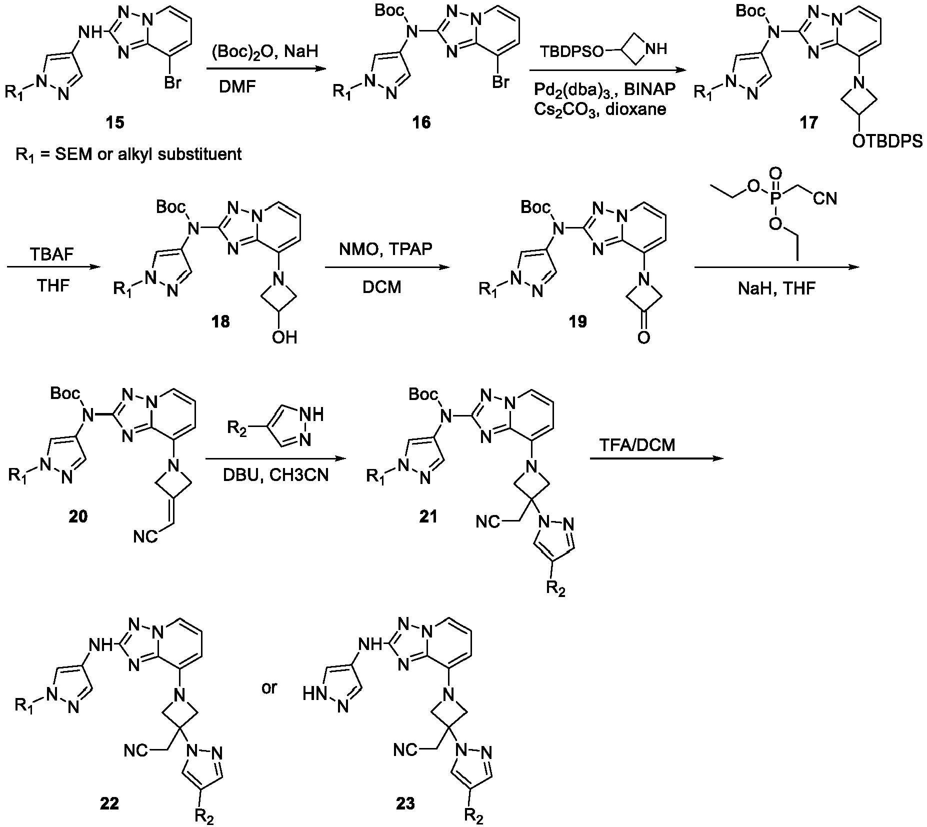 Compounds of formula **22** and **23** may be prepared by general synthetic methods as shown in Scheme 3.

Compounds of formula **16** can be accessed by treatment of **15** with sodium hydride and (Boc)₂O in an organic solvent such as, but not limited to, DMF at room temperature for a time of about 2 h. Coupling of 3-[(tert-butyldiphenylsilyl)oxy]azetidine and compound 16 can occur using a catalyst such as, but not limited to, Pd₂(dba)₃, a ligand such as, but not limited to, BINAP and a base such as, but not limited to, Cs₂CO₃ at a temperature at about 100 °C and for a time varying from 12-24 h. Deprotection of the TBDPS group (TBAF in THF at room temperature) followed by oxidation of the resultant alcohol with an oxidant such as NMO / TPAP in an organic solvent such as DCM at about room temperature and for a time varying from 12-24 h. Compounds of formula **21** can be obtained following the same general sequence outlined in Scheme 1. Treatment of 21 with an acid such as, but not limited to, TFA at about room temperature for a time of about 2 h afforded either compounds of formula **22** or **23.** 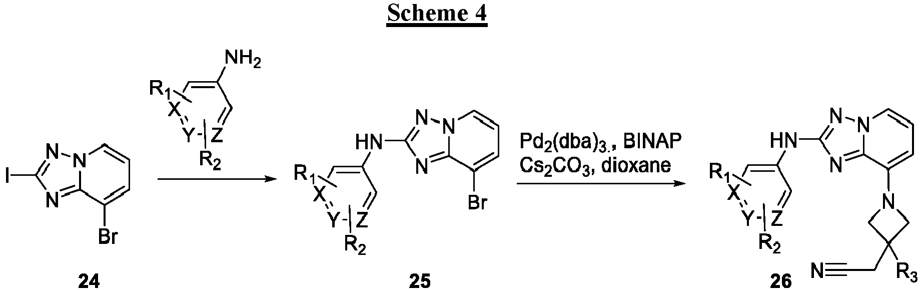 Compounds of formula **26** may be prepared by general synthetic methods as shown in Scheme 4.

Compounds of formula **25** can be synthesized by treatment of **24** with an appropriately substituted 5- or 6-membered aniline, a catalyst such as, but not limited to, Pd₂(dba)₃, a ligand such as, but not limited to, XantPhos and a base such as, but not limited to Cs₂CO₃ at about at 60 °C and for a time varying from 12-24 h. Compounds of formula **26** can be formed from the coupling of **25** with an appropriately substituted azetidine as outlined in Scheme 2.

### Abbreviations

- DCM: Dichloromethane
- DIPEA: Diisopropylethylamine
- DMF: N,N-Dimethylformamide
- DMSO: Dimethylsulfoxide
- DMSO-*d6*: Deuterated dimethylsulfoxide
- EtOAc: Ethyl acetate
- EtOH: Ethanol
- g: Gram
- HATU: (O-(7-azabenzotriazol-1-yl)-N,N,N',N'-tetramethyluronium hexafluorophosphate)
- HCl: Hydrochloric acid
- HM-N: Isolute HM-N is a modified form of diatomaceous earth
- L: Litre
- MeCN: Acetonitrile
- MeOH: Methanol
- mg: Milligram
- mL: Millilitre
- NaOH: Sodium hydroxide
- Pd₂(dba)₃: Tris(dibenzylidineacetone)palladium(0)
- Pd(PPh₃)₄: Tetrakis(triphenylphosphine)palladium(0)
- RT: Ambient temperature
- THF: Tetrahydrofuran
- TFA: Trifluoroacetic acid
- XantPhos: 4,5-Bis(diphenylphosphino)-9,9-dimethylxanthene
- X-phos: 2-Dicyclohexylphosphino-2',4',6'-triisopropylbiphenyl

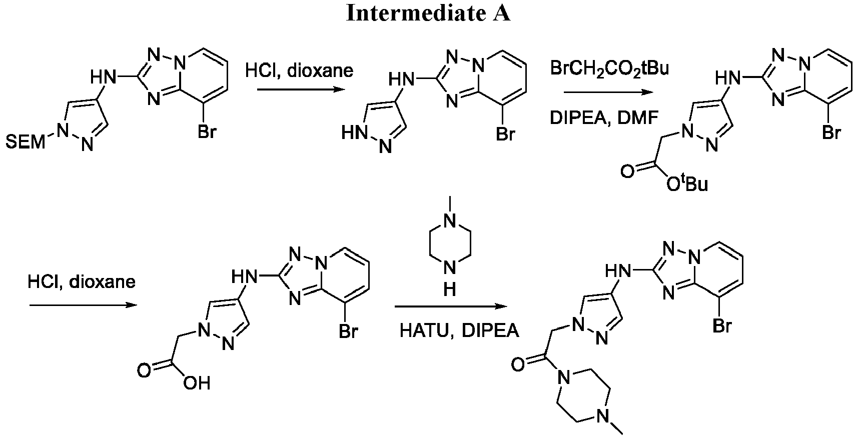

### 2-[4-([8-bromo-[1,2,4]triazolo[1,5-a]pyridin-2-yl]amino)-1H-pyrazol-1-yl]-1-(4-methylpiperazin-1-yl)ethan-1-one

To a 2000-mL round-bottom flask was added 800 mL of saturated HCl solution in dioxane. N-[8-bromo-[1,2,4]triazolo[1,5-a]pyridin-2-yl]-1-[[2-(trimethylsilyl)ethoxy]-methyl]-1H-pyrazol-4-amine (WO 201532286A1, 100 g, 244 mmol) was added in several batches. The solution was stirred for 5 h at room temperature. The resulting mixture was concentrated under vacuum. Water (600 mL) was added and the pH value of the solution was adjusted to 9 with 20% aqueous solution of sodium carbonate. The solids were collected by filtration and dried to give 68.0 g of N-[8-bromo-[1,2,4]triazolo[1,5-a]pyridin-2-yl]-1H-pyrazol-4-amine as a white solid.

To a 2000-mL round-bottom flask was added a solution of N-[8-bromo-[1,2,4]triazolo[1,5-a]pyridin-2-yl]-1H-pyrazol-4-amine (70.0 g, 250 mmol) in N,N-dimethylformamide (1000 mL) followed by DIPEA (98.0 g, 758 mmol). Tert-butyl 2-bromoacetate (98.0 g, 502 mmol) was added at room temperature. The solution was stirred overnight at room temperature. The reaction was then quenched by the addition of 2000 mL of water. The resulting solution was extracted with 3x1000 mL of ethyl acetate and the organic layers combined. The resulting mixture was washed with 1x1000 mL of brine. The mixture was dried over anhydrous sodium sulfate and concentrated under vacuum. The residue was purified by flash chromatography on silica gel eluting with ethyl acetate/hexane (1/1). The appropriate fractions were combined and concentrated under vacuum to afford 80.1 g (81%) of tert-butyl 2-[4-([8-bromo-[1,2,4]triazolo[1,5-a]pyridin-2-yl]amino)-1H-pyrazol-1-yl]acetate as a light yellow solid.

To a 2000-mL round-bottom flask was added saturated HCl solution (1 L) in dioxane, tert-butyl 2-[4-([8-bromo-[1,2,4]triazolo[1,5-a]pyridin-2-yl]amino)-1H-pyrazol-1-yl]acetate (70.0 g, 178 mmol) was added in several batches. The solution was stirred overnight at room temperature. The mixture was concentrated under vacuum and to this residue was added a 20% Na₂CO₃ aqueous solution until the mixture reached pH ~5. The solids were collected by filtration and dried to afford 35.1 g (58%) of 2-[4-([8-bromo-[1,2,4]triazolo[1,5-a]pyridin-2-yl]amino)-1H-pyrazol-1-yl]acetic acid as a yellow solid.

Into a 500-mL round-bottom flask was added 2-[4-([8-bromo-[1,2,4]triazolo[1,5-a]pyridin-2-yl]amino)-1H-pyrazol-1-yl]acetic acid (10.0 g, 29.6 mmol), N,N-dimethylformamide (200 mL), EDC.HCl (11.4 g, 59.6 mmol), HOBt (8.04 g, 59.5 mmol), DIPEA (15.3 g, 118 mmol) and 1-methylpiperazine (5.95 g, 59.4 mmol). The solution was stirred at room temperature overnight. The resulting mixture was concentrated under vacuum. The residue was dissolved in 400 mL of DCM and the mixture was washed with 3x200 mL of 0.5M NaOH solution. The resulting mixture was concentrated under vacuum. The crude product was purified by re-crystallization in 100 ml of DCM resulting in 9.17 g (74%) of 2-[4-([8-bromo-[1,2,4]triazolo[1,5-a]pyridin-2-yl]amino)-1H-pyrazol-1-yl]-1-(4-methylpiperazin-1-yl)ethan-1-one as a gray solid. LC/MS (Method 1, ESI): [M+H]⁺ = 419.1, R_{T} = 1.12 min; ¹H NMR (300 MHz, DMSO-*d*₆) δ (ppm) 9.44 (s, 1 H), 8.72 (dd, *J* = 6.6, 0.9 Hz, 1 H), 7.83 (dd, *J* = 7.8, 0.9 Hz, 1 H), 7.77 (s, 1 H), 7.44 (s, 1 H), 6.89 (dd, *J* = 7.8, 6.9 Hz, 1 H), 5.08 (s, 2 H), 3.48-3.45 (m, 4 H), 2.32-2.27 (m, 4 H), 2.19 (s, 3 H).

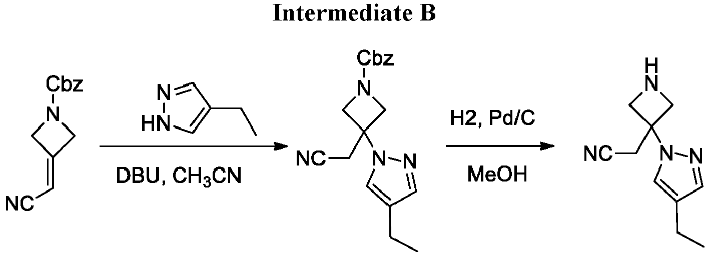

### 2-[3-(4-ethyl-1H-pyrazol-1-yl)azetidin-3-yl]acetonitrile

To a solution of benzyl 3-(cyanomethylidene)azetidine-1-carboxylate (2.40 g, 10.5 mmol) in acetonitrile (20 mL) was added 4-ethyl-1H-pyrazole (1.00 g, 10.4 mmol) and DBU (1.08 g, 7.09 mmol). The solution was stirred for 3 h at 50 °C and concentrated under vacuum. The residue was purified by flash chromatography on silica gel eluting with ethyl acetate/petroleum ether (1/2). The appropriate fractions were combined and concentrated under vacuum yielding 3.00 g (88%) of benzyl 3-(cyanomethyl)-3-(4-ethyl-1H-pyrazol-1-yl)azetidine-1-carboxylate as a light yellow oil. LC/MS (Method 7, ESI): [M+H]⁺ = 325.2, R_{T}= 1.47 min.

To a mixture of 10% Pd/C (100 mg) in methanol (15 mL) was added benzyl 3-(cyanomethyl)-3-(4-ethyl-1H-pyrazol-1-yl)azetidine-1-carboxylate (3.00 g, 9.24 mmol). The reaction mixture was stirred at room temperature overnight under an atmosphere of hydrogen (with balloon). The catalyst was filtered off. The filtrate was concentrated under vacuum. The residue was purified by flash chromatography on silica gel eluting with dichloromethane/methanol (87/13). The appropriate fractions were combined and concentrated under vacuum to afford 1.60 g (91%) of 2-[3-(4-ethyl-1H-pyrazol-1-yl)azetidin-3-yl]acetonitrile as a colorless oil. LC/MS (Method 7, ESI): [M+H]⁺ = 191.3, R_{T}= 0.65 min.

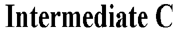

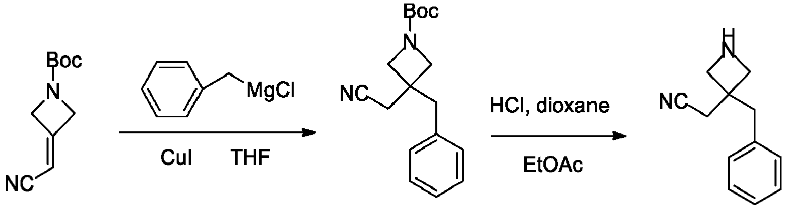

### 2-(3 -benzylazetidin-3 -yl)acetonitrile

A solution of benzyl magnesium chloride (5.00 mL, 1 M in THF) was added dropwise to a mixture of tert-butyl 3-(cyanomethylidene)azetidine-1-carboxylate (500 mg, 2.57 mmol) and CuI (196 mg, 1.02 mmol) in THF (40 mL) under nitrogen. The solution was stirred at room temperature overnight. The reaction was then quenched by the addition of 5 ml of water. The solids were filtered out. The filtrate was concentrated under vacuum. The residue was purified by flash chromatography silica gel eluting with ethyl acetate/petroleum ether (2/3). The appropriate fractions were combined and concentrated to afford 460 mg (62%) of tert-butyl 3-benzyl-3-(cyanomethyl)azetidine-1-carboxylate as a yellow oil.

A solution of tert-butyl 3-benzyl-3-(cyanomethyl)azetidine-1-carboxylate (230 mg, 0.803 mmol), ethyl acetate (2.00 ml) and HCl/dioxane (8 mL, 4M) was stirred for 2 h at room temperature. The resulting mixture was concentrated under vacuum. The residue was dissolved in water (5 mL). The pH value of the solution was adjusted to 9 with sodium bicarbonate. The resulting mixture was concentrated under vacuum. The residue was dissolved in 30 ml of DCM/MeOH (5/1) and the solids were filtered out. The filtrate was then concentrated to afford 2-(3-benzylazetidin-3-yl)acetonitrile as a light yellow solid (200 mg). LC/MS (Method 7, ESI): [M+H]⁺ = 187.3, R_{T}= 0.67 min.

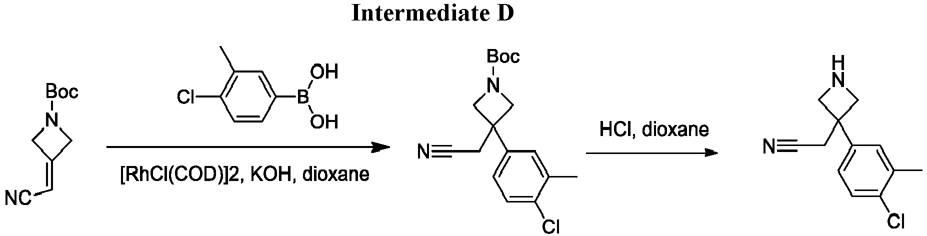

### 2- [3 -(4-chloro-3 -methylphenyl)azetidin-3 -yl] acetonitrile

Into a 30-mL microwave vial purged with nitrogen was added [Rh(COD)Cl]₂ (40.0 mg, 0.081 mmol), tert-butyl 3-(cyanomethylidene)azetidine-1-carboxylate (500 mg, 2.57 mmol), (4-chloro-3-methylphenyl)boronic acid (658 mg, 3.86 mmol) and potassium hydroxide (217 mg, 3.86 mmol) and dioxane (10.0 mL). The reaction vessel was then degassed and filled with nitrogen 3 times. The reaction mixture was irradiated with a microwave for 1 h at 100 °C. The mixture was concentrated under vacuum and the residue was purified by flash chromatography on silica gel eluting with ethyl acetate/petroleum ether (1/9). The appropriate fractions were combined and concentrated under vacuum to afford 547 mg (66%) of tert-butyl 3-(4-chloro-3-methylphenyl)-3-(cyanomethyl)azetidine-1-carboxylate as a yellow solid.

A 4M HCl dioxane solution (5 mL, 20 mmol) was added to a solution of tert-butyl 3-(4-chloro-3-methylphenyl)-3-(cyanomethyl)azetidine-1-carboxylate (547 mg, 1.70 mmol) in dioxane (20 mL). The mixture was stirred for 15 h at 25 °C and concentrated under vacuum. Water (5 mL) was added and the pH value of the solution was adjusted to 9 with sodium bicarbonate. The resulting mixture was concentrated under vacuum. The residue was redissolved in 10 mL of DCM and 10 mL of MeOH. The solids were filtered out. The filtrate was concentrated under vacuum to afford 2-[3-(4-chloro-3-methylphenyl)azetidin-3-yl]acetonitrile as a yellow oil (500 mg).

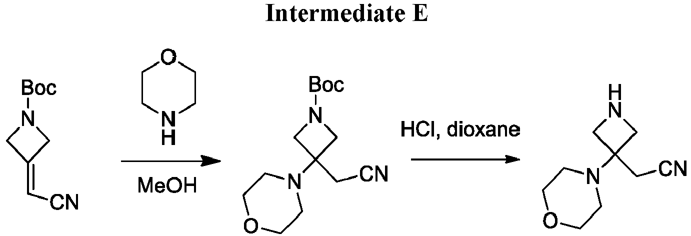

### 2- [3 -(morpholin-4-yl)azetidin-3 -yl] acetonitrile

A solution of tert-butyl 3-(cyanomethylidene)azetidine-1-carboxylate (200 mg, 1.03 mmol) and morpholine (860 mg, 9.87 mmol) in methanol (2 mL) was heated at reflux temperature overnight. The reaction mixture was cooled to room temperature and concentrated under vacuum. The residue was purified by flash chromatography on silica gel eluting with ethyl acetate/hexane (gradient, 50% ethyl acetate in hexane-100% ethyl acetate). The appropriate fractions were combined and concentrated under vacuum affording 200 mg (69%) of tert-butyl 3-(cyanomethyl)-3-(morpholin-4-yl)azetidine-1-carboxylate as a colorless oil.

Tert-butyl 3-(cyanomethyl)-3-(morpholin-4-yl)azetidine-1-carboxylate (210 mg, 0.746 mmol) was added to a 4M HCl dioxane solution (6.00 mL). The solution was stirred for 15 h at 25 °C, and concentrated under vacuum. The residue was dissolved in 5 mL of water. The pH of the solution was adjusted to 8 with potassium carbonate. The resulting mixture was concentrated under vacuum. The residue was redissolved in 30 mL of DCM and 30 mL of MeOH. The solids were filtered out and the filtrate was concentrated under vacuum affording 260 mg of 2-[3-(morpholin-4-yl)azetidin-3-yl]acetonitrile as a white solid.

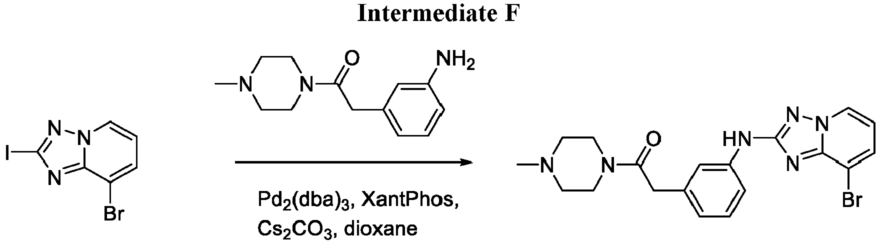

### 2-[3-([8-bromo-[1,2,4]triazolo[1,5-a]pyridin-2-yl]amino)phenyl]-1-(4-methylpiperazin-1-yl)ethan-1-one

To an 8-mL microwave vial purged with nitrogen was placed 8-bromo-2-iodo-[1,2,4]triazolo[1,5-a]pyridine (300 mg, 0.926 mmol), Pd₂(dba)₃ (80.0 mg, 0.087 mmol), 2-(3-aminophenyl)-1-(4-methylpiperazin-1-yl)ethan-1-one (200 mg, 0.857 mmol), XantPhos (99.0 mg, 0.171 mmol), Cs₂CO₃ (580 mg, 1.78 mmol) and dioxane (7.00 mL). The reaction vessel was degassed and filled with nitrogen 3 times. The solution was stirred at 60 °C overnight in an oil bath. The resulting mixture was concentrated under vacuum. The residue was purified by flash chromatography on silica gel eluting with dichloromethane/methanol (20/1) to afford 200 mg (54%) of 2-[3-([8-bromo-[1,2,4]triazolo[1,5-a]pyridin-2-yl]amino)phenyl]-1-(4-methylpiperazin-1-yl)ethan-1-one as a yellow solid.

### Example 1 (General Procedure A)

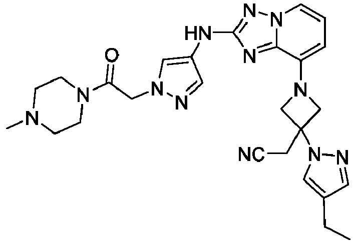

### 2-[3-(4-ethyl-1H-pyrazol-1-yl)-1-[2-([1-[2-(4-methylpiperazin-1-yl)-2-oxoethyl]-1H-pyrazol-4-yl]amino)-[1,2,4]triazolo[1,5-a]pyridin-8-yl]azetidin-3-yl]acetonitrile

Into a 30-mL microwave vial purged with nitrogen was placed 2-[4-([8-bromo-[1,2,4]triazolo[1,5-a]pyridin-2-yl]amino)-1H-pyrazol-1-yl]-1-(4-methylpiperazin-1-yl)ethan-1-one (**Intermediate A**, 1.50 g, 3.57 mmol), Cs₂CO₃ (2.34 g, 7.18 mmol), Pd₂(dba)₃ (743 mg, 0.811 mmol), BINAP (893 mg, 1.43 mmol), dioxane (20.0 mL) and 2-[3-(4-ethyl-1H-pyrazol-1-yl)azetidin-3-yl]acetonitrile (**Intermediate B**, 682 mg, 3.58 mmol). The reaction vessel was degassed and filled with nitrogen 3 times. The solution was stirred at 100 °C overnight. The resulting mixture was concentrated under vacuum. The residue was applied onto a silica gel column eluting with dichloromethane/methanol (90/10). The appropriate fractions were combined and concentrated under vacuum. The crude product was purified by Prep-HPLC (Column, XBridge Shield RP18 OBD Column, Sum, 19*150mm; mobile phase, water (0.05% NH₄OH) and ACN (15.0% ACN up to 45.0% in 8 min); Detector, UV 254/220nm) to afford the title compound (718 mg, 38%) as an off-white solid. LC/MS (Method 1, ESI): [M+H]⁺ = 529.3, R_{T}= 1.42 min; ¹H NMR (400 MHz, CD₃OD): δ (ppm) 8.00 (d, *J* = 6.4 Hz, 1 H), 7.97 (s, 1 H), 7.81(s, 1H), 7.60 (s, 1H), 7.50 (s, 1H), 6.83 (dd, *J* = 8.0, 6.8 Hz, 1H), 6.48 (d, *J* = 8.0 Hz, 1H), 5.12 (s, 2H), 4.60 (d, *J* = 8.8 Hz, 2H), 4.54 (d, *J* = 8.8 Hz, 2H), 3.63-3.54 (m, 4H), 3.50 (s, 2H), 2.56 (q, *J* =7.6 Hz, 2H), 2.50-2.44 (m, 4H), 2.33 (s, 3H), 1.24 (t, *J* = 7.6 Hz, 3H).

### Example 2 (General Procedure B)

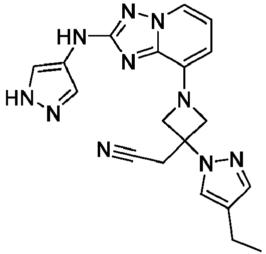

### 2-[3-(4-ethyl-1H-pyrazol-1-yl)-1-[2-[(1H-pyrazol-4-yl)amino]-[1,2,4]triazolo[1,5-a]pyridin-8-yl]azetidin-3-yl]acetonitrile

To a nitrogen flushed microwave tube was placed Pd₂(dba)₃ (700 mg, 0.764 mmol), BINAP (930 mg, 1.49 mmol), dioxane (16.0 mL) Cs₂CO₃ (2.50 g, 7.67 mmol), 2-[3-(4-ethyl-1H-pyrazol-1-yl)azetidin-3-yl]acetonitrile (**Intermediate B**, 850 mg, 4.46 mmol) and N-[8-bromo-[1,2,4]triazolo[1,5-a]pyridin-2-yl]-1-[[2-(trimethylsilyl)ethoxy]methyl]-1H-pyrazol-4-amine (1.50g, 3.66 mmol). The vessel was degased and filled with nitrogen 3 times. The reaction mixture was stirred overnight at 100 °C and allowed to cool to room temperature. The resulting mixture was concentrated under vacuum. The residue was purified by flash chromatography on silica gel eluting with ethyl acetate/petroleum ether (gradient, 0-50% ethyl acetate in petroleum ether) to afford 1.70 g (89%) of 2-[3-(4-ethyl-1H-pyrazol-1-yl)-1-[2-[(1-[[2-(trimethylsilyl)ethoxy]methyl]-1H-pyrazol-4-yl)amino]-[1,2,4]triazolo[1,5-a]pyridin-8-yl]azetidin-3-yl]acetonitrile as a yellow solid.

To a solution of 2-[3-(4-ethyl-1H-pyrazol-1-yl)-1-[2-[(1-[[2-(trimethylsilyl)ethoxy] methyl]-1H-pyrazol-4-yl)amino]-[1,2,4]triazolo[1,5-a]pyridin-8-yl]azetidin-3-yl]acetonitrile (4.35 g, 8.38 mmol) in dichloromethane 400 mL) was added trifluoroacetic acid (40 mL) at room temperature. The resulting solution was stirred at room temperature for 3 h and concentrated under vacuum. The residue was redissolved in dichloromethane (50 mL), and DIPEA (2 mL) was added. The resulting mixture was concentrated under vacuum. The residue was purified by flash chromatography on silica gel eluting with dichloromethane/methanol (3%-5% MeOH in DCM) and the appropriate fractions were combined and concentrated in vacuum. The residue was further purified by Prep-HPLC (Column, XBridge Shield RP18 OBD Column, Sum, 19^{∗}150mm; mobile phase, water (0.05% NH₄OH) and ACN (15.0% ACN up to 45.0% in 7 min); Detector, UV 254/220nm) to afford the title compound (300.2 mg, 9.2%) as a white solid. LC/MS (Method 2, ESI): [M+H]⁺ = 389.1, R_{T}= 2.42 min; ¹H NMR (400 MHz, CD₃OD): δ (ppm) 8.02 (d, *J=* 6.8 Hz, 1H), 7.92-7.81 (broad, 1H), 7.80 (s, 1H), 7.79-7.55 (broad, 1H), 7.51 (s, 1H), 6.84 (dd, *J=* 7.6, 6.8 Hz, 1H), 6.51 (d, *J* = 7.6 Hz, 1H), 4.61 (d, *J* = 8.8 Hz, 2H), 4.55 (d, *J* = 8.8 Hz, 2H), 3.55 (s, 2H), 2.56 (q, *J* = 7.6 Hz, 2H), 1.24 (t, *J=* 7.6 Hz, 3H).

### Example 3 (General Procedure C)

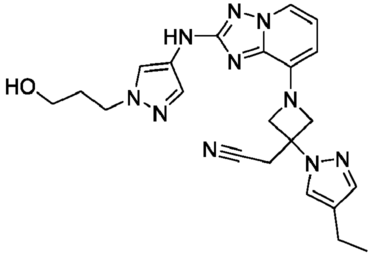

### 2-[3-(4-ethyl-1H-pyrazol-1-yl)-1-(2-[[1-(3-hydroxypropyl)-1H-pyrazol-4-yl]amino]-[1,2,4]triazolo[1,5-a]pyridin-8-yl)azetidin-3-yl]acetonitrile

To 50 mL flask (flushed with nitrogen) was added 3-bromopropan-1-ol (370 mg, 2.66 mmol), 2-[3-(4-ethyl-1H-pyrazol-1-yl)-1-[2-[(1H-pyrazol-4-yl)amino]-[1,2,4]triazolo[1,5-a]-pyridin-8-yl]azetidin-3-yl]acetonitrile (200 mg, 0.515 mmol), Cs₂CO₃ (840 mg, 2.58 mmol) and N,N-dimethylformamide (10 mL). The reaction mixture was stirred at 90 °C overnight and concentrated under vacuum. The residue was filtered through a short pad of silica gel eluting with dichloromethane/methanol (10/1). The crude product was purified by Prep-HPLC (Column, XBridge Shield RP18 OBD Column, Sum, 19*150mm; mobile phase: water (0.05% NH4OH) and ACN (15.0% ACN up to 45.0% in 7 min); Detector, UV 254/220nm) to afford the title compound (3.7 mg, 1.6%) as a white solid. LC/MS (Method 3, ESI): [M+H]⁺ = 447.2, R_{T}= 1.63 min. ¹H NMR (300 MHz, CD₃OD): δ (ppm) 8.01 (d, *J* = 6.6 Hz, 1H), 7.92 (s, 1H), 7.78 (s, 1H), 7.56 (s, 1H), 7.50 (s, 1H), 6.84 (dd, *J* = 7.5, 6.6 Hz, 1H), 6.50 (d, *J* = 7.5 Hz, 1H), 4.59 (d, *J* = 8.7 Hz, 2H), 4.54 (d, *J* = 8.7 Hz, 2H), 4.24 (t, *J* = 6.9 Hz, 2H), 3.64-3.54 (m, 4H), 2.55 (q, *J* = 7.5 Hz, 2H), 2.10-2.02 (m, 2H), 1.23 (t, *J* = 7.65 Hz, 3H).

### Example 4 (General Procedure D)

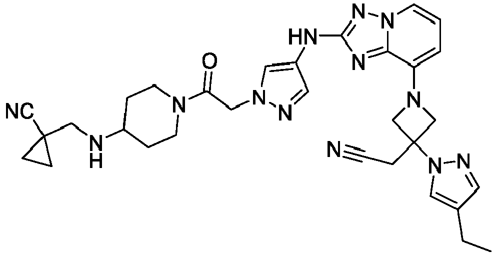

### 1-[[(1-[2-[4-([8-[3-(cyanomethyl)-3-(4-ethyl-1H-pyrazol-1-yl)azetidin-1-yl]-[1,2,4]triazolo[1,5-a]pyridin-2-yl] amino)- 1H-pyrazol-1 -yl] acetyl]piperidin-4-yl)amino]methyl]cyclopropane-1-carbonitrile

To 1-(2-bromoacetyl)piperidin-4-one (490 mg, 2.22 mmol) in a round-bottom flask was added to N-[8-bromo-[1,2,4]triazolo[1,5-a]pyridin-2-yl]-1H-pyrazol-4-amine (300 mg, 1.07 mmol), Cs₂CO₃ (1.08 g, 3.31 mmol) and N,N-dimethylformamide (15.0 mL). The solution was stirred for 1 h at 60 °C and concentrated under vacuum. The residue was purified by flash chromatography on silica gel eluting with dichloromethane/methanol (20/1). The appropriate fractions were combined and concentrated under vacuum to afford 1-[2-[4-([8-bromo-[1,2,4]triazolo[1,5-a]pyridin-2-yl]amino)-1H-pyrazol-1-yl]acetyl]piperidin-4-one as a yellow solid (260 mg).

NaBH(OAc)₃ (820 mg, 3.86 mmol) was added portion-wise to a solution of 1-[2-[4-([8-bromo-[1,2,4]triazolo[1,5-a]pyridin-2-yl]amino)-1H-pyrazol-1-yl]acetyl]piperidin-4-one (400 mg, 0.956 mmol) and 1-(aminomethyl)cyclopropane-1-carbonitrile (190 mg, 1.97 mmol) in dichloromethane (20 mL). The solution was stirred at room temperature overnight and concentrated under vacuum. The residue was purified by flash chromatography on silica gel eluting with dichloromethane/methanol (10/1). The appropriate fractions were combined and concentrated under vacuum to afford 400 mg (84%) of 1-[[(1-[2-[4-([8-bromo-[1,2,4]triazolo[1,5-a]pyridin-2-yl]amino)-1H-pyrazol-1-yl]acetyl]piperidin-4-yl)amino]methyl]cyclopropane-1-carbonitrile as a yellow solid.

To an 8-mL microwave vial purged with nitrogen gas was placed 1-[[(1-[2-[4-([8-bromo-[1,2,4]triazolo[1,5-a]pyridin-2-yl]amino)-1H-pyrazol-1-yl]acetyl]piperidin-4-yl)amino]methyl]cyclopropane-1-carbonitrile (100 mg, 0.201 mmol), 2-[3-(4-ethyl-1H-pyrazol-1-yl)azetidin-3-yl]acetonitrile (**Intermediate B**, 50.0 mg, 0.263 mmol), Cs₂CO₃ (135 mg, 0.414 mmol), Pd₂(dba)₃.CHCl₃ (42.0 mg, 0.041 mmol), BINAP (51.0 mg, 0.082 mmol) and dioxane (6.00 mL). The vessel was degassed and filled with nitrogen 3 times. The mixture was stirred at 100 °C overnight. The mixture was concentrated under vacuum. The residue was purified by flash chromatography on silica gel eluting with dichloromethane/methanol (10/1). The appropriate fractions were combined and concentrated under reduced pressure. The crude product was purified by Prep-HPLC (Column, XBridge Shield RP18 OBD Column, Sum, 19*150mm; mobile phase: water (0.05% NH₄OH) and ACN (10.0% ACN up to 50.0% in 10 min); Detector, UV 254/220nm) to afford the title compound (59.5 mg, 49%) as a white solid. LC/MS (Method 4, ESI): [M+H]⁺ = 608.4, R_{T}= 2.03 min; ¹H NMR (300 MHz, CD₃OD): δ (ppm) 8.00 (d, *J* = 6.3 Hz, 1H), 7.96 (s, 1H), 7.80 (s, 1H), 7.59 (s, 1H), 7.50 (s, 1H), 6.83 (dd, *J* = 6.6, 6.3 Hz, 1H), 6.48 (d, *J* = 6.6 Hz, 1H), 5.15 (d, *J* = 16.5 Hz, 1H), 5.07 (d, *J* = 16.5 Hz, 1H), 4.60 (d, *J* = 8.7 Hz, 2H), 4.54 (d, *J* = 8.7 Hz, 2H), 4.40-4.30 (m, 1H), 4.00-3.85 (m, 1H), 3.60-3.50 (m, 3H), 3.30-3.15 (m, 1H), 2.87-2.83 (m, 2H), 2.73 (s, 2H), 2.54 (q, *J* = 7.5 Hz, 2H), 2.05-1.85 (m, 2H), 1.40-1.26 (m, 1H), 1.25-1.20 (m, 5H), 0.98 (dd, *J* = 7.2, 4.8 Hz, 2H).

### Example 5 (General Procedure E)

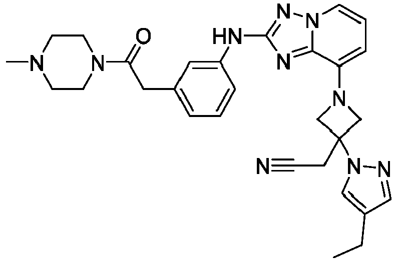

### 2-[3-(4-ethyl-1H-pyrazol-1-yl)-1-[2-([3-[2-(4-methylpiperazin-1-yl)-2-oxoethyl]phenyl]amino)-[1,2,4]triazolo[1,5-a]pyridin-8-yl]azetidin-3-yl]acetonitrile

To an 8-mL microwave vial purged with nitrogen was placed 2-[3-([8-bromo-[1,2,4]triazolo[1,5-a]pyridin-2-yl]amino)phenyl]-1-(4-methylpiperazin-1-yl)ethan-1-one (**Intermediate F**, 150 mg, 0.349 mmol), 2-[3-(4-ethyl-1H-pyrazol-1-yl)azetidin-3-yl]acetonitrile (**Intermediate B**, 70.0 mg, 0.368 mmol), Pd₂(dba)₃ (70.0 mg, 0.076 mmol), BINAP (90.0 mg, 0.145 mmol), Cs₂CO₃ (250 mg, 0.767 mmol) and dioxane (6 mL). The reaction vessel was degassed and filled with nitrogen 3 times. The solution was stirred at 100 °C overnight in an oil bath. The resulting mixture was concentrated under vacuum. The residue was passed through a short pad of silica gel eluting with dichloromethane/methanol (10/1). The appropriate fractions were combined and concentrated under vacuum. The residue was further purified by Prep-HPLC (Column, XBridge Shield RP18 OBD Column, Sum, 19*150mm; mobile phase, water (0.05% NH₄OH) and ACN (15.0% ACN up to 45.0% in 9 min); Detector, UV 254/220nm) to afford the title compound (57.4 mg, 30%) as a white solid. LC/MS (Method 5, ESI): [M+H]⁺ = 539.4, R_{T}= 2.45 min; ¹H NMR (300 MHz, CDCl₃): δ (ppm) 7.96 (d, *J* = 6.9 Hz, 1H), 7.64 (s, 1H), 7.55 (s, 1H), 7.44 (s, 1H), 7.41 (d, *J* = 8.1 Hz, 1H), 7.28 (dd, *J* = 8.1, 7.5 Hz, 1H), 6.92 (s, 1H), 6.83 (d, *J* = 7.5 Hz, 1H), 6.75 (dd, *J* = 7.5, 6.9 Hz, 1H), 6.31 (d, *J* = 7.5 Hz, 1H), 4.61 (s, 4H), 3.76 (s, 2H), 3.75-3.65 (m, 2H), 3.55-3.45 (m, 2H), 3.41 (s, 2H), 2.50 (q, *J* = 7.5 Hz, 2H), 2.45-2.35 (m, 2H), 2.27-2.15 (m, 5H), 1.20 (t, *J* = 7.5 Hz, 3H).

### Example 6 (General Procedure F)

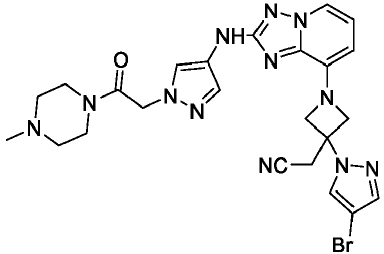

### 2-[3-(4-bromo-1H-pyrazol-1-yl)-1-[2-([1-[2-(4-methylpiperazin-1-yl)-2-oxoethyl]-1H-pyrazol-4-yl]amino)-[1,2,4]triazolo[1,5-a]pyridin-8-yl]azetidin-3-yl]acetonitrile

Sodium hydride (60% in mineral oil, 384 mg, 9.44 mmol) was added in portions to a solution of 2-[4-([8-bromo-[1,2,4]triazolo[1,5-a]pyridin-2-yl]amino)-1H-pyrazol-1-yl]-1-(4-methylpiperazin-1-yl)ethan-1-one (**Intermediate A**, 2.00 g, 4.77 mmol) in DMF (30 mL) under nitrogen at 0°C. The resulting solution was stirred for 0.5 h at room temperature before (Boc)₂O (2.10 g, 9.62 mmol) was added. The solution was stirred for 2 h at room temperature. Water (10 mL) was added and the mixture was concentrated under vacuum. The residue was purified by flash chromatography on silica gel eluting with dichloromethane/methanol (gradient, 5% MeOH in DCM to 10% MeOH in DCM). The appropriate fractions were combined and concentrated under vacuum to afford 2.20 g (89%) of tert-butyl N-[8-bromo-[1,2,4]triazolo[1,5-a]pyridin-2-yl]-N-[1-[2-(4-methylpiperazin-1-yl)-2-oxoethyl]-1H-pyrazol-4-yl]carbamate as a light yellow solid. LC/MS (Method 6, ESI): [M+H]⁺ = 519.2, R_{T} = 0.96 min.

To a 10-mL microwave vial purged nitrogen was placed tert-butyl N-[8-bromo-[1,2,4]triazolo[1,5-a]pyridin-2-yl]-N-[1-[2-(4-methylpiperazin-1-yl)-2-oxoethyl]-1H-pyrazol-4-yl]carbamate (200 mg, 0.385 mmol), Pd₂(dba)₃ (70.0 mg, 0.0760 mmol), BINAP (96.0 mg, 0.154 mmol), 3-[(tert-butyldiphenylsilyl)oxy]azetidine (160 mg, 0.514 mmol), Cs₂CO₃ (252 mg, 0.773 mmol) and dioxane (4.00 mL). The resulting solution was stirred at 100 °C overnight in an oil bath. The reaction mixture was cooled and the mixture was concentrated under vacuum. The reaction was repeated seven times on the same scale above. The residue was purified by flash chromatography on silica gel eluting with dichloromethane/methanol (gradient, 5% MeOH in DCM to 10% MeOH in DCM). The appropriate fractions were combined and concentrated under vacuum to afford tert-butyl N-(8-[3-[(tert-butyldiphenylsilyl)oxy]azetidin-1-yl]-[1,2,4]triazolo[1,5-a]pyridin-2-yl)-N-[1-[2-(4-methylpiperazin-1-yl)-2-oxoethyl]-1H-pyrazol-4-yl]carbamate as a light yellow solid (1.94 g). LC/MS (Method 6, ESI): [M+H]⁺ = 750.2, R_{T} = 1.40 min.

TBAF (2.70 mL, 1 M in THF, 2.70 mmol) was added to a solution of tert-butyl N-(8-[3-[(tert-butyldiphenylsilyl)oxy]azetidin-1-yl]-[1,2,4]triazolo[1,5-a]pyridin-2-yl)-N-[1-[2-(4-methylpiperazin-1-yl)-2-oxoethyl]-1H-pyrazol-4-yl]carbamate (2.00 g, 2.66 mmol) in anhydrous THF (30 mL) under nitrogen. The solution was stirred for 2 h at room temperature. The mixture was concentrated under vacuum. The residue was purified by flash chromatography on silica gel eluting with dichloromethane/methanol (gradient, 10% MeOH in DCM to 20% MeOH in DCM). The appropriate fractions were combined and concentrated under vacuum to afford 1.35 g (99%) of tert-butyl N-[8-(3-hydroxyazetidin-1-yl)-[1,2,4]triazolo[1,5-a]pyridin-2-yl]-N-[1-[2-(4-methylpiperazin-1-yl)-2-oxoethyl]-1H-pyrazol-4-yl]carbamate as a light yellow solid.

NMO (430 mg, 3.67 mg) was added to a solution of tert-butyl N-[8-(3-hydroxyazetidin-1-yl)-[1,2,4]triazolo[1,5-a]pyridin-2-yl]-N-[1-[2-(4-methylpiperazin-1-yl)-2-oxoethyl]-1H-pyrazol-4-yl]carbamate (1.55 g, 3.03 mmol) in dichloromethane (30 mL) and TPAP (55.0 mg, 0.157 mmol). The solution was stirred at room temperature overnight and the mixture was concentrated under vacuum. The residue was purified by flash chromatography on silica gel eluting with dichloromethane/methanol (gradient, 10% MeOH in DCM to 20% MeOH in DCM). The appropriate fractions were combined and concentrated under vacuum to afford 765 mg (50%) of tert-butyl N-[1-[2-(4-methylpiperazin-1-yl)-2-oxoethyl]-1H-pyrazol-4-yl]-N-[8-(3-oxoazetidin-1-yl)-[1,2,4]triazolo[1,5-a]pyridin-2-yl]carbamate as a light yellow solid.

To a 50-mL round-bottom flask purged with nitrogen was placed diethyl (cyanomethyl)phosphonate (292 mg, 0.824 mmol) in THF (10 mL). To this solution sodium hydride (60.0 mg, 1.50 mmol, 60% in mineral oil) was added at 0°C. The resulting solution was stirred for 0.5 h at room temperature. Half of the resulting solution was added into tert-butyl N-[1-[2-(4-methylpiperazin-1-yl)-2-oxoethyl]-1H-pyrazol-4-yl]-N-[8-(3-oxoazetidin-1-yl)-[1,2,4]triazolo[1,5-a]pyridin-2-yl]carbamate (765 mg, 1.50 mmol) in THF (10 mL). The resulting solution was stirred for an additional 2 h at room temperature. Water (1 mL) was added. The resulting mixture was concentrated under vacuum. The residue was purified by flash chromatography on silica gel eluting with dichloromethane/methanol (6/1). The appropriate fractions were combined and concentrated under vacuum to afford tert-butyl N-[8-[3-(cyanomethylidene)azetidin-1-yl]-[1,2,4]triazolo[1,5-a]pyridin-2-yl]-N-[1-[2-(4-methylpiperazin-1-yl)-2-oxoethyl]-1H-pyrazol-4-yl]carbamate as a light yellow solid (635 mg, 79%). LC/MS (Method 6, ESI): [M+H]⁺ =533.4, R_{T} = 1.01 min.

4-Bromo-1H-pyrazole (102 mg, 0.694 mmol) was added to a solution of tert-butyl N-[8-[3-(cyanomethylidene)azetidin-1-yl]-[1,2,4]triazolo[1,5-a]pyridin-2-yl]-N-[1-[2-(4-methylpiperazin-1-yl)-2-oxoethyl]-1H-pyrazol-4-yl]carbamate (75.0 mg, 0.141 mmol) in CH₃CN (5 mL) under nitrogen. DBU (15.0 mg, 0.0990 mmol) was added and the mixture was stirred for 1.5 h at 50 °C in an oil bath. The reaction mixture was cooled and concentrated under vacuum. The residue was purified by flash chromatography on silica gel eluting with dichloromethane/methanol (6/1). The appropriate fractions were combined and concentrated under vacuum to afford 85.0 mg (89%) of tert-butyl N-[8-[3-(4-bromo-1H-pyrazol-1-yl)-3-(cyanomethyl)azetidin-1-yl]-[1,2,4]triazolo[1,5-a]pyridin-2-yl]-N-[1-[2-(4-methylpiperazin-1-yl)-2-oxoethyl]-1H-pyrazol-4-yl]carbamate as a light yellow oil.

Trifluoroacetic acid (0.40 mL) was added dropwise to a solution of tert-butyl N-[8-[3-(4-bromo-1H-pyrazol-1-yl)-3-(cyanomethyl)azetidin-1-yl]-[1,2,4]triazolo[1,5-a]pyridin-2-yl]-N-[1-[2-(4-methylpiperazin-1-yl)-2-oxoethyl]-1H-pyrazol-4-yl]carbamate (75.0 mg, 0.110 mmol) in dichloromethane (8.00 mL). The solution was stirred for 2 h at room temperature. The mixture was concentrated under vacuum before DCM (5 mL) and DIPEA (0.20 mL) were added. The resulting mixture was concentrated under vacuum. The residue was purified by flash chromatography on silica gel eluting with dichloromethane/methanol (gradient, 10% MeOH in DCM to 20% MeOH in DCM). The appropriate fractions were combined and concentrated under vacuum. The crude product was further purified by Prep-HPLC (Column, XBridge Shield RP18 OBD Column, Sum, 19*150mm; mobile phase, water (0.05% NH₄OH) and ACN (15.0% ACN up to 45.0% in 7 min); Detector, UV 254/220nm) to afford the title compound (18.3 mg, 29%) as an off-white solid. LC/MS (Method 1, ESI): [M+H]⁺ = 581.2; R_{T}= 1.43 min; ¹H NMR (300 MHz, CD₃OD): δ (ppm) 8.18 (s, 1H), 7.99 (d, *J* = 6.6 Hz, 1H), 7.95 (s, 1H), 7.63 (s, 1H), 7.57 (s, 1H), 6.82 (dd, *J* = 7.8, 6.6 Hz, 1H), 6.48 (d, *J* = 7.8 Hz, 1H), 5.10 (s, 2H), 4.62 (d, *J* = 8.7 Hz, 2H), 4.53 (d, *J* = 8.7 Hz, 2H), 3.67-3.57 (m, 4H), 3.55 (s, 2H), 2.53-2.39 (m, 4 H), 2.30 (s, 3H).

### Example 127 (General Procedure G)

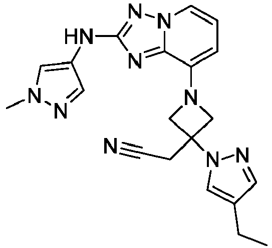

### 2-(3-(4-ethyl-1H-pyrazol-1-yl)-1-(2-((1-methyl-1H-pyrazol-4-yl)amino)-[1,2,4]triazolo[1, 5-a]pyridin-8-yl)azetidin-3 -yl)acetonitrile

To a solution of N-[8-bromo-[1,2,4]triazolo[1,5-a]pyridin-2-yl]-1-methyl-1H-pyrazol-4-amine (1.00 g, 3.41 mmol, Intermediate G) in dioxane (12.0 mL) was added 2-[3-(4-ethyl-1H-pyrazol-1-yl)azetidin-3-yl]acetonitrile (780 mg, 4.10 mmol, Intermediate B), Pd₂(dba)₃.CHCl₃ (710 mg, 0.686 mmol), BINAP (852 mg, 1.36 mmol) and Cs₂CO₃ (2.23 g, 6.84 mmol) under nitrogen. The resulting solution was stirred overnight at 100 °C in an oil bath. The reaction mixture was cooled. The resulting mixture was concentrated under vacuum. The residue was applied onto a silica gel column eluting with ethyl acetate/petroleum ether (1/1∼3/1). The appropriate fractions were combined and concentrated under vacuum. The crude product was further purified by Prep-HPLC with the following conditions: Column, XBridge Prep C18 OBD Column, 30*100mm,5um; mobile phase, Water(10MMOL/L NH₄HCO₃) and ACN (25.0% ACN up to 57.0% in 5 min); Detector, UV 254/220nm, followed by recrystallization in t-BuOMe (3 ml/100 mg) to give 714 mg (52%) of 2-[3-(4-ethyl-1H-pyrazol-1-yl)-1-[2-[(1-methyl-1H-pyrazol-4-yl)amino]-[1,2,4]triazolo[1,5-a]pyridin-8-yl]azetidin-3-yl]acetonitrile as an off-white solid. LC/MS (Method 2, ESI): [M+H]⁺ = 403.2, R_{T}= 2.71 min; ¹H NMR (400 MHz, CD₃OD): δ (ppm) 8.01 (dd, *J* = 6.8, 0.8 Hz, 1H), 7.85 (d, *J* = 0.8 Hz, 1H), 7.79 (d, *J* = 0.8 Hz, 1H), 7.54 (d, *J* = 0.8 Hz, 1H), 7.50 (d, *J* = 0.8 Hz, 1H), 6.83 (dd, *J* = 8.0, 6.8 Hz, 1H), 6.49 (dd, *J* = 8.0, 0.8 Hz, 1H), 4.58 (d, *J* = 8.8 Hz, 2H), 4.53 (d, *J* = 8.8 Hz, 2H), 3.88 (s, 3H), 3.54 (s, 2H), 2.55 (q, *J* = 7.6 Hz, 2H), 1.23 (t, *J* = 7.6 Hz, 3H).

### Example 152 (General Procedure H)

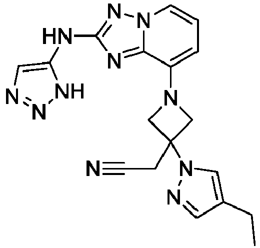

### 2-(1-(2-((1H-1,2,3-triazol-5-yl)amino)-[1,2,4]triazolo[1,5-a]pyridin-8-yl)-3-(4-ethyl-1H-pyrazol-1-yl)azetidin-3-yl)acetonitrile

To a solution of 5-nitro-1H-1,2,3-triazole (300 mg, 2.63 mmol) in tetrahydrofuran (20.0 mL) was added into sodium hydride (215 mg, 60% dispersion in mineral oil, 5.37 mmol). Then [2-(chloromethoxy)ethyl]trimethylsilane (700 mg, 4.19 mmol) was added in several batches. The resulting solution was stirred overnight at room temperature. The reaction was then quenched by the addition of 1 ml of water. The resulting mixture was concentrated under vacuum. The residue was applied onto a silica gel column with ethyl acetate/petroleum ether (1:4) to obtain 400 mg (62%) of 5-nitro-1-[[2-(trimethylsilyl)ethoxy]methyl]-1H-1,2,3-triazole as a yellow solid. TLC: R_{f} = 0.3; ethyl acetate/hexane =1/8.

To a solution of 5-nitro-1-[[2-(trimethylsilyl)ethoxy]methyl]-1H-1,2,3-triazole (400 mg, 1.63 mmol) in ethanol (20 mL) and water (2.0 mL) were added NH₄Cl (710 mg, 13.2 mmol) and iron powder (740 mg, 13.2 mmol). The resulting mixture was stirred for 2 h at 90°C in an oil bath, allowed to cool to room temperature, and filtered through celite, washed with ethanol. The filtrate was concentrated under vacuum. The residue was applied onto a silica gel column with ethyl acetate/petroleum ether (4:1) to give 300 mg (85%) of 1-[[2-(trimethylsilyl)ethoxy]methyl]-1H-1,2,3-triazol-5-amine as yellow oil. TLC: R_{f} = 0.3; ethyl acetate/hexane =1/1.

To a solution of 1-[[2-(trimethylsilyl)ethoxy]methyl]-1H-1,2,3-triazol-5-amine (250 mg, 1.16 mmol) in dioxane (5.0 mL) were added Xantphos (75.0 mg, 0.130 mmol), 8-bromo-2-iodo-[1,2,4]triazolo[1,5-a]pyridine (450 mg, 1.38 mmol), Pd₂(dba)₃.CHCl₃ (60.0 mg, 0.0580 mmol) and Cs₂CO₃ (800 mg, 2.45 mmol) under nitrogen. The resulting mixture was stirred overnight at 70°C in an oil bath. The resulting mixture was concentrated under vacuum. The residue was applied onto a silica gel column eluting with ethyl acetate/petroleum ether (1:1) to obtain 200 mg (42%) of N-[8-bromo-[1,2,4]triazolo[1,5-a]pyridin-2-yl]-1-[[2-(trimethylsilyl)ethoxy]methyl]-1H-1,2,3-triazol-5-amine as a yellow solid. TLC: R_{f} = 0.3; ethyl acetate/hexane =1/1.

To a solution of N-[8-bromo-[1,2,4]triazolo[1,5-a]pyridin-2-yl]-1-[[2-(trimethylsilyl)ethoxy]methyl]-1H-1,2,3-triazol-5-amine (100 mg, 0.244 mmol) in dioxane (5.0 mL) were added BINAP (33.0 mg, 0.0530 mmol), 2-[3-(4-ethyl-1H-pyrazol-1-yl)azetidin-3-yl]acetonitrile (55.0 mg, 0.289 mmol), Pd₂(dba)₃.CHCl₃ (30.0 mg, 0.0290 mmol) and Cs₂CO₃ (160 mg, 0.491 mmol) under nitrogen. The resulting mixture was stirred overnight at 100°C in an oil bath, and allowed to cool to room temperature and concentrated under vacuum. The residue was applied onto a silica gel column eluting with ethyl acetate/petroleum ether (2:1) to give 80 mg (63%) of 2-[3-(4-ethyl-1H-pyrazol-1-yl)-1-[2-[(1-[[2-(trimethylsilyl)ethoxy]methyl]-1H-1,2,3-triazol-5-yl)amino]-[1,2,4]triazolo[1,5-a]pyridin-8-yl]azetidin-3-yl]acetonitrile as a yellow solid. TLC: R_{f} = 0.3; ethyl acetate/hexane =2/1.

Trifluoroacetic acid (1 mL) was added to a solution of 2-[3-(4-ethyl-1H-pyrazol-1-yl)-1-[2-[(1-[[2-(trimethylsilyl)ethoxy]methyl]-1H-1,2,3-triazol-5-yl)amino]-[1,2,4]triazolo[1,5-a]pyridin-8-yl]azetidin-3-yl]acetonitrile (80.0 mg, 0.154 mmol) in dichloromethane (10 mL). The solution was stirred for 4 h at room temperature. The resulting mixture was concentrated under vacuum. The pH value of the solution was adjusted to 7 with DIPEA. The residue was applied onto a silica gel column eluting with ethyl acetate/petroleum ether (4:1). The appropriate fractions were combined and concentrated under vacuum. The crude product was further purified by Prep-HPLC with the following conditions (2#-AnalyseHPLC-SHIMADZU(HPLC-10)): Column, XBridge Shield RP₁₈ OBD Column, Sum, 19^{∗}150mm; mobile phase, Waters(0.05%NH₃H₂O) and ACN (15.0% ACN up to 55.0% in 10 min); Detector, UV 254/220nm to obtain 20 mg (33%) of 2-[3-(4-ethyl-1H-pyrazol-1-yl)-1-[2-[(1H-1,2,3-triazol-5-yl)amino]-[1,2,4]triazolo[1,5-a]pyridin-8-yl]azetidin-3-yl]acetonitrile as a white solid. LC/MS (Method 3, ESI): [M+H]⁺ = 390.2, R_{T}= 1.52 min; ¹H NMR (400 MHz, DMSO-*d₆*): δ (ppm) 14.19 (s, 1H), 10.01 (s, 1H), 8.16 (dd, *J=* 6.8, 1.0 Hz, 1H), 8.00 (s, 1H), 7.96 (d, *J* = 0.8 Hz, 1H), 7.48 (d, *J* = 0.8 Hz, 1H), 6.88 (dd, *J* = 7.6, 6.8 Hz, 1H), 6.48 (dd, *J* = 7.6, 1.0 Hz, 1H), 4.61 (d, *J* = 8.8 Hz, 2H), 4.49 (d, *J* = 8.8 Hz, 2H), 3.65 (s, 2H), 2.47 (q, *J* = 7.6 Hz, 2H), 1.16 (t, *J* = 7.6 Hz, 3H).

### Examples 153 (General Procedure I)

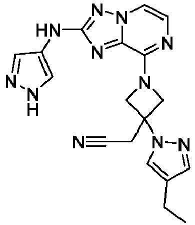

### 2-(1-(2-((1H-pyrazol-4-yl)amino)-[1,2,4]triazolo[1,5-a]pyrazin-8-yl)-3-(4-ethyl-1H-pyrazol-1-yl)azetidin-3 -yl)acetonitrile

A solution of 3-chloropyrazin-2-amine (2000 mg, 15.44 mmol) in dioxane (80 mL), was added ethoxycarbonyl isothiocyanate (2227 mg, 2.01 mL, 16.98 mmol) at 25 °C. The reaction mixture was stirred at room temperature for 12 hours. Then the reaction mixture was concentrated down to dry. The residue was used in the next step without further purification. LC/MS (Method A, ESI): [M+H]⁺ = 261.0, R_{T}= 1.17 min.

To a solution of hydroxylamine hydrochloride (5364 mg, 77.19 mmol), and N,N-diisopropylethylamine (8.08 mL, 46.31 mmol) in ethanol (24 mL) and methanol (24 mL), was added the solution of ethyl N-[(3-chloropyrazin-2-yl)carbamothioyl]carbamate (4024 mg, 15.44 mmol) in ethanol (24 mL) and methanol (24 mL). The reaction mixture was stirred at room temperature for 1 hour. Then the reaction mixture was heated to 60 °C for 3 hours. The reaction mixture was cooled down, was concentrated down to dry. The residue was dissolved into ethyl acetate, washed with water and saturated aqueous sodium chloride solution. The organic was dried over magnesium sulfate, filtered, and concentrated under reduced pressure to afford 8-chloro-[1,2,4]triazolo[1,5-a]pyridin-2-amine (725 mg, 28%) as a tan solid, which was used in the next step without further purification. LC/MS (Method A, ESI): [M+H]⁺ = 170.2, R_{T}= 1.01 min. 1HNMR (500 MHz, DMSO-*d*6) δ 8.74 (d, *J=* 4.2 Hz, 1H), 7.81 (d, *J=* 4.3 Hz, 1H), 6.71 (s, 2H). Total yield is 28% after two steps.

A solution of 2-(3-(4-ethyl-1H-pyrazol-1-yl)azetidin-3-yl)acetonitrile (185 mg, 0.97 mmol), 8-chloro-[1,2,4]triazolo[1,5-a]pyrazin-2-amine (181 mg, 1.07 mmol), N,N-diisopropylethylamine (1.36 mL, 7.78 mmol) in dimethyl sulfoxide (6 mL) was stirred at 180 °C in microwave tube for 40 minutes. After completion, ethyl acetate was added to the reaction mixture, washed with water and saturated aqueous sodium chloride solution. The organic was dried over magnesium sulfate, filtered, and concentrated. The residue was purified by column chromatography (silica gel, 100-200 mesh, 0 to 20% methonal in dichloromethane) affording 2-(1-(2-amino-[1,2,4]triazolo[1, 5-a]pyrazin-8-yl)-3 -(4-ethyl- 1H-pyrazol-1 -yl)azetidin-3 - yl)acetonitrile (110 mg, 35%) as an off-white solid. LC/MS (Method A, ESI): [M+H]⁺ = 324.2, R_{T}= 1.28 min.

To a solution of 2-(1-(2-amino-[1,2,4]triazolo[1,5-a]pyrazin-8-yl)-3-(4-ethyl-1H-pyrazol-1-yl)azetidin-3-yl)acetonitrile (188 mg, 0.58 mmol) and p-toluene sulphonic acid (425 mg, 2.23 mmol) in acetonitrile (10 mL) was added a solution of potassium iodide (318 mg, 1.92 mmol) and sodium nitrite (101 mg, 1.46 mmol) in water (1.5 mL) at 24 °C. After 18 h, isopropyl acetate was added to the reaction mixture. The resulting solution was washed with water (2x) and saturated aqueous sodium chloride solution. The organic was dried over magnesium sulfate, filtered, and concentrated. The residue was purified by column chromatography (silica gel, 100-200 mesh, 0 to 20% methonal in dichloromethane) affording 2-(3-(4-ethyl-1H-pyrazol-1-yl)-1-(2-iodo-[1,2,4]triazolo[1,5-a]pyrazin-8-yl)azetidin-3-yl)acetonitrile (115 mg, 46%) as a light orange solid. LC/MS (Method A, ESI): [M+H]⁺ = 435.1, R_{T}= 1.56 min.

Tris(dibenzylideneacetone)dipalladium(0) (8.0 mg, 0.0092 mmol), 4,5-bis(diphenylphosphino)-9,9-dimethylxanthene (5.0 mg, 0.0092 mmol), cesium carbonate (120 mg, 0.37 mmol), palladium(ii) acetate (2 mg, 0.0092 mmol), 2-(3-(4-ethyl-1H-pyrazol-1-yl)-1-(2-iodo-[1,2,4]triazolo[1,5-a]pyrazin-8-yl)azetidin-3-yl)acetonitrile (40 mg, 0.092 mmol), 1-(2-trimethylsilylethoxymethyl)pyrazol-4-amine (79 mg, 0.37 mmol) were added to a screw-cap vial. Nitrogen was then purged through the reaction vial for 5 min. The solids were dissolved in 1,4-dioxane (1 mL) and 1,2-dimethoxyethane (1 mL). The reaction flask was purged with Nitrogen. The vial was placed in a 90 °C heating block and was allowed to stir for 1h. The crude residue was dissolved in isopropyl acetate, and filtered through Celite. The crude residue was purified by flash column chromatography (silica gel, 0 to 20% methonal in dichloromethane) to afford 2-(3-(4-ethyl-1H-pyrazol-1-yl)-1-(2-((1-((2-(trimethylsilyl)ethoxy)methyl)-1H-pyrazol-4-yl)amino)-[1,2,4]triazolo[1,5-a]pyrazin-8-yl)azetidin-3-yl)acetonitrile (23 mg, 48%). LC/MS (Method A, ESI): [M+H]⁺ = 520.3, R_{T}= 1.78 min.

To a solution of 2-(3-(4-ethyl-1H-pyrazol-1-yl)-1-(2-((1-((2-(trimethylsilyl)ethoxy)methyl)-1H-pyrazol-4-yl)amino)-[1,2,4]triazolo[1,5-a]pyrazin-8-yl)azetidin-3-yl)acetonitrile (15 mg, 0.029 mmol) in dichloromethane (2 mL) was added trifluoroacetic acid (0.2 mL). The resulting solution was stirred for 2 h at room temperature under N2. The reaction mixture was concentrated down to dry. To this residue was added dichloromethane (0.5 mL), and N,N-diisopropylethylamine (0.02 mL). The resulting solution was stirred for 5 minutes at room temperature, then was concentrated down to dry. The residue was purified by preparative RP-HPLC (5-50% acetonitrile in water + 0.1% Ammonium Hydroxide, Gemini-NX C18 Sum, 110 A, 50 x 30 mm, 10 mins) to afford 2-(1-(2-((1H-pyrazol-4-yl)amino)-[1,2,4]triazolo[1,5-a]pyrazin-8-yl)-3-(4-ethyl-1H-pyrazol-1-yl)azetidin-3-yl)acetonitrile (5 mg, 36%). LC/MS (Method 8, ESI): [M+H]⁺ = 309.2, R_{T}= 1.37 min. ¹H NMR (400 MHz, DMSO-*d*₆) δ 9.28 (s, 1H), 8.11 (d, *J=* 4.5 Hz, 1H), 7.97 (s, 1H), 7.69 - 7.66 (m, 2H), 7.51 (d, *J* = 4.6 Hz, 1H), 7.48 (s, 1H), 4.82 - 4.75 (m, 2H), 4.70 - 4.63 (m, 2H), 3.71 (s, 2H), 2.49 - 2.42 (m, 2H), 1.16 (t, *J* = 7.5 Hz, 3H).

### LC/MS Conditions

### LC/MS Method 1:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.0 | 95 | 5 |
| 2.20 | 1.0 | 0 | 100 |
| 3.20 | 1.0 | 0 | 100 |
| 3.30 | 1.0 | 95 | 5 |

| | | | |
|---|---|---|---|
| Detection - UV (254 nm) and ELSD | | | |

### LC/MS Method 2:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.2 | 95 | 5 |
| 3.50 | 1.2 | 30 | 70 |
| 3.70 | 1.2 | 0 | 100 |
| 4.50 | 1.2 | 0 | 100 |
| 4.75 | 1.2 | 95 | 5 |

| | | | |
|---|---|---|---|
| Detection - UV (254 nm) and ELSD | | | |

### LC/MS Method 3:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.2 | 95 | 5 |
| 2.00 | 1.2 | 5 | 95 |
| 2.70 | 1.2 | 5 | 95 |
| 2.75 | 1.2 | 95 | 5 |

| | | | |
|---|---|---|---|
| Detection - UV (254 nm) and ELSD | | | |

### LC/MS Method 4:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.0 | 80 | 20 |
| 3.00 | 1.0 | 60 | 40 |
| 3.60 | 1.0 | 0 | 100 |
| 4.60 | 1.0 | 0 | 100 |
| 4.80 | 1.0 | 95 | 5 |

| | | | |
|---|---|---|---|
| Detection - UV (254 nm) and ELSD | | | |

### LC/MS Method 5:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.0 | 95 | 5 |
| 3.50 | 1.0 | 30 | 70 |
| 3.80 | 1.0 | 0 | 100 |
| 4.60 | 1.0 | 0 | 100 |
| 4.75 | 1.0 | 95 | 5 |

| | | | |
|---|---|---|---|
| Detection - UV (254 nm) and ELSD | | | |

### LC/MS Method 6:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.2 | 95 | 5 |
| 1.10 | 1.2 | 0 | 100 |
| 1.70 | 1.2 | 0 | 100 |
| 1.75 | 1.2 | 95 | 5 |

| | | | |
|---|---|---|---|
| Detection - UV (254 nm) and ELSD | | | |

### LC/MS Method 7:

SHIMADZU 20A HPLC with a C18-reverse-phase column (50 x 3.0 mm Shim-pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow mL/min | %A | %B |
|---|---|---|---|
| 0.01 | 1.0 | 95 | 5 |
| 1.20 | 1.0 | 0 | 100 |
| 2.20 | 1.0 | 0 | 100 |
| 2.30 | 1.0 | 95 | 5 |

### LC/MS Method 8:

SHIMADZU LCMS-2020 with a C18-reverse-phase column (Waters BEH 30 x 2.1mm, 1.7 µm particle size ) , elution with solvent A: water + 0.1% formic acid; solvent B: acetonitrile + 0.1% formic acid. Gradient:

| Gradient - Time ( min ) | flow ml/min | %A | %B |
|---|---|---|---|
| 0 | 0.7 | 98 | 2 |
| 2 | 0.7 | 2 | 98 |
| 2.19 | 0.7 | 2 | 98 |
| 2.2 | 0.7 | 98 | 2 |
| 2.5 | 0.7 | 98 | 2 |

| | | | |
|---|---|---|---|
| Detection - UV (254 nm ) | | | |

¹H NMR spectra were recorded at ambient temperature using a a Bruker Avance III 300 (300MHz) spectrometer with a 5mm Broadband liquid probe BBFO with ATM+Z and a Bruker Avance III HD (400MHz) spectrometer with a 5mm Broadband liquid probe BBFO with ATM+Z.

The examples in the following table were prepared using similar methods as described above. Compounds listed as using General Procedure A can be prepared by coupling Intermediate A or other substituted N-[8-bromo-[1,2,4]triazolo[1,5-a]pyridin-2-yl]-1H-pyrazol-4-amine with various azetidine intermediates. Different classes of azetidine intermediate can be prepared by the general methods described above.

| **Ex.** | **General Procedur e** | **Structure** | **m/z** |
|---|---|---|---|
| **7** | A | 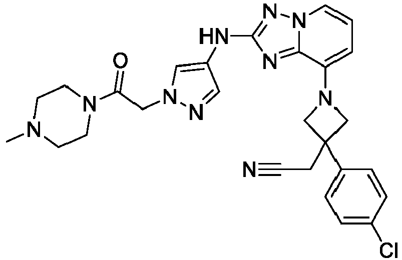 | 545. 3 |
| **8** | A | 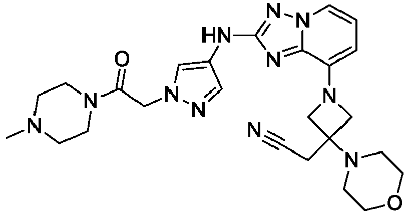 | 520. 4 |
| **9** | A | 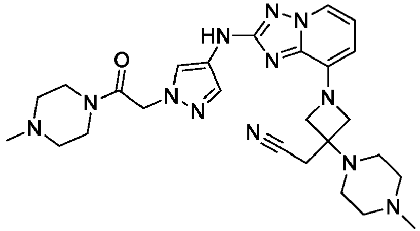 | 533. 4 |
| **10** | A | 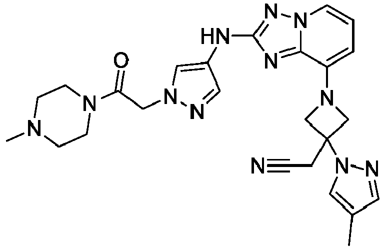 | 515. 4 |
| **11** | A | 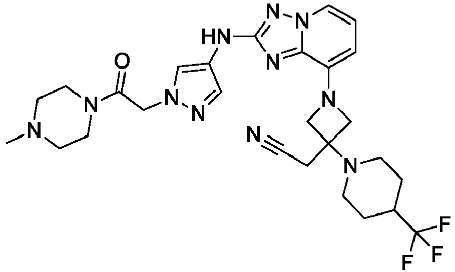 | 586. 4 |
| **12** | A | 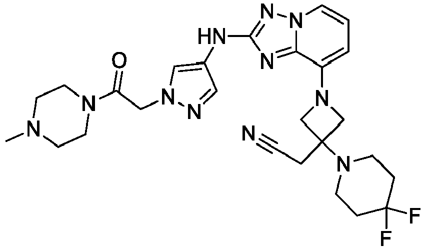 | 554. 4 |
| **13** | A | 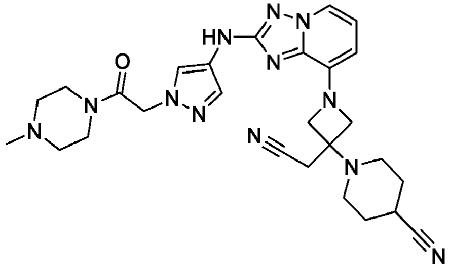 | 543. 4 |
| **14** | A | 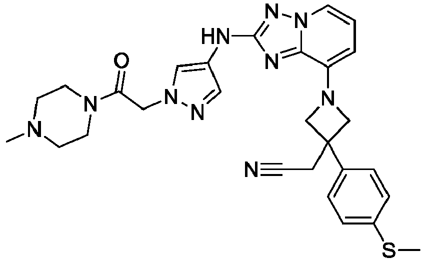 | 557. 3 |
| **15** | A | 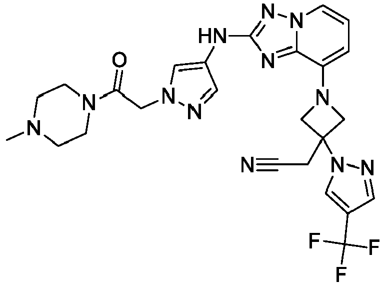 | 569. 3 |
| **16** | A | 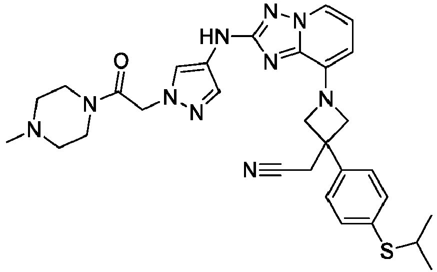 | 585. 4 |
| **17** | A | 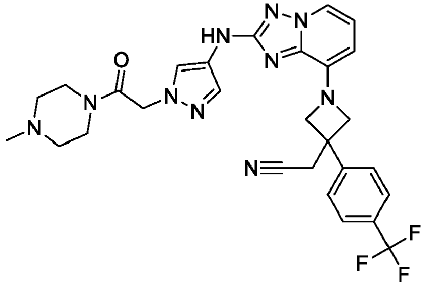 | 579. 4 |
| **18** | A | 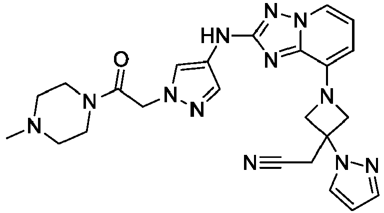 | 501. 3 |
| **19** | A | 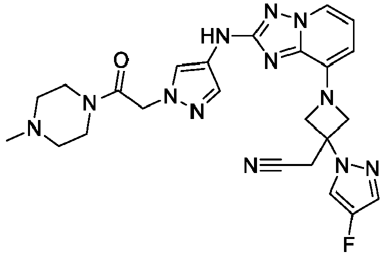 | 519. 3 |
| **20** | A | 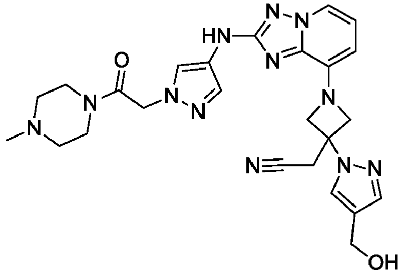 | 531. 3 |
| **21** | A | 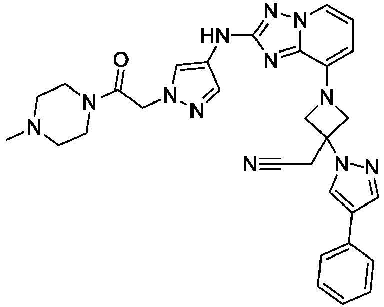 | 577. 4 |
| **22** | A | 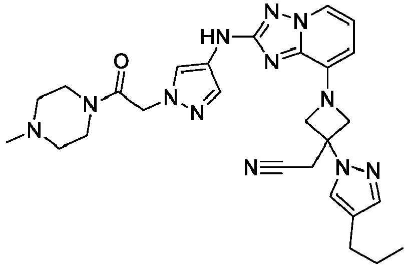 | 543. 4 |
| **23** | A | 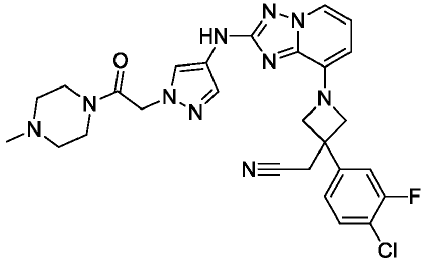 | 563. 3 |
| **24** | A | 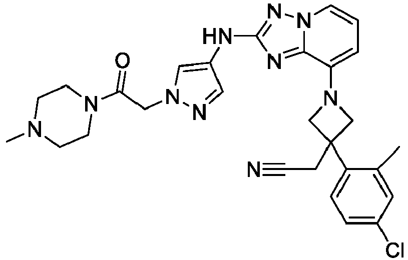 | 559. 3 |
| **25** | A | 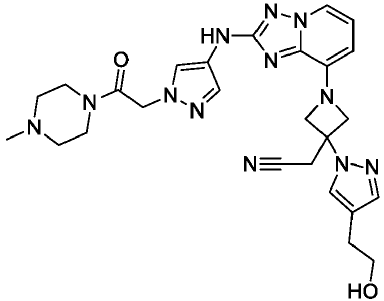 | 545. 3 |
| **26** | A | 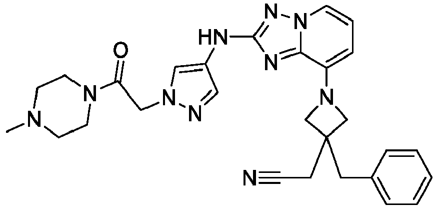 | 525. 4 |
| **27** | A | 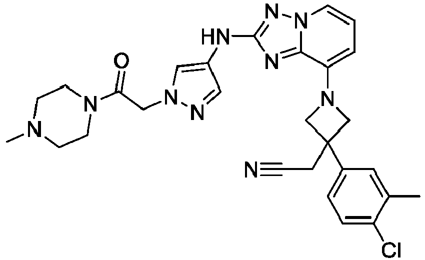 | 559. 3 |
| **28** | A | 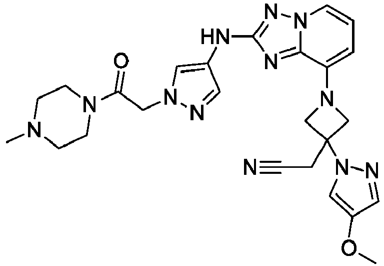 | 531. 4 |
| **29** | A | 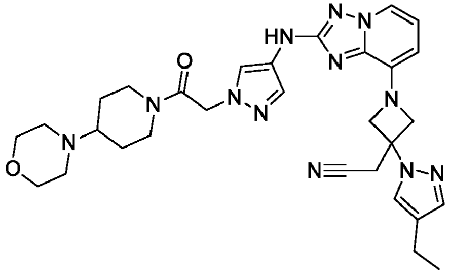 | 599. 3 |
| **30** | D | 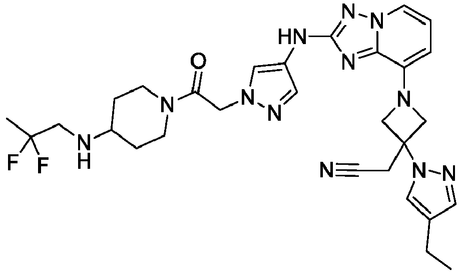 | 607. 4 |
| **31** | A | 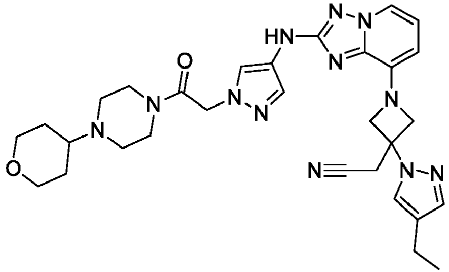 | 599. 4 |
| **32** | A | 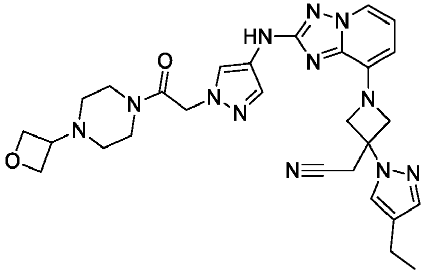 | 571. 3 |
| **33** | D | 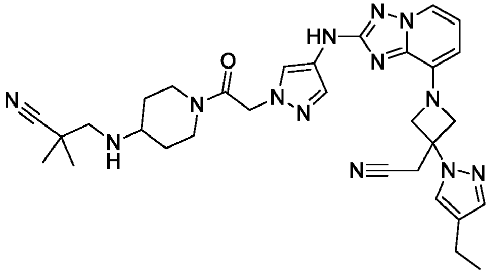 | 610. 3 |
| **34** | A | 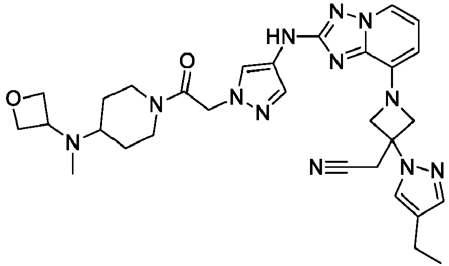 | 599. 4 |
| **35** | A | 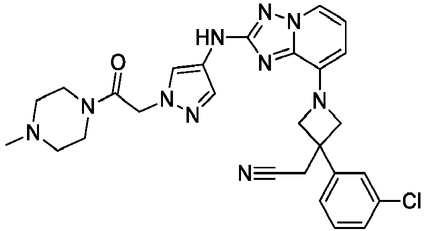 | 545. 3 |
| **36** | E | 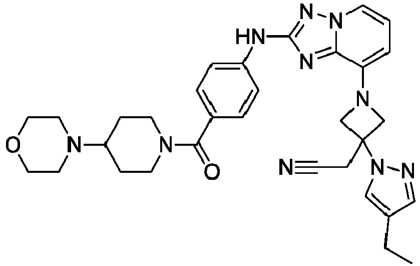 | 595. 3 |
| **37** | E | 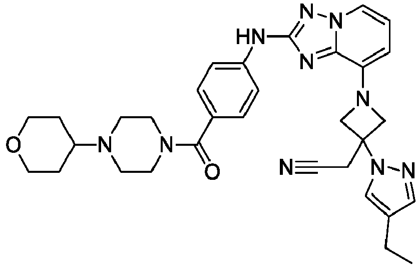 | 595. 3 |
| **38** | E | 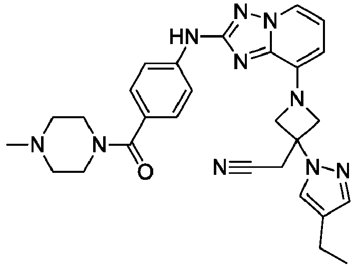 | 525. 3 |
| **39** | A | 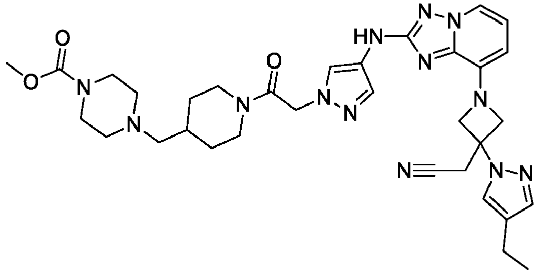 | 670. 3 |
| **40** | A | 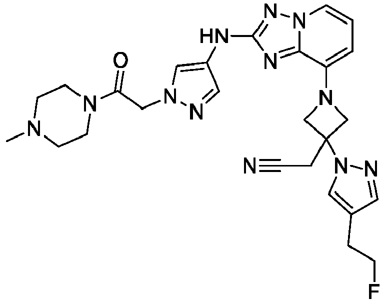 | 547. 3 |
| **41** | A | 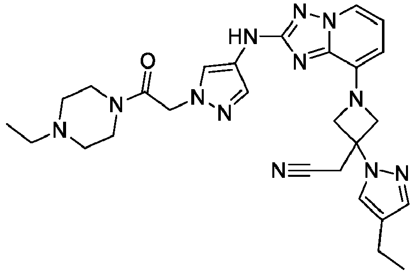 | 543. 3 |
| **42** | A | 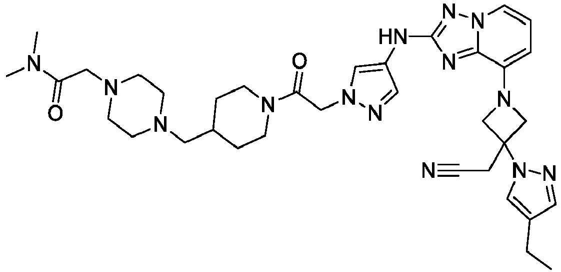 | 697. 4 |
| **43** | A | 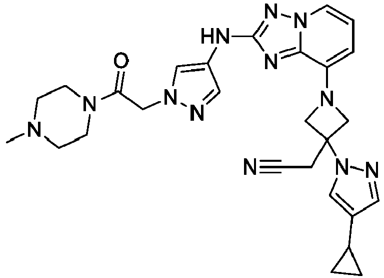 | 541. 3 |
| **44** | E | 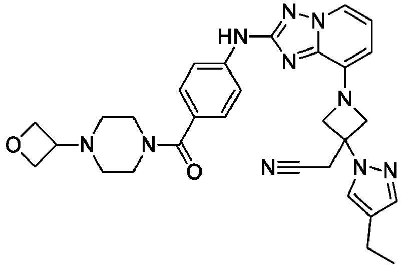 | 567. 4 |
| **45** | D | 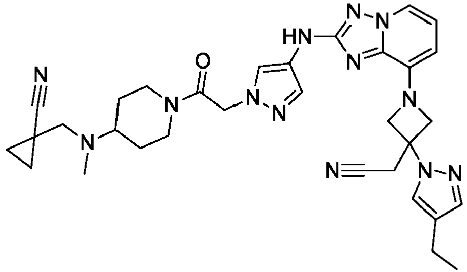 | 622. 5 |
| **46** | A | 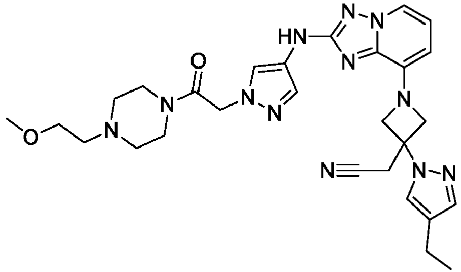 | 573. 3 |
| **47** | A | 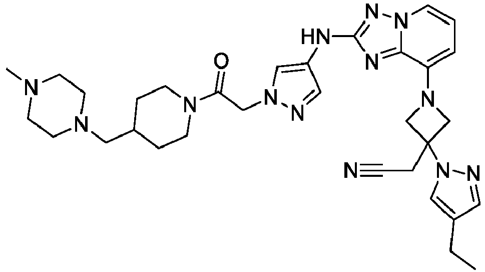 | 626. 5 |
| **48** | A | 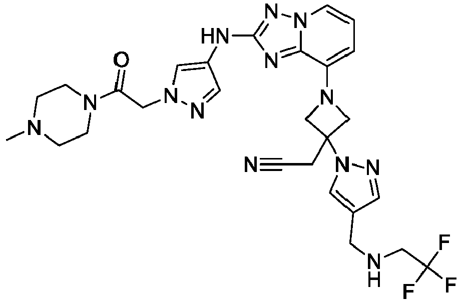 | 612. 3 |
| **49** | A | 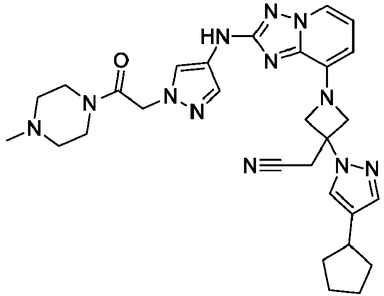 | 569. 4 |
| **50** | E | 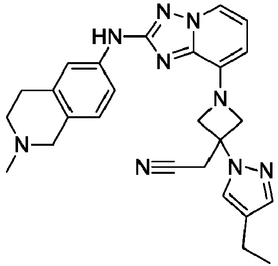 | 468. 3 |
| **51** | E | 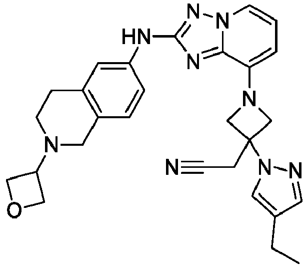 | 510. 3 |
| **52** | D | 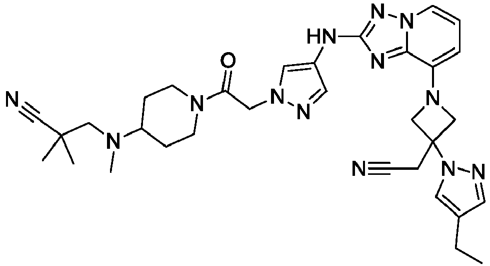 | 624. 5 |
| **53** | A | 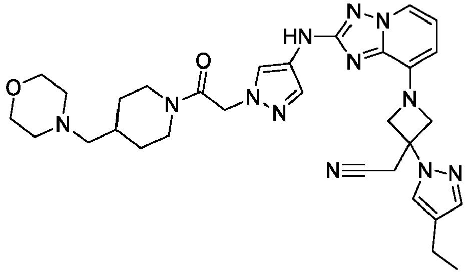 | 613. 4 |
| **54** | A | 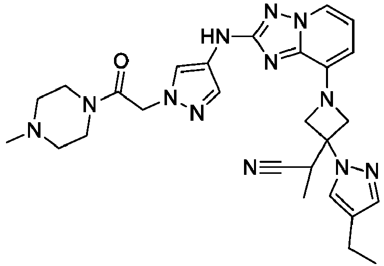 | 543. 3 |
| **55** | A | 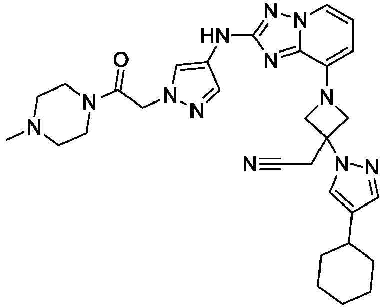 | 583. 3 |
| **56** | A | 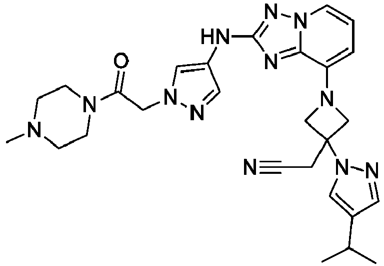 | 543. 4 |
| **57** | E | 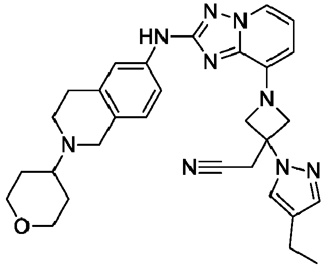 | 538. 4 |
| **58** | A | 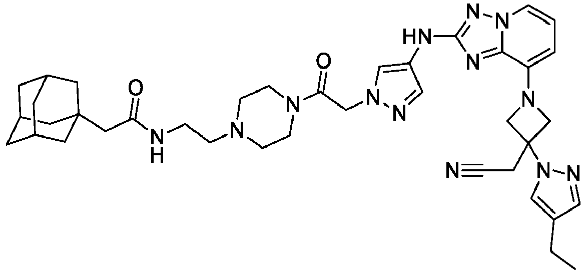 | 734. 5 |
| **59** | A | 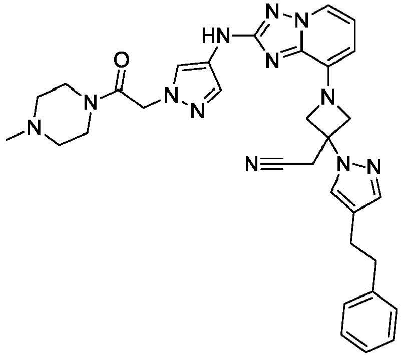 | 605. 4 |
| **60** | A | 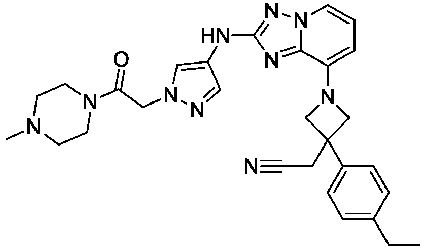 | 539. 3 |
| **61** | E | 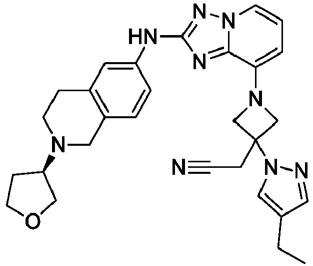 | 524. 4 |
| **62** | E | 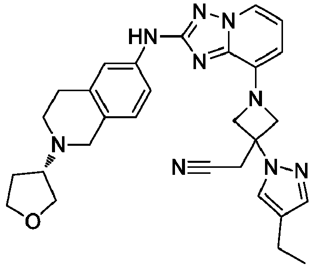 | 524. 4 |
| **63** | A | 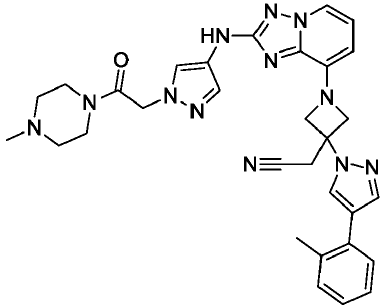 | 591. 4 |
| **64** | E | 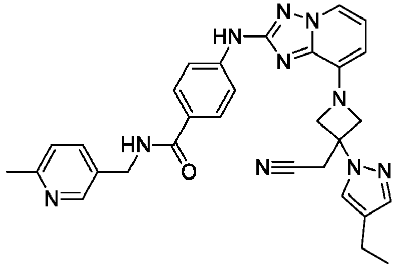 | 547. 4 |
| **65** | E | 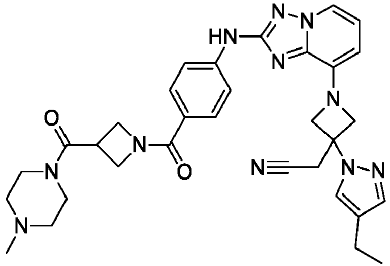 | 608. 5 |
| **66** | E | 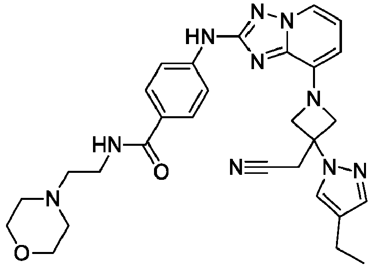 | 555. 4 |
| **67** | E | 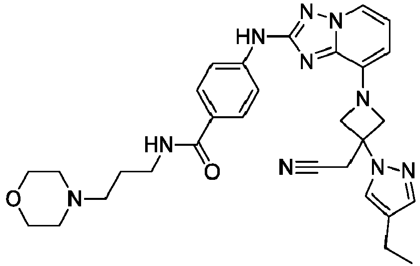 | 569. 4 |
| **68** | A | 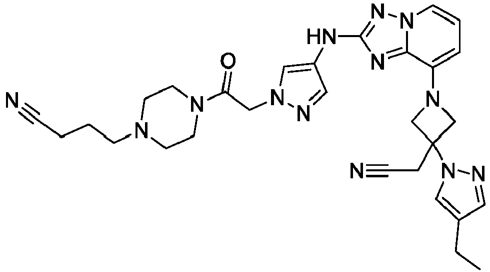 | 582. 4 |
| **69** | A | 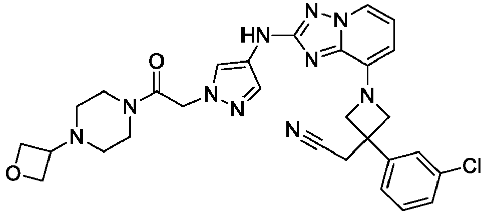 | 587. 3 |
| **70** | A | 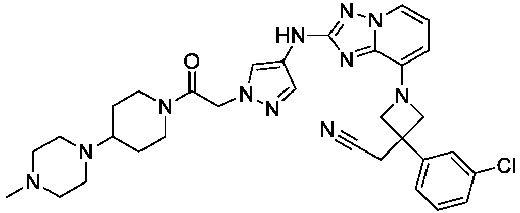 | 628. 4 |
| **71** | A | 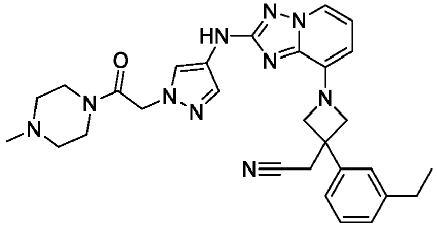 | 539. 4 |
| **72** | A | 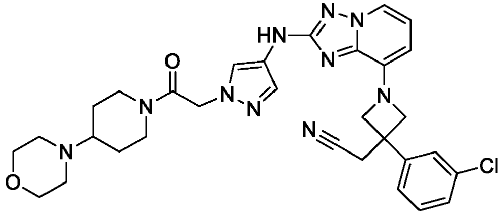 | 615. 4 |
| **73** | E | 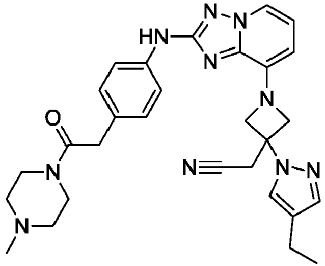 | 539. 4 |
| **74** | E | 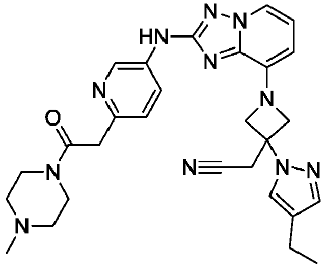 | 540. 3 |
| **75** | E | 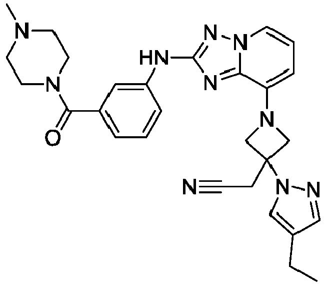 | 525. 4 |
| **76** | A | 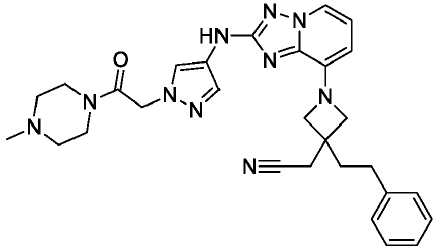 | 539. 3 |
| **77** | A | 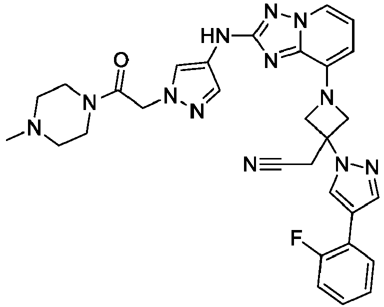 | 595. 3 |
| **78** | A | 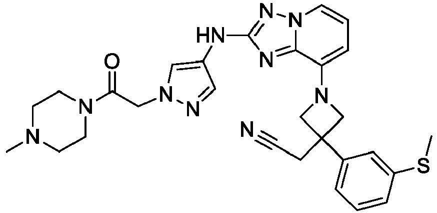 | 557. 3 |
| **79** | A | 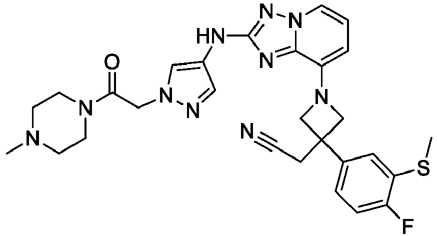 | 575. 3 |
| **80** | A | 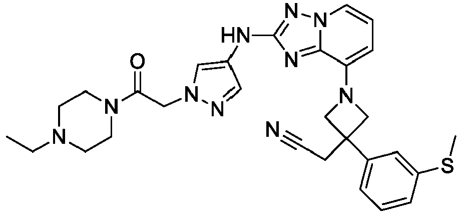 | 571. 3 |
| **81** | A | 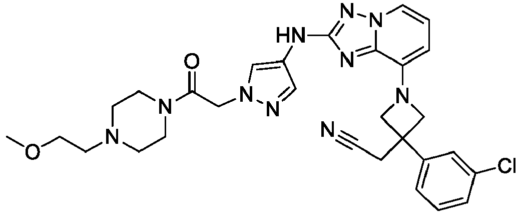 | 589. 3 |
| **82** | A | 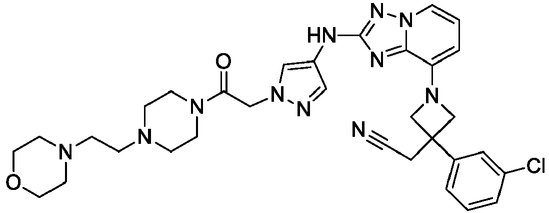 | 644. 4 |
| **83** | A | 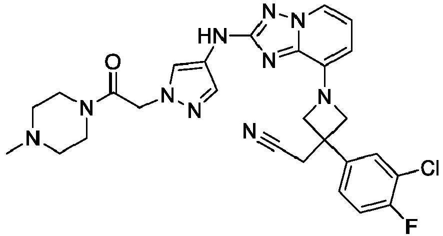 | 563. 3 |
| **84** | E | 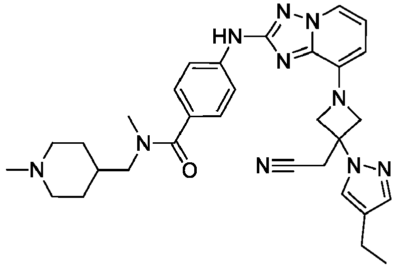 | 567. 4 |
| **85** | E | 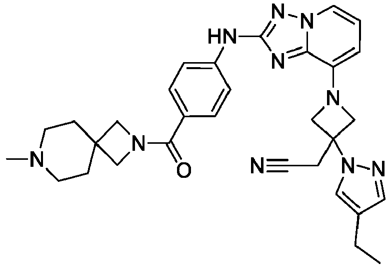 | 565. 4 |
| **86** | E | 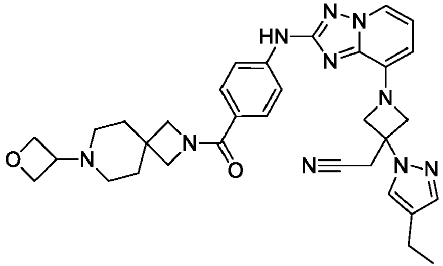 | 607. 4 |
| **87** | A | 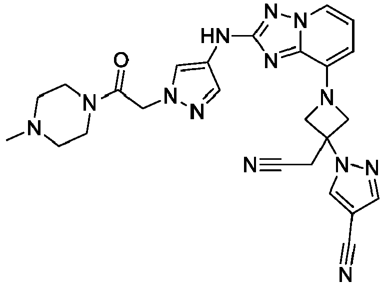 | 526. 3 |
| **88** | E | 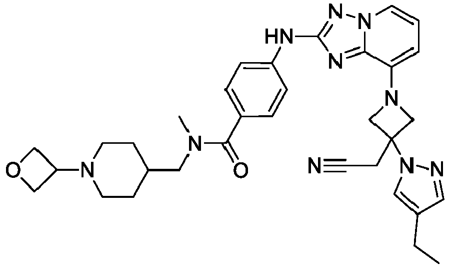 | 609. 5 |
| **89** | F | 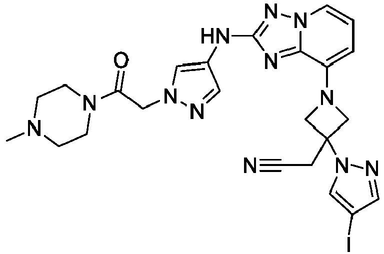 | 627. 3 |
| **90** | A | 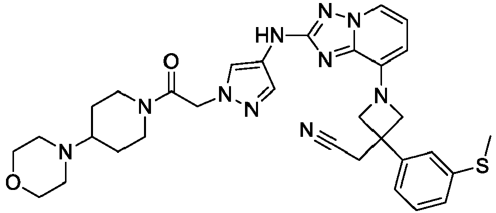 | 627. 3 |
| **91** | E | 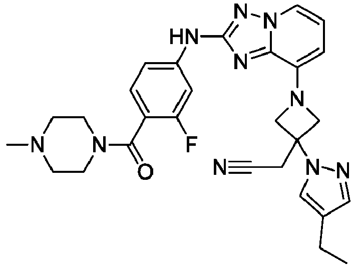 | 543. 3 |
| **92** | E | 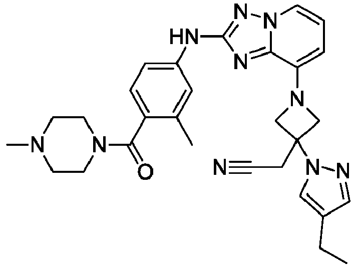 | 539. 4 |
| **93** | D | 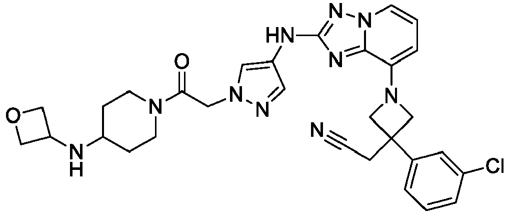 | 601. 3 |
| **94** | A | 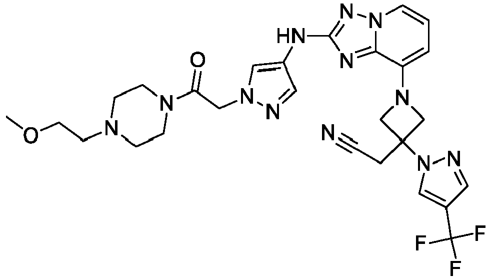 | 613. 3 |
| **95** | A | 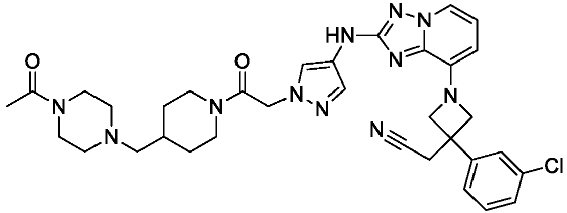 | 670. 4 |
| **96** | A | 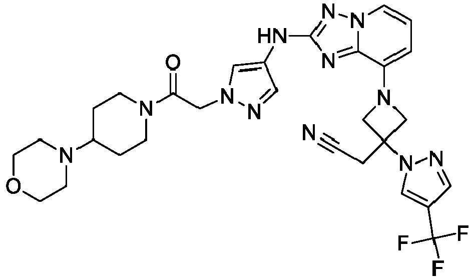 | 639. 4 |
| **97** | A | 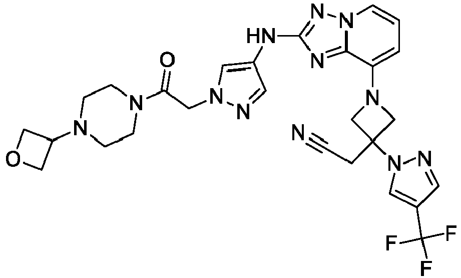 | 611. 3 |
| **98** | A | 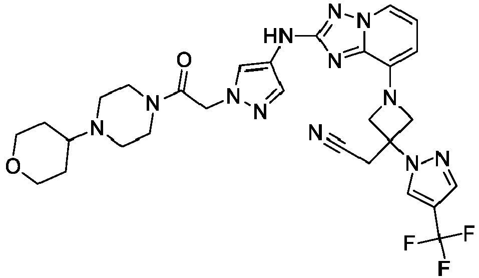 | 639. 4 |
| **99** | D | 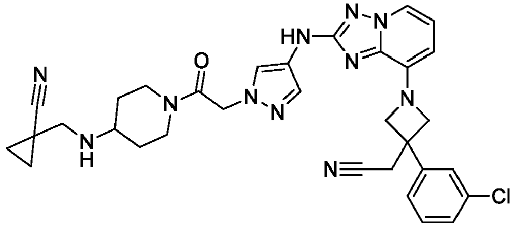 | 624. 4 |
| **10 0** | D | 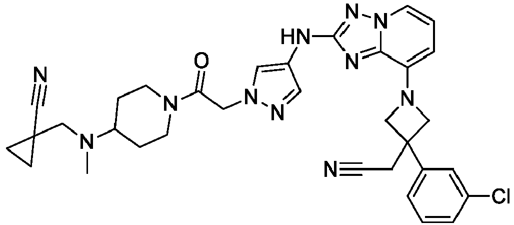 | 638. 4 |
| **10 1** | A | 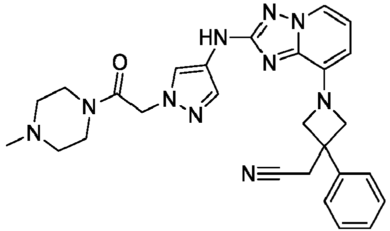 | 511. 3 |
| **10 2** | A | 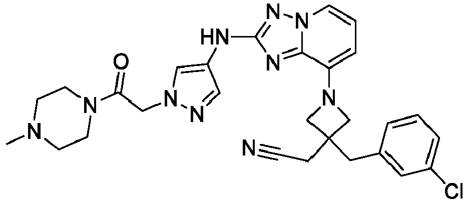 | 559. 2 |
| **10 3** | E | 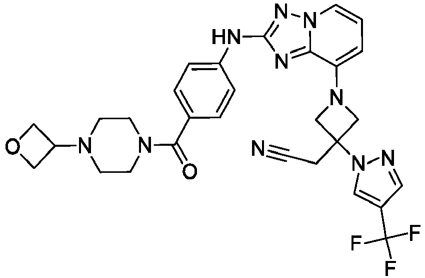 | 607. 3 |
| **10 4** | A | 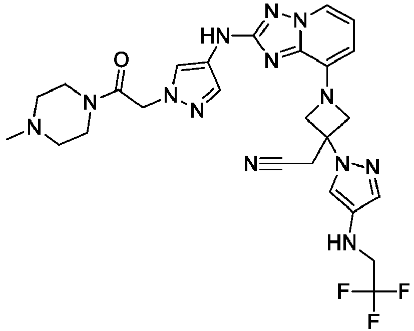 | 598. 4 |
| **10 5** | F | 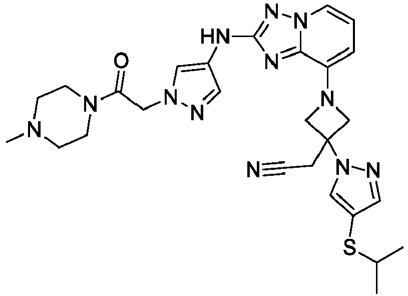 | 575. 4 |
| **10 6** | E | 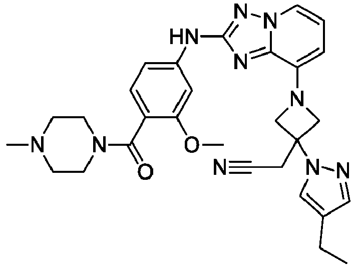 | 555. 4 |
| **10 7** | D | 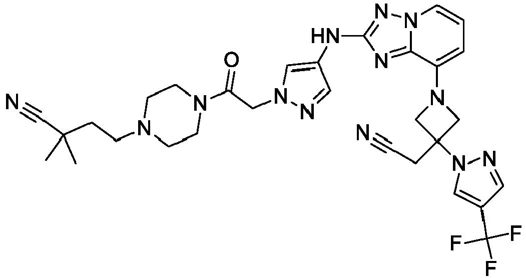 | 650. 4 |
| **10 8** | D | 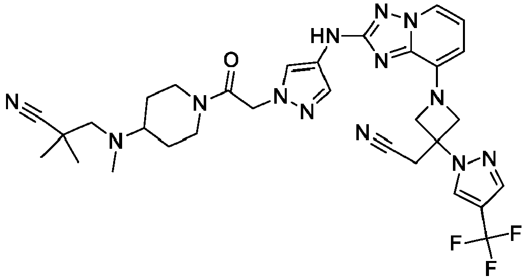 | 664. 5 |
| **10 9** | D | 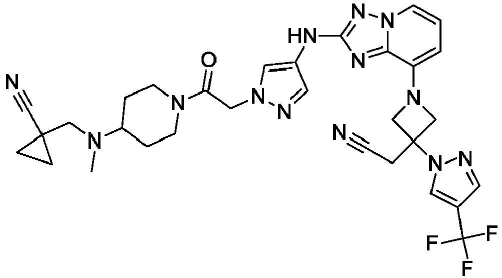 | 662. 4 |
| **11 0** | E | 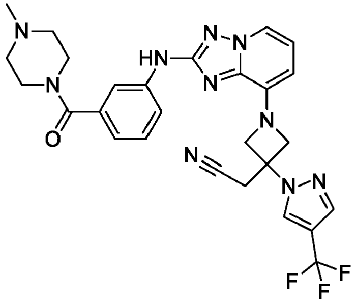 | 565. 3 |
| **11 1** | E | 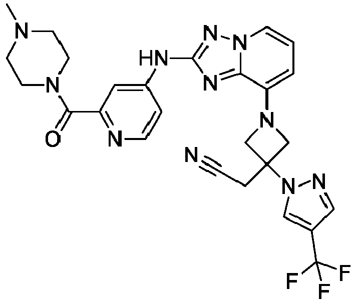 | 566. 3 |
| **11 2** | E | 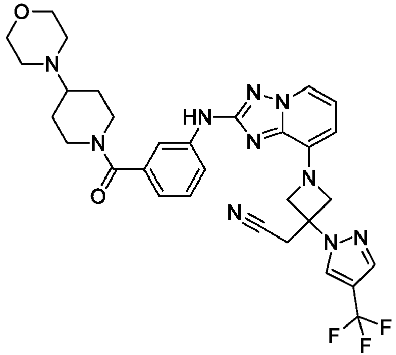 | 635. 4 |
| **11 3** | E | 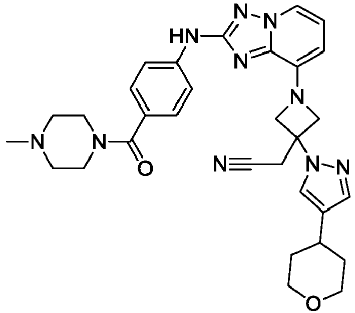 | 581. 4 |
| **11 4** | A | 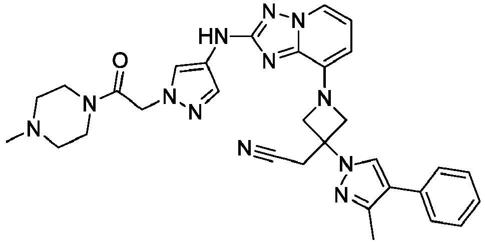 | 591. 4 |
| **11 5** | E | 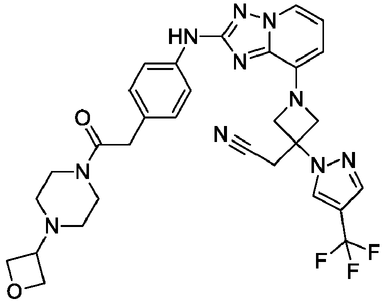 | 621. 3 |
| **11 6** | E | 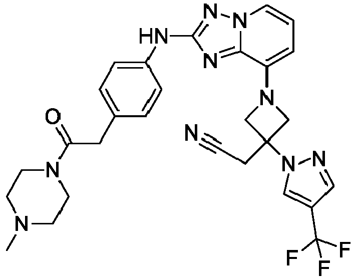 | 579. 4 |
| **11 7** | A | 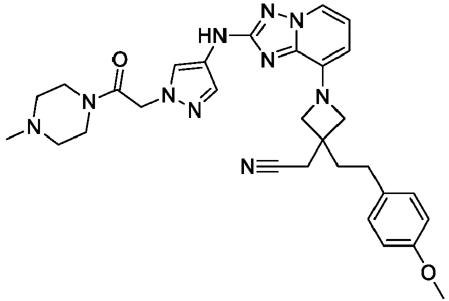 | 569. 4 |
| **11 8** | D | 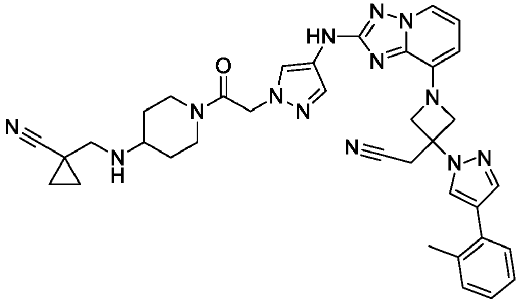 | 670. 5 |
| **11 9** | F | 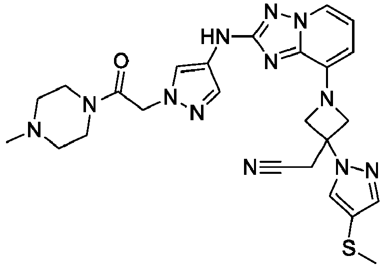 | 547. 4 |
| **12 0** | A | 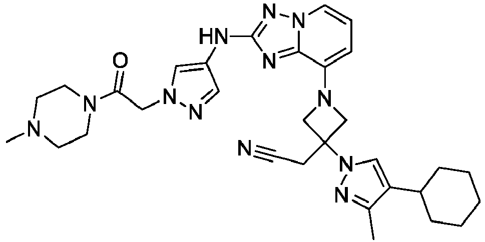 | 597. 4 |
| **12 1** | E | 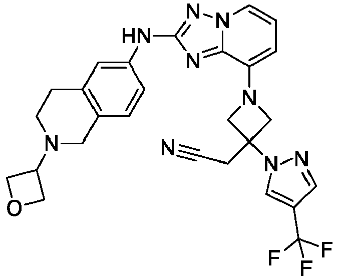 | 550. 3 |
| **12 2** | A | 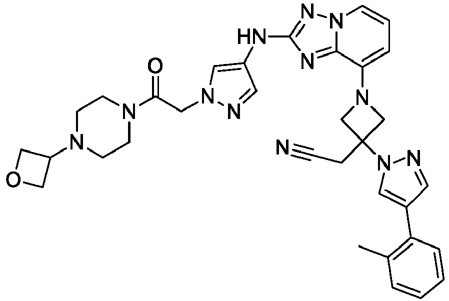 | 633. 5 |
| **12 3** | A | 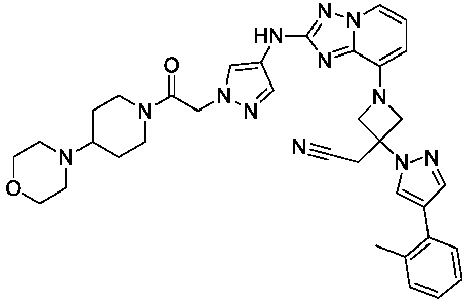 | 661. 5 |
| **12 4** | A | 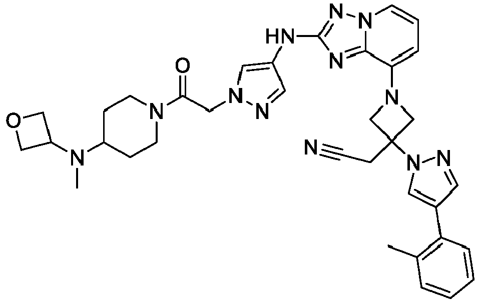 | 661. 4 |
| **12 5** | A | 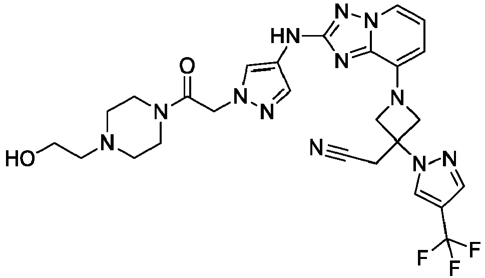 | 599. 3 |
| **12 6** | A | 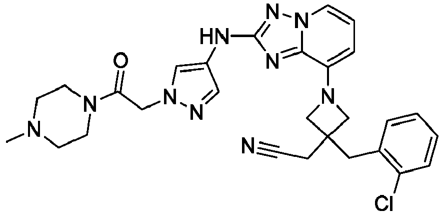 | 559. 4 |
| **12 7** | C | 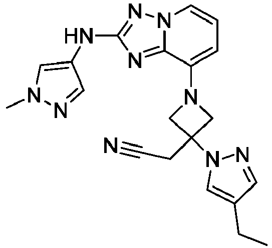 | 403. 2 |
| **12 8** | F | 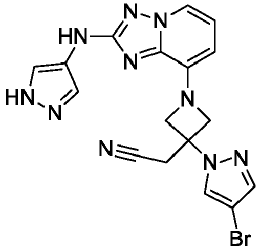 | 439. 1 |
| **12 9** | F | 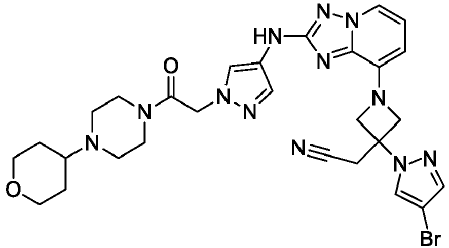 | 651. 3 |
| **13 0** | F | 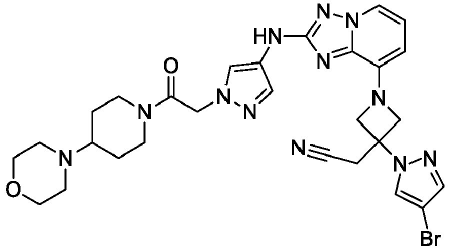 | 651. 3 |
| **13 1** | B | 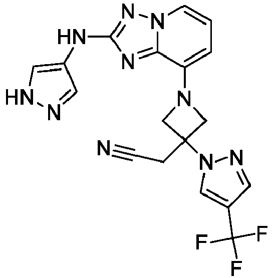 | 429. 2 |
| **13 2** | F | 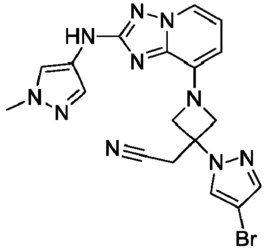 | 453. 1 |
| **13 3** | C | 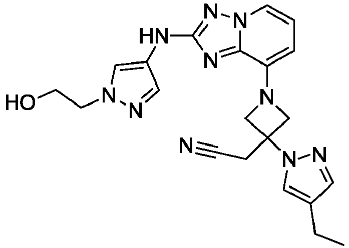 | 433. 2 |
| **13 4** | C | 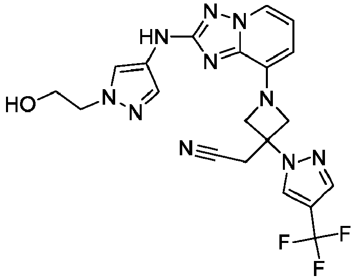 | 473. 2 |
| **13 5** | C | 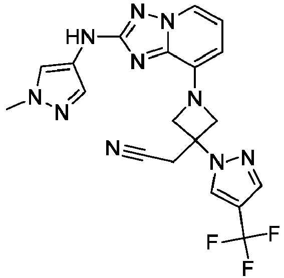 | 443. 2 |
| **13 6** | C | 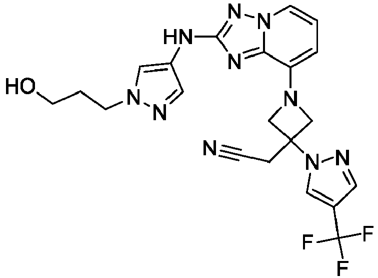 | 487. 3 |
| **13 7** | G | 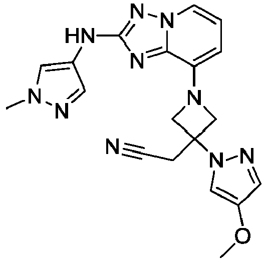 | 405. 2 |
| **13 8** | G | 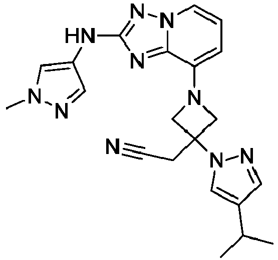 | 417. 3 |
| **13 9** | B | 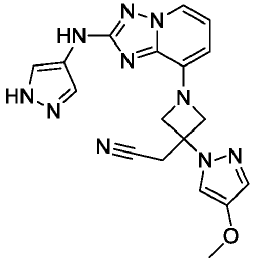 | 391. 2 |
| **14 0** | B | 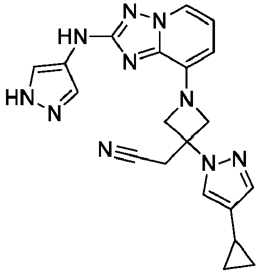 | 401. 2 |
| **14 1** | B | 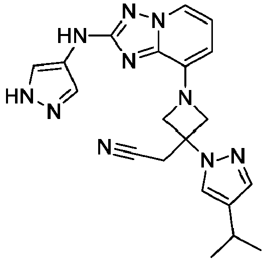 | 403. 3 |
| **14 2** | G | 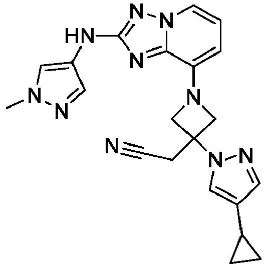 | 415. 2 |
| **14 3** | A | 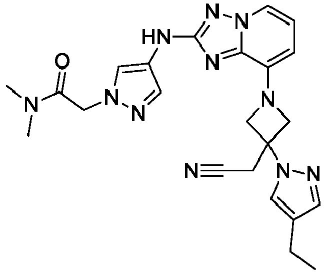 | 474. 3 |
| **14 4** | F | 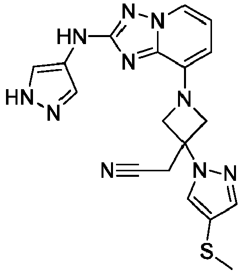 | 407. 1 |
| **14 5** | G | 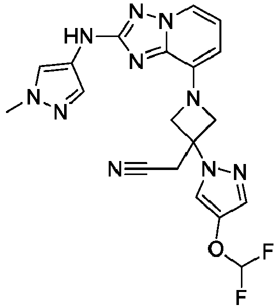 | 441. 2 |
| **14 6** | B | 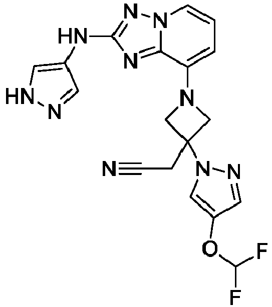 | 427. 2 |
| **14 7** | F | 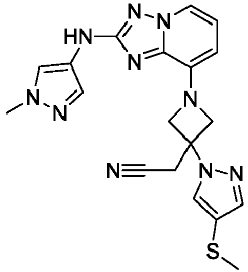 | 421. 2 |
| **14 8** | C | 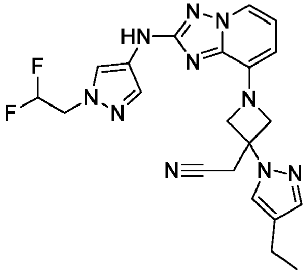 | 453. 3 |
| **14 9** | B | 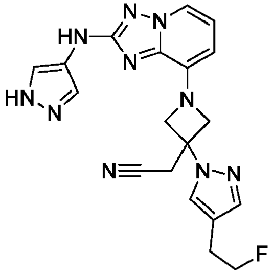 | 407. 3 |
| **15 0** | C | 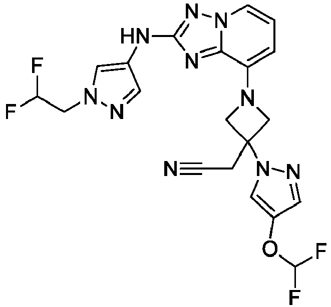 | 491. 2 |
| **15 1** | F | 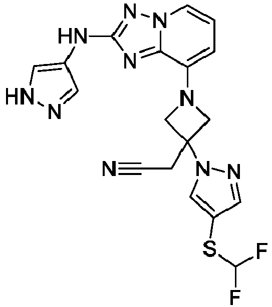 | 443. 2 |

### JAK Enzyme Assays were carried out as follows:

The activity of the isolated recombinant JAK1 and JAK2 kinase domain was measured by monitoring phosphorylation of a peptide derived from JAK3 (Val-Ala-Leu-Val-Asp-Gly-Tyr-Phe-Arg-Leu-Thr-Thr, fluorescently labeled on the N-terminus with 5-carboxyfluorescein) using the Caliper LabChip^{®} technology (Caliper Life Sciences, Hopkinton, MA). To determine inhibition constants (Ki), compounds were diluted serially in DMSO and added to 50 µL kinase reactions containing purified enzyme (1.5 nM JAK1, or 0.2 nM JAK2), 100 mM HEPES buffer (pH 7.2), 0.015% Brij-35, 1.5 µM peptide substrate, ATP (25 µM), 10 mM MgCl2, 4 mM DTT at a final DMSO concentration of 2%. Reactions were incubated at 22 °C in 384-well polypropylene microtiter plates for 30 minutes and then stopped by addition of 25 µL of an EDTA containing solution (100 mM HEPES buffer (pH 7.2), 0.015% Brij-35, 150 mM EDTA), resulting in a final EDTA concentration of 50 mM. After termination of the kinase reaction, the proportion of phosphorylated product was determined as a fraction of total peptide substrate using the Caliper LabChip^{®} 3000 according to the manufacturer's specifications. Ki values were then determined using the Morrison tight binding model (Morrison, J.F., Biochim. Biophys. Acta. 185:269-296 (1969); William, J.W. and Morrison, J.F., Meth. Enzymol., 63:437-467 (1979)) modified for ATP-competitive inhibition [Ki = Ki,app / (1 + [ATP] / Km,app)].

### JAK1 Pathway Assay in Cell Lines was carried out as follows:

Inhibitor potency (EC50) was determined in cell-based assays designed to measure JAK1 dependent STAT phosphorylation. As noted above, inhibition of IL-4, IL-13, and IL-9 signalling by blocking the Jak/Stat signaling pathway can alleviate asthmatic symptoms in pre-clinical lung inflammation models (Mathew et al., 2001, J Exp Med 193(9): 1087-1096; Kudlacz et. al., 2008, Eur J. Pharmacol 582(1-3): 154-161).

In one assay approach, TF-1 human erythroleukemia cells obtained from the American Type Culture Collection (ATCC; Manassas, VA) were used to measure JAK1-dependent STAT6 phosphorylation downstream of IL-13 stimulation. Prior to use in the assays, TF-1 cells were starved of GM-CSF overnight in OptiMEM medium (Life Technologies, Grand Island, NY) supplemented with 0.5% charcoal/dextran stripped fetal bovine serum (FBS), 0.1 mM non-essential amino acids (NEAA), and 1 mM sodium pyruvate. The assays were run in 384-well plates in serum-free OptiMEM medium using 300,000 cells per well. In a second assay approach, BEAS-2B human bronchial epithelial cells obtained from ATCC were plated at 100,000 cells per well of a 96-well plate one day prior to the experiment. The BEAS-2B assay was run in complete growth medium (bronchial epithelial basal medium plus bulletkit; Lonza; Basel, Switzerland).

Test compounds were serially diluted 1:2 in DMSO and then diluted 1:50 in medium just before use. Diluted compounds were added to the cells, for a final DMSO concentration of 0.2%, and incubated for 30 min (for the TF-1 assay) or 1 hr (for the BEAS-2B assay) at 37 °C. Then, cells were stimulated with human recombinant cytokine at their respective EC90 concentrations, as previously determined for each individual lot. Cells were stimulated with IL-13 (R&D Systems, Minneapolis, MN) for 15 min at 37°C. The TF-1 cell reactions were stopped by the direct addition of 10x lysis buffer (Cell Signaling Technologies, Danvers, MA), whereas the BEAS-2B cell incubations were halted by the removal of medium and addition of 1x lysis buffer. The resultant samples were frozen in the plates at -80 °C. Compound mediated inhibition of STAT6 phosphorylation was measured in the cell lysates using MesoScale Discovery (MSD) technology (Gaithersburg, MD). EC50 values were determined as the concentration of compound required for 50% inhibition of STAT phosphorylation relative to that measured for the DMSO control.

| **Example** | **JAK1 *K*ᵢ (uM)** | **JAK2 *K*ᵢ (uM)** | **IL-13 p-STAT6 BEAS-2B EC₅₀ (uM)** |
|---|---|---|---|
| **1** | 0.0008 | 0.0020 | 0.029 |
| **2** | 0.0175 | 0.0328 | |
| **3** | 0.0402 | 0.0640 | |
| **4** | 0.0006 | 0.0046 | 0.039 |
| **5** | 0.0019 | 0.0031 | 0.120 |
| **6** | 0.0095 | 0.0157 | |
| **7** | 0.0372 | 0.0504 | |
| **8** | 0.0003 | 0.0015 | 0.012 |
| **9** | 0.0006 | 0.0025 | 0.034 |
| **10** | 0.0004 | 0.0015 | 0.018 |
| **11** | 0.0013 | 0.0058 | 0.037 |
| **12** | 0.0009 | 0.0024 | 0.027 |
| **13** | 0.0016 | 0.0053 | 0.082 |
| **14** | 0.0018 | 0.0045 | 0.072 |
| **15** | 0.0022 | 0.0094 | >1 |
| **16** | 0.0006 | 0.0037 | 0.019 |
| **17** | 0.0003 | 0.0012 | 0.018 |
| **18** | 0.0007 | 0.0015 | 0.021 |
| **19** | 0.0044 | 0.0040 | 0.255 |
| **20** | 0.0012 | 0.0096 | >1 |
| **21** | 0.0011 | 0.0077 | 0.043 |
| **22** | 0.0010 | 0.0030 | 0.036 |
| **23** | 0.0007 | 0.0061 | 0.040 |
| **24** | 0.0004 | 0.0023 | 0.087 |
| **25** | 0.0004 | 0.0023 | 0.137 |
| **26** | 0.0004 | 0.0021 | 0.042 |
| **27** | 0.0005 | 0.0018 | 0.051 |
| **28** | 0.0004 | 0.0014 | 0.054 |
| **29** | 0.0006 | 0.0021 | 0.078 |
| **30** | 0.0007 | 0.0027 | 0.104 |
| **31** | 0.0004 | 0.0011 | 0.019 |
| **32** | 0.0004 | 0.0002 | 0.019 |
| **33** | 0.0003 | 0.0002 | 0.018 |
| **34** | 0.0004 | 0.0003 | 0.015 |
| **35** | 0.0008 | 0.0028 | 0.039 |
| **36** | 0.0004 | 0.0016 | 0.052 |
| **37** | 0.0005 | 0.0029 | 0.023 |
| **38** | 0.0007 | 0.0041 | 0.615 |
| **39** | 0.0003 | 0.0016 | 0.024 |
| **40** | 0.0003 | 0.0002 | 0.018 |
| **41** | 0.0005 | 0.0019 | 0.065 |
| **42** | 0.0004 | 0.0022 | 0.042 |
| **43** | 0.0006 | 0.0040 | 0.048 |
| **44** | 0.0022 | 0.0295 | 0.648 |
| **45** | 0.0003 | 0.0008 | 0.021 |
| **46** | 0.0002 | 0.0002 | 0.007 |
| **47** | 0.0004 | 0.0002 | 0.011 |
| **48** | 0.0005 | 0.0015 | 0.033 |
| **49** | 0.0006 | 0.0024 | 0.030 |
| **50** | 0.0018 | 0.0020 | 0.065 |
| **51** | 0.0003 | 0.0016 | 0.044 |
| **52** | 0.0002 | 0.0005 | 0.013 |
| **53** | 0.0009 | 0.0006 | 0.031 |
| **54** | 0.0010 | 0.0028 | 0.231 |
| **55** | 0.0010 | 0.0057 | 0.137 |
| **56** | 0.0023 | 0.0105 | 0.319 |
| **57** | 0.0003 | 0.0013 | 0.012 |
| **58** | 0.0004 | 0.0003 | 0.013 |
| **59** | 0.0002 | 0.0002 | 0.008 |
| **60** | 0.0004 | 0.0029 | 0.019 |
| **61** | 0.0004 | 0.0004 | 0.008 |
| **62** | 0.0004 | 0.0004 | 0.049 |
| **63** | 0.0002 | 0.0002 | 0.013 |
| **64** | 0.0003 | 0.0002 | 0.015 |
| **65** | 0.0005 | 0.0013 | 0.086 |
| **66** | 0.0003 | 0.0005 | 0.031 |
| **67** | 0.0005 | 0.0011 | 0.167 |
| **68** | 0.0003 | 0.0005 | 0.014 |
| **69** | 0.0003 | 0.0005 | 0.021 |
| **70** | 0.0002 | 0.0003 | 0.010 |
| **71** | 0.0003 | 0.0003 | 0.026 |
| **72** | 0.0006 | 0.0006 | 0.024 |
| **73** | 0.0007 | 0.0003 | 0.018 |
| **74** | 0.0004 | 0.0018 | 0.020 |
| **75** | 0.0004 | 0.0020 | 0.026 |
| **76** | 0.0003 | 0.0009 | 0.016 |
| **77** | 0.0002 | 0.0006 | 0.017 |
| **78** | 0.0003 | 0.0009 | 0.017 |
| **79** | 0.0003 | 0.0007 | 0.016 |
| **80** | 0.0005 | 0.0008 | 0.051 |
| **81** | 0.0004 | 0.0010 | 0.027 |
| **82** | 0.0002 | 0.0002 | 0.022 |
| **83** | 0.0002 | 0.0002 | 0.014 |
| **84** | 0.0003 | 0.0003 | 0.016 |
| **85** | 0.0011 | 0.0093 | 0.197 |
| **86** | 0.0002 | 0.0002 | 0.011 |
| **87** | 0.0002 | 0.0007 | 0.017 |
| **88** | 0.0004 | 0.0010 | 0.035 |
| **89** | 0.0064 | 0.0188 | |
| **90** | 0.0004 | 0.0002 | 0.022 |
| **91** | 0.0004 | 0.0009 | 0.213 |
| **92** | 0.0004 | 0.0017 | 0.019 |
| **93** | 0.0004 | 0.0007 | 0.079 |
| **94** | 0.0003 | 0.0010 | 0.054 |
| **95** | 0.0004 | 0.0013 | 0.041 |
| **96** | 0.0003 | 0.0008 | 0.028 |
| **97** | 0.0005 | 0.0008 | 0.051 |
| **98** | 0.0005 | 0.0008 | 0.032 |
| **99** | 0.0020 | 0.0040 | 0.071 |
| **100** | 0.0003 | 0.0015 | 0.026 |
| **101** | 0.0003 | 0.0002 | 0.016 |
| **102** | 0.0012 | 0.0051 | 0.290 |
| **103** | 0.0003 | 0.0017 | 0.033 |
| **104** | 0.0003 | 0.0001 | 0.022 |
| **105** | 0.0007 | 0.0016 | 0.042 |
| **106** | 0.0005 | 0.0013 | 0.040 |
| **107** | 0.0004 | 0.0011 | 0.044 |
| **108** | 0.0006 | 0.0004 | 0.037 |
| **109** | 0.0005 | 0.0004 | 0.058 |
| **110** | 0.0008 | 0.0009 | 0.100 |
| **111** | 0.0007 | 0.0004 | 0.039 |
| **112** | 0.0010 | 0.0046 | 0.069 |
| **113** | 0.0004 | 0.0006 | 0.043 |
| **114** | 0.0003 | 0.0003 | 0.016 |
| **115** | 0.0024 | 0.0229 | 0.139 |
| **116** | 0.0006 | 0.0040 | 0.090 |
| **117** | 0.0004 | 0.0024 | 0.020 |
| **118** | 0.0011 | 0.0073 | 0.099 |
| **119** | 0.0003 | 0.0002 | 0.016 |
| **120** | 0.0008 | 0.0061 | 0.046 |
| **121** | 0.0005 | 0.0044 | 0.031 |
| **122** | 0.0005 | 0.0037 | 0.043 |
| **123** | 0.0003 | 0.0013 | 0.087 |
| **124** | 0.0020 | 0.0038 | 0.085 |
| **125** | 0.0004 | 0.0004 | 0.021 |
| **126** | 0.0002 | 0.0004 | 0.012 |
| **127** | 0.0002 | 0.0004 | 0.010 |
| **128** | 0.0002 | 0.0002 | 0.008 |
| **129** | 0.0003 | 0.0008 | 0.049 |
| **130** | 0.0004 | 0.0011 | 0.117 |
| **131** | 0.0002 | 0.0004 | 0.043 |
| **132** | 0.0003 | 0.0005 | 0.051 |
| **133** | 0.0003 | 0.0003 | 0.012 |
| **134** | 0.0003 | 0.0003 | 0.013 |
| **135** | 0.0003 | 0.0005 | 0.010 |
| **136** | 0.0009 | 0.0008 | 0.020 |
| **137** | 0.0005 | 0.0028 | 0.039 |
| **138** | 0.0002 | 0.0003 | 0.016 |
| **139** | 0.0004 | 0.0009 | 0.039 |
| **140** | 0.0001 | 0.0002 | 0.011 |
| **141** | 0.0001 | 0.0001 | 0.009 |
| **142** | 0.0004 | 0.0005 | 0.014 |
| **143** | 0.0006 | 0.0017 | 0.031 |
| **144** | 0.0001 | 0.0003 | 0.007 |
| **145** | 0.0002 | 0.0003 | 0.006 |
| **146** | 0.0002 | 0.0003 | 0.007 |
| **147** | 0.0003 | 0.0006 | 0.010 |
| **148** | 0.0001 | 0.0004 | 0.008 |
| **149** | 0.0003 | 0.0006 | 0.021 |
| **150** | 0.0001 | 0.0002 | 0.006 |
| **151** | 0.0001 | 0.0014 | 0.042 |
| **152** | 0.0007 | 0.0001 | 0.005 |
| **153** | 0.0090 | 0.0155 | |

### Aspects of the present invention:

1. A compound of Formula (I) or (II): 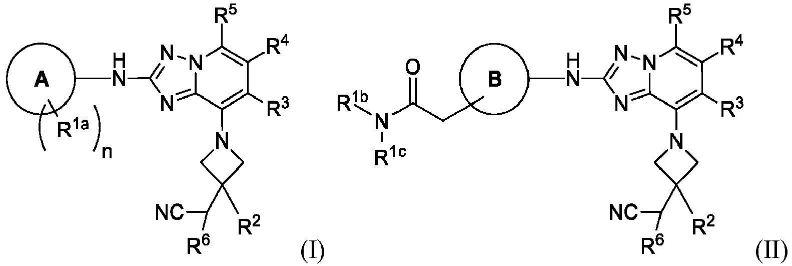 or a stereoisomer, tautomer, solvate, prodrug or salt thereof, wherein:
   Ring A is phenyl, pyridinyl, pyrazolyl or isoquinolinyl;
   Ring B is phenyl or 5-6 membered heteroaryl;
   n is 0, 1 or 2;
   R² is selected from
      (i) -(C0-C6 alkylene)-R^{c},
      (ii) -C(O)-NH-(C1-C6 alkyl optionally substituted by halogen, OH or CN) or
      (iii) -C(O)-(azetidinyl optionally substituted by C1-C6 alkyl or C1-C6 haloalkyl);
   R³, R⁴ and R⁵ are each independently selected from the group consisting of hydrogen, CH₃, CH₂CH₃, OCH₃, CF₃, F and Cl;
   R⁶ is H or C1-C3 alkyl;
   R^{1a} independently at each occurrence is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or - C(O)-(3-7 membered heterocycloalkyl optionally substituted by C1-C6 alkyl);
   R^{1b} and R^{1c} taken together form a 3-10 membered heterocycloalkyl optionally substituted by C1-C6 alkyl optionally substituted by halogen, CN, OH or C1-C6 alkoxy, -(CO-C6 alkylene)-(3-7 membered heterocycloalkyl optionally substituted by C1-C6 alkyl, -C(O)-(C1-C6 alkyl), -C(O)O-(C1-C6 alkyl) or - (C0-C6 alkylene)-C(O)-NR^{a}R^{b}) or -(CO-C6 alkylene)-NR^{a}R^{b};
   R^{a} and R^{b} are independently selected from a group consisting of hydrogen, -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) and 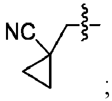 and
   R^{c} is 3-10 membered cycloalkyl, 3-10 membered heterocycloalkyl, phenyl or 5-6 membered heteroaryl, wherein R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).
2. The compound of aspect 1, further defined as a compound of Formula (I) or a stereoisomer, tautomer, solvate, prodrug or salt thereof.
3. The compound of aspect 1 or aspect 2, wherein R² is -C(O)-NH-(C1-C6 alkyl optionally substituted by halogen, OH or CN) or -C(O)-(azetidinyl optionally substituted by C1-C6 alkyl or C1-C6 haloalkyl).
4. The compound of aspect 1 or aspect 2, wherein R² is -(CO-C6 alkylene)-R^{c}.
5. The compound of aspect 4, wherein R^{c} is 3-10 membered cycloalkyl, 3-10 membered heterocycloalkyl or phenyl, where in R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).
6. The compound of aspect 4, wherein R^{c} is 5-6 membered heteroaryl optionally substituted by halogen, CN, OH, C1-C6 alkoxy optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).
7. The compound as in any one of aspects 1-6, wherein n is 0.
8. The compound as in any one of aspects 1-6, wherein n is 1 and R^{1a} is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or -C(O)-(3-7 membered heterocycloalkyl optionally substituted by C1-C6 alkyl).
9. The compound as in any one of aspects 1-8, wherein n is 1 and R^{1a} independently at each occurrence is halogen, C1-C6 alkyl optionally substituted by halogen, CN or OH, C1-C6 alkoxy, 3-10 membered cycloalkyl or 3-10 membered heterocycloalkyl.
10. The compound as in any one of aspects 1-9, wherein R^{a} is hydrogen or C1-C3 alkyl, and R^{b} is -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) or 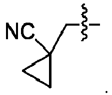
11. The compound as in any one of aspects 1-10, wherein Ring A is phenyl, pyridinyl or pyrazolyl.
12. The compound as in any one of aspects 1-11, wherein Ring A is pyrazolyl.
13. The compound of aspect 1, further defined as a compound of Formula (II) or a stereoisomer, tautomer, solvate, prodrug or salt thereof.
14. The compound of aspect 1 or aspect 13, wherein R² is -C(O)-NH-(C1-C6 alkyl optionally substituted by halogen, OH or CN) or -C(O)-(azetidinyl optionally substituted by C1-C6 alkyl or C1-C6 haloalkyl).
15. The compound of aspect 13, wherein R² is -(CO-C6 alkylene)-R^{c}.
16. The compound of aspect 15, wherein R^{c} is 3-10 membered cycloalkyl, 3-10 membered heterocycloalkyl or phenyl, where in R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).
17. The compound of aspect 15, wherein R^{c} is 5-6 membered heteroaryl optionally substituted by halogen, CN, OH, C1-C6 alkoxy optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).
18. The compound as in any one of aspects 1 and 14-17, wherein R^{1b} and R^{1c} taken together form a 3-10 membered heterocycloalkyl optionally substituted by -C1-C6 alkyl optionally substituted by halogen, CN, OH or C1-C6 alkoxy, -(C0-C6 alkylene)-(3-7 membered heterocycloalkyl optionally substituted by -C1-C6 alkyl, -C(O)-(C1-C6 alkyl), -C(O)O-(C1-C6 alkyl) or -(C0-C6 alkylene)-C(O)-NR^{a}R^{b}), -(C0-C6 alkylene)-NR^{a}R^{b} or -C(O)-(3-7 heterocycloalkyl optionally substituted by C1-C6 alkyl or C1-C6 haloalkyl).
19. The compound as in any one of aspects 1 and 14-18, wherein Ring B is phenyl.
20. The compound as in any one of aspects 1 and 14-18, wherein Ring B is 5-6 membered heteroaryl.
21. The compound of aspect 20, wherein Ring B is pyrazolyl.
22. The compound as in any one of aspects 1-21, wherein R³, R⁴ and R⁵ are each independently selected from the group consisting of hydrogen, CH₃, CH₂CH₃, CF₃, F and Cl.
23. The compound as in any one of aspects 1-22, wherein R³, R⁴ and R⁵ are each hydrogen.
24. The compound as in any one of aspects 1-23, wherein R⁶ is hydrogen.
25. The compound of aspect 1, selected from Table 1, or a salt thereof.
26. A pharmaceutical composition comprising a compound of any of aspects 1-25 or a stereoisomer, tautomer, solvate, prodrug or salt thereof and a pharmaceutically acceptable carrier, diluent or excipient.
27. Use of a compound of any of aspects 1-25 or a stereoisomer, tautomer, solvate, prodrug or salt thereof in therapy.
28. Use of a compound of any of aspects 1-25 or a stereoisomer, tautomer, solvate, prodrug or salt thereof in the treatment of an inflammatory disease.
29. Use of a compound of any of aspects 1-25 or a stereoisomer, tautomer, solvate, prodrug or salt thereof for the preparation of a medicament for the treatment of an inflammatory disease.
30. A compound of any of aspects 1-25 or a stereoisomer, tautomer, solvate, prodrug or salt thereof for use in the treatment of an inflammatory disease.
31. The use or compound of any of aspects 28-30 or a stereoisomer, tautomer, solvate, prodrug or salt thereof, wherein the inflammatory disease is asthma.
32. A method of preventing, treating or lessening the severity of a disease or condition responsive to the inhibition of a Janus kinase activity in a patient, comprising administering to the patient a therapeutically effective amount of a compound of any of aspects 1-25 or a stereoisomer, tautomer, solvate, prodrug or salt thereof.
33. The method of aspect 32, wherein the disease or condition is asthma.
34. The method of aspect 32, wherein the Janus kinase is JAK1.

## Claims

1. A compound of Formula (1): 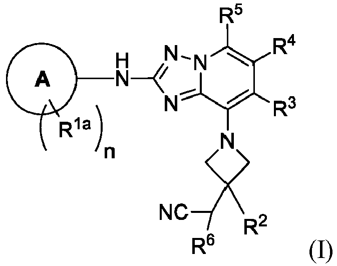 or a stereoisomer, tautomer, solvate, prodrug or salt thereof, wherein:
Ring A is phenyl, pyridinyl, pyrazolyl or isoquinolinyl;
n is 0, 1 or 2;
R² is -(CO-C6 alkylene)-R^{c},
R³, R⁴ and R⁵ are each independently selected from the group consisting of hydrogen, CH₃, CH₂CH₃, OCH₃, CF₃, F and Cl;
R⁶ is H or C1-C3 alkyl;
R^{1a} independently at each occurrence is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or - C(O)-(3-7 membered heterocycloalkyl optionally substituted by C1-C6 alkyl);
R^{a} and R^{b} are independently selected from a group consisting of hydrogen, -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) and 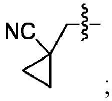 and
R^{c} is 5-6 membered heteroaryl optionally substituted by halogen, CN, OH, C1-C6 alkoxy optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, -(C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), - (C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).membered heteroaryl, wherein R^{c} is optionally substituted by halogen, CN, OH, C1-C6 alkyl optionally substituted by halogen, OH, CN, C1-C6 alkoxy or C1-C6 thioalkyl, C1-C6 alkoxy optionally substituted by halogen, C1-C6 thioalkyl optionally substituted by halogen, -(C0-C6 alkylene)-NR^{a}R^{b}, - (C0-C6 alkylene)-3-7 membered cycloalkyl, -(CO-C6 alkylene)-(3-10 membered heterocycloalkyl optionally substituted by halogen, OH, CN, C1-C6 alkyl or C1-C6 haloalkyl), -(C0-C6 alkylene)-(phenyl optionally substituted by halogen, OH, CN, C1-C6 alkyl or C1-C6 haloalkyl) or -(C0-C6 alkylene)-(5-6 membered heteroaryl optionally substituted by halogen, OH, CN, C1-C6 alkyl or C1-C6 haloalkyl).

2. The compound as in claim 1, wherein n is 0.

3. The compound as in any one of claims 1-2, wherein n is 1 and R^{1a} is halogen, C1-C6 alkyl optionally substituted by halogen, CN or OH, C1-C6 alkoxy, 3-10 membered cycloalkyl, 3-10 membered heterocycloalkyl, -C(O)-NR^{a}R^{b}, -C(O)-(3-10 membered heterocycloalkyl optionally substituted by C1-C6 alkyl, 3-7 membered heterocycloalkyl or -C(O)-(3-7 membered heterocycloalkyl optionally substituted by C1-C6 alkyl).

4. The compound as in any one of claims 1-3, wherein n is 1 and R^{1a} independently at each occurrence is halogen, C1-C6 alkyl optionally substituted by halogen, CN or OH, C1-C6 alkoxy, 3-10 membered cycloalkyl or 3-10 membered heterocycloalkyl.

5. The compound as in any one of claims 1-4, wherein R^{a} is hydrogen or C1-C3 alkyl, and R^{b} is -C1-C6 alkyl optionally substituted by halogen, OH, CN or C1-C6 alkoxy, -C(O)-(C1-C6 alkylene)-(3-10 membered cycloalkyl), -(C0-C6 alkylene)-(5-6 membered heteroaryl optionally substituted by C1-C6 alkyl), -(C0-C6 alkylene)-(3-7 membered heterocycloalkyl optionally substituted by C1-C6 alkyl or 3-7 membered heterocycloalkyl) or

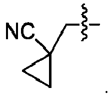

6. The compound as in any one of claims 1-5, wherein Ring A is phenyl, pyridinyl or pyrazolyl.

7. The compound as in any one of claims 1-6, wherein R³, R⁴ and R⁵ are each independently selected from the group consisting of hydrogen, CH₃, CH₂CH₃, CF₃, F and Cl.

8. The compound as in any one of claims 1-7, wherein R³, R⁴ and R⁵ are each hydrogen.

9. The compound as in any one of claims 1-8, wherein R⁶ is hydrogen.

10. The compound of claim 1, selected from Table 1, or a salt thereof.

11. A pharmaceutical composition comprising a compound of any of claims 1-10 or a stereoisomer, tautomer, solvate, prodrug or salt thereof and a pharmaceutically acceptable carrier, diluent or excipient.

12. A compound of any of claims 1-10 or a stereoisomer, tautomer, solvate, prodrug or salt thereof for use in the treatment of an inflammatory disease.
